(19) Europäisches Patentamt
European Patent Office
Office européen des brevets

(11) EP 3 798 213 B1

(12) **EUROPEAN PATENT SPECIFICATION**

(45) Date of publication and mention of the grant of the patent:
**10.01.2024 Bulletin 2024/02**

(21) Application number: **19199923.4**

(22) Date of filing: **26.09.2019**

(51) International Patent Classification (IPC):
***C07D 401/14*** (2006.01) ***H10K 85/60*** (2023.01)
***H10K 50/16*** (2023.01) ***H10K 50/165*** (2023.01)
***H10K 50/17*** (2023.01)

(52) Cooperative Patent Classification (CPC):
**C07D 401/14; H05B 33/20; H10K 85/654; H10K 85/6572;** H10K 50/165

(54) **ORGANIC SEMICONDUCTOR LAYER, ORGANIC ELECTRONIC DEVICE COMPRISING THE SAME AND COMPOUNDS THEREFOR**

ORGANISCHE HALBLEITERSCHICHT, ORGANISCHE ELEKTRONISCHE VORRICHTUNG DAMIT UND VERBINDUNGEN DAFÜR

COUCHE SEMI-CONDUCTRICE ORGANIQUE, DISPOSITIF ÉLECTRONIQUE ORGANIQUE LA COMPRENANT ET COMPOSÉS CORRESPONDANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**31.03.2021 Bulletin 2021/13**

(73) Proprietor: **Novaled GmbH**
**01099 Dresden (DE)**

(72) Inventors:
- **GALAN GARCIA, Elena**
**01099 Dresden (DE)**
- **SCHULZE, Benjamin**
**01099 Dresden (DE)**
- **UVAROV, Vladimir**
**01099 Dresden (DE)**
- **SENKOVSKYY, Volodymir**
**01099 Dresden (DE)**

(74) Representative: **Michalski Hüttermann & Partner Patentanwälte mbB**
**Kaistraße 16A**
**40221 Düsseldorf (DE)**

(56) References cited:
**EP-A1- 2 468 731**
**WO-A1-2015/083948**
**WO-A2-2016/089165**
**CN-A- 105 810 837**
**CN-A- 107 602 538**
**KR-A- 20150 012 974**
**KR-A- 20150 134 923**
**KR-A- 20150 135 626**
**US-A1- 2015 318 510**
**US-A1- 2019 263 834**

EP 3 798 213 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

The present invention relates to an organic semiconductor layer and an organic electronic device comprising the same. The invention further relates to a display device comprising the organic electronic device.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

US 20150318510 A1 refers to an organic light-emitting device includes an anode, a cathode, and an organic layer between the anode and the cathode, wherein the organic layer includes a mixed organic layer, and the mixed organic layer includes at least two different compounds, and a triplet energy of at least one compound of the at least two different compounds is 2.2 eV or higher.

WO 201689165 A2 refers to a compound that can be applied to a light emission layer, an electron injection layer, or an electron transport layer, and the use of the compound and a carbazol derivative together as hosts of the organic light emitting element.

CN 105810837 A refers to an organic light-emitting device includes a first electrode; a second electrode; an organic layer between the first electrode and the second electrode, the organic layer including an emission layer; and an electron transport region between the emission layer and the second electrode, wherein the emission layer includes a first material, and the electron transport region includes a second material.

KR 20150012974 A refers to a compound for an organic electroluminescent device and an organic electroluminescent device including the same.

US 20190263834 A1 refers to a hetero-cyclic compound represented by Chemical Formula 1, and an organic light emitting device comprising the same.

CN 110156755 A refers to an organic polymer luminescent material, and describes tetra pyridine pheryl pyrazine.

CN 106916170 A refers to a disubstituted derivative of carboline, and a preparation method and application thereof.

CN 107602538 A refers to an organic compound based on pyridine and quinoxaline and use of the organic compound to an OLED device.

KR 20150135626 A refers to a compound which can obtain high light emitting efficiency and low driving voltage of a device and expand lifespan of the device; an organic electric device using the same; and an electronic apparatus thereof.

KR 20150134923 A refers to a a compound which can obtain high light emitting efficiency and low driving voltage of a device and expand lifespan of the device; an organic electric device using the same; and an electronic apparatus thereof.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic electronic device, such as an organic light emitting diode, may be applied to a large-size flat panel display.

Further, development of an organic semiconductor layer being capable to have an extended life span at higher current density and thereby at higher brightness is needed. In particular the development of an organic semiconductor material or semiconductor layer is needed with respect to lowering the operating voltage, which is important for reducing power consumption and increasing battery life, for example of a mobile display device.

There remains a need to improve performance of organic semiconductor materials, organic semiconductor layers, as well as organic electronic devices thereof, in particular to achieve increased lifetime through improving the characteristics of the compounds comprised therein.

**DISCLOSURE**

An aspect of the present invention provides an organic electronic device comprising an anode, a cathode and at least one organic semiconductor layer, wherein the organic electronic device further comprises at least one emission layer and the at least one organic semiconductor layer is arranged between the at least one emission layer and the cathode, wherein the at least one organic semiconductor layer comprises:

- a metal dopant, and
- a compound represented by the following Formula (I):

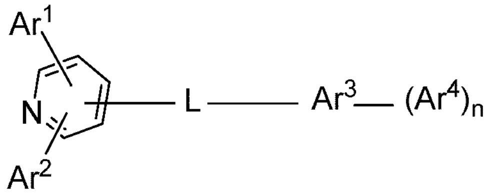

(I)

wherein,

$Ar^1$ and $Ar^2$ are same or different and independently selected from hydrogen, substituted or unsubstituted $C_6$ to $C_{36}$ aryl, substituted or unsubstituted $C_3$ to $C_{36}$ heteroaryl, wherein, at least one of the $Ar^1$ and $Ar^2$ group comprises a substituted or unsubstituted $C_3$ to $C_{36}$ heteroaryl;

L is a single bond, a substituted or unsubstituted $C_6$ to $C_{18}$ arylene, a substituted or unsubstituted $C_3$ to $C_{18}$ heteroarylene, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group selected from a substituted $C_4$ N-heteroarylene, a substituted or unsubstituted $C_{12}$ heteroarylene comprising at least three fused rings and one hetero atom selected from O, S or N, a substituted pyrazinylene, a substituted pyrimidinylene, a substituted or unsubstituted acridinylene, a substituted or unsubstituted benzoacridinylene, a substituted or unsubstituted dibenzoacridinylene, a substituted or unsubstituted dibenzofuranylene, a substituted or unsubstituted carbazolylene, a substituted or unsubstituted benzoquinolinylene, a substituted or unsubstituted phenanthridinylene, a substituted or unsubstituted dinaphthofuranylene or a substituted or unsubstituted dinaphthothiophenylene;

$Ar^4$ is a group independently selected from H, a substituted or unsubstituted $C_6$ to $C_{18}$ aryl, a substituted or unsubstituted $C_3$ to $C_{20}$ heteroaryl, an unsubstituted phenyl, an unsubstituted biphenyl, an unsubstituted naphthyl, an unsubstituted anthracenyl, an unsubstituted phenanthridinyl, an unsubstituted pyridyl, an unsubstituted quinolinyl, an unsubstituted pryrimidyl, or having the formula (II) or (III),

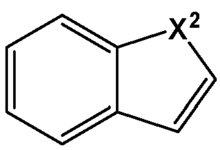

(II),

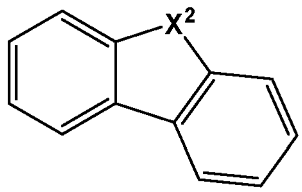

(III),

wherein the group of formula (II) and (III) are substituted or unsubstituted and $X^2$ is selected from N, S, O or $C(R^1)_2$;

n is 0, 1, 2, 3 or 4;

wherein the substituents of L, $Ar^1$, $Ar^2$, $Ar^3$, $Ar^4$ and/or N are independently selected from:

- $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or

perfluorinated $C_1$ to $C_{16}$ alkoxy, partially or perdeuterated $C_1$ to $C_{16}$ alkyl, partially or perdeuterated $C_1$ to $C_{16}$ alkoxy, $-PX^3(R^2)_2$, D, F or CN;

- $R^1$ and $R^2$ are independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy and
- two $R^1$ can be linked together to form a ring
- $X^3$ is selected from S or O.

Hetero atoms, if not otherwise stated, may be individually selected from N, O, S, B, Si, P, Se, preferably from N, O and S and more preferred is N.

If not otherwise stated H can represent hydrogen or deuterium.

If not otherwise stated the final syllable "ene"for the group members of $Ar^3$ for n=0 or if considered by an expert may be "yl". For example for n=0, the group members of $Ar^3$ may be read or replaced with "substituted $C_4$ N-heteroaryl, a substituted or unsubstituted $C_{12}$ heteroaryl comprising at least three fused rings and one hetero atom selected from O, S or N, a substituted pyrazinyl, a substituted pyrimidinyl, a substituted or unsubstituted acridinyl, a substituted or unsubstituted benzoacridinyl, a substituted or unsubstituted dibenzoacridinyl, a substituted or unsubstituted dibenzofuranyl, a substituted or unsubstituted carbazolyl, a substituted or unsubstituted benzoquinolinyl, a substituted or unsubstituted phenanthridinyl, a substituted or unsubstituted dinaphthofuranyl or a substituted or unsubstituted dinaphthothiophenyl" .

According to one embodiment, the organic electronic device comprising an anode, a cathode and at least one organic semiconductor layer, wherein the at least one organic semiconductor layer comprises:

- a metal dopant, and
- a compound represented by the following Formula (I):

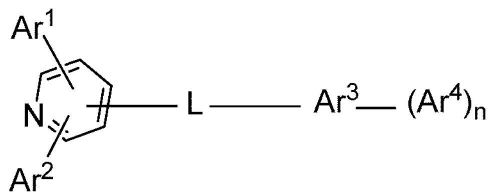

(I)

wherein,

| | |
|---|---|
| $Ar^1$ and $Ar^2$ | may be same or different and independently selected from hydrogen, substituted or unsubstituted $C_6$ to $C_{36}$ aryl, substituted or unsubstituted $C_3$ to $C_{36}$ heteroaryl, wherein, at least one of the $Ar^1$ and $Ar^2$ group comprises a substituted or unsubstituted $C_3$ to $C_{36}$ heteroaryl; |
| L | may be a single bond, a substituted or unsubstituted $C_6$ to $C_{18}$ arylene, a substituted or unsubstituted $C_3$ to $C_{18}$ heteroarylene, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; |
| $Ar^3$ | is a group may be selected from a substituted $C_4$ N-heteroarylene, a substituted or unsubstituted $C_{12}$ heteroarylene comprising at least three fused rings and one hetero atom selected from O, S or N, a substituted pyrazinylene, a substituted pyrimidinylene, a substituted or unsubstituted dibenzofuranylene, a substituted or unsubstituted carbazolylene, a substituted or unsubstituted benzoquinolinylene, a substituted or unsubstituted phenanthridinylene, a substituted or unsubstituted dinaphthofuranylene or a substituted or unsubstituted dinaphthothiophenylene; |
| $Ar^4$ | is a group may be independently selected from H, a substituted or unsubstituted $C_6$ to $C_{18}$ aryl, a substituted or unsubstituted $C_3$ to $C_{20}$ heteroaryl, an unsubstituted phenyl, an unsubstituted biphenyl, an unsubstituted naphthyl, an unsubstituted anthracenyl, an unsubstituted phenanthridinyl, an unsubstituted pyridyl, an unsubstituted quinolinyl, an unsubstituted pryrimidyl, or having the formula (II) or (III), |

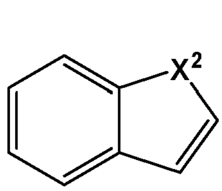 (II), 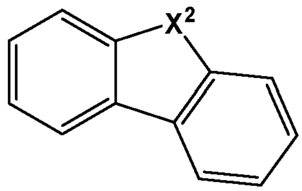 (III),

wherein the group of formula (II) and (III) are substituted or unsubstituted and $X^2$ is selected from N, S,O or $C(R^1)_2$;

n may be 0, 1, 2, 3 or 4;

wherein the substituents of L, $Ar^1$, $Ar^2$, $Ar^3$, $Ar^4$ and/or N may be independently selected from:

- $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $-PX^3(R^2)_2$, D, F or CN;
- $R^1$ and $R^2$ may be independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy and
- two $R^1$ can be linked together to form a ring
- $X^3$ is selected from S or O.

According to one embodiment, the organic electronic device comprising an anode, a cathode and at least one organic semiconductor layer, wherein the at least one organic semiconductor layer comprises:

- a metal dopant, and
- a compound represented by the following Formula (I):

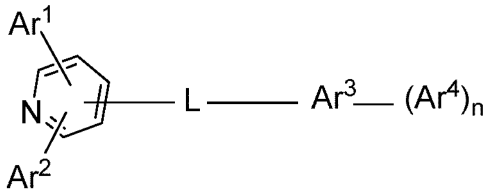

(I)

wherein,

$Ar^1$ and $Ar^2$ may be same or different and independently selected from hydrogen, substituted or unsubstituted $C_6$ to $C_{36}$ aryl, substituted or unsubstituted $C_3$ to $C_{36}$ heteroaryl, wherein, at least one of the $Ar^1$ and $Ar^2$ group comprises a substituted or unsubstituted $C_3$ to $C_{36}$ heteroaryl;

L may be a single bond, a substituted or unsubstituted $C_6$ to $C_{18}$ arylene, a substituted or unsubstituted $C_3$ to $C_{18}$ heteroarylene, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted or unsubstituted acridinylene, a substituted or unsubstituted benzoacridinylene, a substituted or unsubstituted dibenzoacridinylene;

$Ar^4$ is a group may be independently selected from H, a substituted or unsubstituted $C_6$ to $C_{18}$ aryl, a substituted or unsubstituted $C_3$ to $C_{20}$ heteroaryl, an unsubstituted phenyl, an unsubstituted biphenyl, an unsubstituted naphthyl, an unsubstituted anthracenyl, an unsubstituted phenanthridinyl, an unsubstituted pyridyl, an unsubstituted quinolinyl, an unsubstituted pryrimidyl, or having the formula (II) or (III),

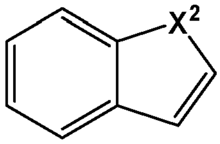 (II), 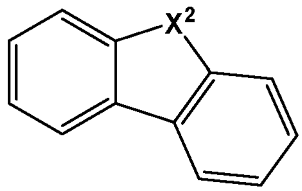 (III),

wherein the group of formula (II) and (III) are substituted or unsubstituted and $X^2$ is selected from N, S,O or $C(R^1)_2$;

n may be 0, 1, 2, 3 or 4;

wherein the substituents o L, $Ar^1$, Ar, Ar, $Ar^4$ and/or N may be independently selected from:

- $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $-PX^3(R^2)_2$, D, F or CN;
- $R^1$ and $R^2$ may be independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy and
- two $R^1$ can be linked together to form a ring
- $X^3$ is selected from S or O.

According to one embodiment, the organic electronic device comprising an anode, a cathode and at least one organic semiconductor layer, wherein the at least one organic semiconductor layer comprises:

- a metal dopant, and
- a compound represented by the following Formula (I):

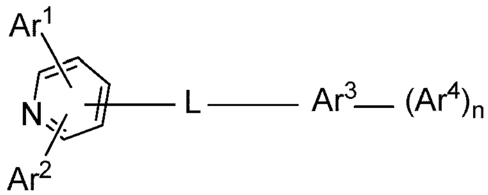

(I)

wherein,

$Ar^1$ and $Ar^2$ may be same or different and independently selected from hydrogen, unsubstituted $C_6$ to $C_{36}$ aryl, unsubstituted $C_3$ to $C_{36}$ heteroaryl, wherein, at least one of the $Ar^1$ and $Ar^2$ group comprises an unsubstituted $C_3$ to $C_{36}$ heteroaryl;

L may be a single bond, an unsubstituted $C_6$ to $C_{18}$ arylene, an unsubstituted $C_3$ to $C_{18}$ heteroarylene, an unsubstituted phenylene, an unsubstituted biphenylene, an unsubstituted heterobiphenylene, an unsubstituted terphenylene, an unsubstituted heteroterphenylene, an unsubstituted anthracenylene, an unsubstituted dibenzofuranylene, an unsubstituted dibenzothiophenylene, an unsubstituted carbazolylene, an unsubstituted pyridinylene, an unsubstituted phenylpyridinylene, an unsubstituted quinolinylene;

$Ar^3$ is a group may be selected from a substituted $C_4$ N-heteroarylene, an unsubstituted $C_{12}$ heteroarylene comprising at least three fused rings and one hetero atom selected from O, S or N, a substituted pyrazinylene, a substituted pyrimidinylene, an unsubstituted acridinylene, an unsubstituted benzoacridinylene, an unsubstituted dibenzoacridinylene, an unsubstituted dibenzofuranylene, an unsubstituted carbazolylene, an unsubstituted benzoquinolinylene, an unsubstituted phenanthridinylene, an unsubstituted dinaphthofuranylene or an unsubstituted dinaphthothiophenylene;

$Ar^4$ is a group may be independently selected from H, a substituted or unsubstituted $C_6$ to $C_{18}$ aryl, a substituted or unsubstituted $C_3$ to $C_{20}$ heteroaryl, an unsubstituted phenyl, an unsubstituted biphenyl, an unsubstituted naphthyl, an unsubstituted anthracenyl, an unsubstituted phenanthridinyl, an unsubstituted pyridyl, an unsubstituted quinolinyl, an unsubstituted pryrimidyl, or having the formula (II) or (III),

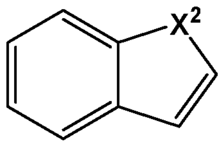 (II), 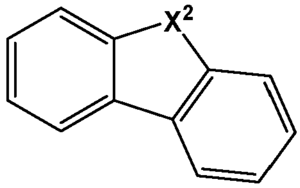 (III),

wherein the group of formula (II) and (III) are substituted or unsubstituted and $X^2$ is selected from N, S,O or $C(R^1)_2$;

n may be 0, 1, 2, 3 or 4;

wherein the substituents of $Ar^4$ may be independently selected from $-PX^3(R^2)_2$, D, F or CN;

- $R^1$ and $R^2$ may be independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy and
- two $R^1$ can be linked together to form a ring
- $X^3$ is selected from S or O.

According to one embodiment, the organic electronic device comprising an anode, a cathode and at least one organic semiconductor layer, wherein the at least one organic semiconductor layer comprises:

- a metal dopant, and
- a compound represented by the following Formula (I):

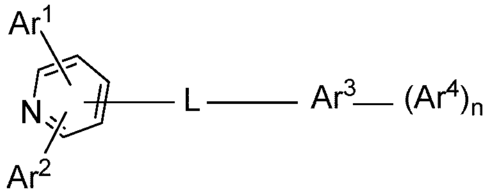

(I)

wherein,

$Ar^1$ and $Ar^2$ may be same or different and independently selected from hydrogen, unsubstituted $C_6$ to $C_{36}$ aryl, unsubstituted $C_3$ to $C_{36}$ heteroaryl, wherein, at least one of the $Ar^1$ and $Ar^2$ group comprises an unsubstituted $C_3$ to $C_{36}$ heteroaryl;

L may be a single bond, an unsubstituted $C_6$ to $C_{18}$ arylene, an unsubstituted $C_3$ to $C_{18}$ heteroarylene, an unsubstituted phenylene, an unsubstituted biphenylene, an unsubstituted heterobiphenylene, an unsubstituted terphenylene, an unsubstituted heteroterphenylene, an unsubstituted anthracenylene, an unsubstituted dibenzofuranylene, an unsubstituted dibenzothiophenylene, an unsubstituted carbazolylene, an unsubstituted pyridinylene, an unsubstituted phenylpyridinylene, an unsubstituted quinolinylene;

$Ar^3$ is a group may be selected from a substituted $C_4$ N-heteroarylene, an unsubstituted $C_{12}$ heteroarylene comprising at least three fused rings and one hetero atom selected from O, S or N, a substituted pyrazinylene, a substituted pyrimidinylene, an unsubstituted acridinylene, an unsubstituted benzoacridinylene, an unsubstituted dibenzoacridinylene, an unsubstituted dibenzofuranylene, an unsubstituted carbazolylene, an unsubstituted benzoquinolinylene, an unsubstituted phenanthridinylene, an unsubstituted dinaphthofuranylene or an unsubstituted dinaphthothiophenylene;

$Ar^4$ is a group may be independently selected from H, a substituted or unsubstituted $C_6$ to $C_{18}$ aryl, a substituted or unsubstituted $C_3$ to $C_{20}$ heteroaryl, an unsubstituted phenyl, an unsubstituted biphenyl, an unsubstituted naphthyl, an unsubstituted anthracenyl;

n may be 0, 1, 2, 3 or 4;

wherein the substituents of $Ar^4$ may be independently selected from $-PX^3(R^2)_2$, D, F or CN;

- $R^2$ may be independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy and
- $X^3$ is selected from S or O.

According to one embodiment, the organic electronic device comprising an anode, a cathode and at least one organic semiconductor layer, wherein the at least one organic semiconductor layer comprises:

- a metal dopant, and
- a compound represented by the following Formula (I):

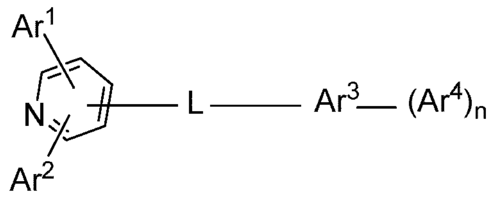

(I)

wherein,

Ar1 and Ar2 — may be same or different and independently selected from hydrogen, unsubstituted $C_6$ to $C_{36}$ aryl, unsubstituted $C_3$ to $C_{36}$ heteroaryl, wherein, at least one of the $Ar^1$ and $Ar^2$ group comprises an unsubstituted $C_3$ to $C_{36}$ heteroaryl;

L — may be a single bond, an unsubstituted $C_6$ to $C_{18}$ arylene, an unsubstituted $C_3$ to $C_{18}$ heteroarylene, an unsubstituted phenylene, an unsubstituted biphenylene, an unsubstituted heterobiphenylene, an unsubstituted terphenylene, an unsubstituted heteroterphenylene, an unsubstituted anthracenylene, an unsubstituted dibenzofuranylene, an unsubstituted dibenzothiophenylene, an unsubstituted carbazolylene, an unsubstituted pyridinylene, an unsubstituted phenylpyridinylene, an unsubstituted quinolinylene;

$Ar^3$ — is a group may be selected from a substituted $C_4$ N-heteroarylene, an unsubstituted $C_{12}$ heteroarylene comprising at least three fused rings and one hetero atom selected from O, S or N, a substituted pyrazinylene, a substituted pyrimidinylene, an unsubstituted acridinylene, an unsubstituted benzoacridinylene, an unsubstituted dibenzoacridinylene, an unsubstituted dibenzofuranylene, an unsubstituted carbazolylene, an unsubstituted benzoquinolinylene, an unsubstituted phenanthridinylene, an unsubstituted dinaphthofuranylene or an unsubstituted dinaphthothiophenylene;

$Ar^4$ — is a group may be independently selected from H, an unsubstituted $C_6$ to $C_{18}$ aryl, an unsubstituted $C_3$ to $C_{20}$ heteroaryl, an unsubstituted phenyl, an unsubstituted biphenyl, an unsubstituted naphthyl, an unsubstituted anthracenyl, an unsubstituted phenanthridinyl, an unsubstituted pyridyl, an unsubstituted quinolinyl, an unsubstituted pryrimidyl, or having the formula (II) or (III),

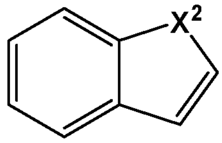

(II),

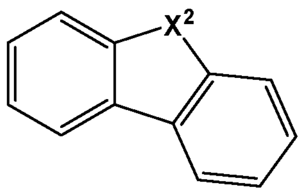

(III),

wherein the group of formula (II) and (III) are unsubstituted and $X^2$ is selected from N, S or O;

n — may be 0, 1, 2, 3 or 4.

According to one embodiment, the organic electronic device comprising an anode, a cathode and at least one organic semiconductor layer, wherein the at least one organic semiconductor layer comprises:

- a metal dopant, and

- a compound represented by the following Formula (I):

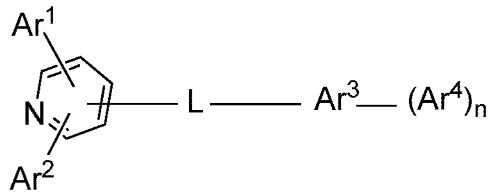

(I)

wherein,

| | |
|---|---|
| $Ar^1$ and $Ar^2$ | may be same or different and independently selected from hydrogen, substituted or unsubstituted $C_6$ to $C_{36}$ aryl, substituted or unsubstituted $C_3$ to $C_{36}$ heteroaryl, wherein, at least one of the $Ar^1$ and $Ar^2$ group comprises a substituted or unsubstituted $C_3$ to $C_{36}$ heteroaryl; |
| L | may be a single bond, a substituted or unsubstituted $C_6$ to $C_{18}$ arylene, a substituted or unsubstituted $C_3$ to $C_{18}$ heteroarylene, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; |
| $Ar^3$ | is a group may be selected from a substituted $C_4$ N-heteroarylene, an unsubstituted $C_{12}$ heteroarylene comprising at least three fused rings and one hetero atom selected from O, S or N, a substituted pyrazinylene, a substituted pyrimidinylene, an unsubstituted acridinylene, an unsubstituted benzoacridinylene, an unsubstituted dibenzoacridinylene, an unsubstituted dibenzofuranylene, an unsubstituted carbazolylene, an unsubstituted benzoquinolinylene, an unsubstituted phenanthridinylene, an unsubstituted dinaphthofuranylene or an unsubstituted dinaphthothiophenylene; |
| $Ar^4$ | is a group may be independently selected from H, unsubstituted $C_6$ to $C_{18}$ aryl, unsubstituted phenyl, unsubstituted biphenyl, unsubstituted naphthyl, an unsubstituted anthracenyl; |
| n | may be 0, 1, 2, 3 or 4; |

wherein the substituents of L, $Ar^1$ and/or $Ar^2$ may be independently selected from:

- $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $-PX^3(R^2)_2$, D, F or CN.

According to another embodiment, the organic semiconductor layer and/or the organic semiconductor layer of the organic electronic device comprises the compound of Formula (I), wherein according to Formula (I) the hetero atom of the $C_3$ to $C_{36}$ heteroaryl, $C_3$ to $C_{24}$ heteroaryl, $C_3$ to $C_{20}$ heteroaryl, $C_3$ to $C_{18}$ heteroarylene, $C_{12}$ heteroarylene, $C_3$ to $C_{12}$ heteroaryl, $C_3$ to $C_{12}$ heteroarylene, may be selected from N, O or S.

According to another embodiment, the organic semiconductor layer and/or the organic semiconductor layer of the organic electronic device comprises the compound of Formula (I), wherein according to Formula (I) the hetero atom of the heteroaryl and/or heteroarylene, may be selected from N or O.

According to another embodiment, the organic semiconductor layer and/or the organic semiconductor layer of the organic electronic device comprises the compound of Formula (I), wherein according to Formula (I) the hetero atom of the heteroaryl and/or heteroarylene, may be selected from N.

According to a further preferred embodiment, wherein the organic semiconductor layer and/or the organic semiconductor layer of the organic electronic device may comprises in addition a metal dopant, and

- a compound represented by the following Formula (I):

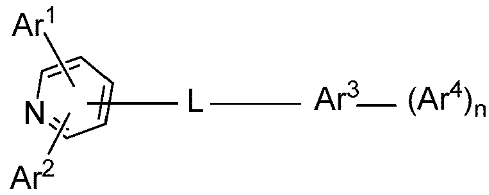

(I)

wherein

Ar¹ and Ar² may be same or different and independently selected from hydrogen, substituted or unsubstituted $C_5$ to $C_{17}$ heteroaryl, wherein, at least one of the $Ar^1$ and $Ar^2$ group comprising at least a substituted or unsubstituted $C_5$ to $C_{17}$ heteroaryl;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted pyrazinylene, a substituted pyrimidinylene, a substituted or unsubstituted benzoacridinylene, a substituted or unsubstituted dibenzoacridinylene, a substituted or unsubstituted dibenzofuranylene, a substituted or unsubstituted carbazolylene, a substituted or unsubstituted benzoquinolinylene, a substituted or unsubstituted phenanthridinylene, a substituted or unsubstituted dinaphthofuranylene or a substituted or unsubstituted dinaphthothiophenylene;

$Ar^4$ is a group may be independently selected from H, an unsubstituted $C_6$ to $C_{12}$ aryl, an unsubstituted $C_3$ to $C_{12}$ heteroaryl, an unsubstituted phenyl, an unsubstituted biphenyl, an unsubstituted naphthyl, an unsubstituted pyridyl, an unsubstituted anthracenyl, or having the formula (II) or (III),

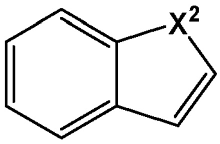

(II),

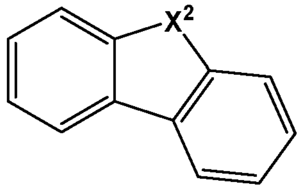

(III),

wherein the group of formula (II) and (III) are substituted or unsubstituted and $X^2$ is selected from N, S or O;

n may be 0, 1, 2, 3 or 4;

wherein the substituents of L, $Ar^1$, $Ar^2$, $Ar^3$ and/or $Ar^4$ may be independently selected from:
$C_6$ to $C_{12}$ aryl, $C_3$ to $C_{17}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $-PX^3(R^2)_2$, D, F or CN, wherein

$R^2$ is independently selected from $C_6$ to $C_{24}$ aryl, $C_3$ to $C_{23}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy and
$X^3$ is selected from S and O.

According to a further preferred embodiment, wherein the organic semiconductor layer and/or the organic semiconductor layer of the organic electronic device may comprises:

- a metal dopant, and
- a compound represented by the following Formula (I):

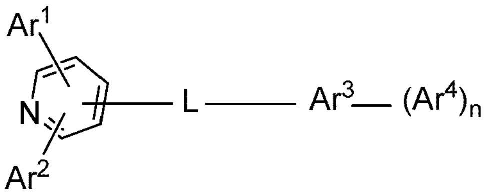

(I)

wherein,

$Ar^1$ and $Ar^2$ may be same or different and independently selected from hydrogen, an unsubstituted $C_5$ to $C_{17}$ heteroaryl, wherein, at least one of the $Ar^1$ and $Ar^2$ group comprising at least an unsubstituted $C_5$ to $C_{17}$ heteroaryl;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted pyrazinylene, a substituted pyrimidinylene, a substituted or unsubstituted benzoacridinylene, a substituted or unsubstituted dibenzoacridinylene, a substituted or unsubstituted benzoquinolinylene, a substituted or unsubstituted phenanthridinylene, a substituted or unsubstituted dinaphthofuranylene or a substituted or unsubstituted dinaphthothiophenylene;

$Ar^4$ is a group may be independently selected from H, an unsubstituted phenyl, an unsubstituted biphenyl, an unsubstituted naphthyl, an unsubstituted pyridyl or having the formula (II) or (III),

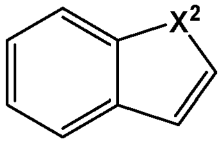

(II),

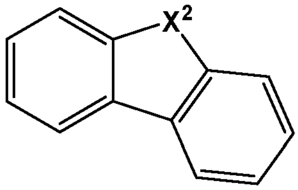

(III),

wherein

$X^2$ is selected from S or O;

n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

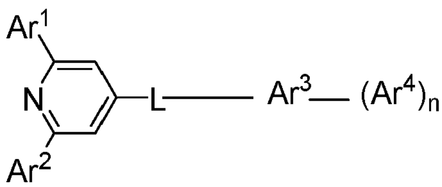

(I)

wherein,

$Ar^1$ and $Ar^2$ are pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted

or unsubstituted phenylpyridinylene, a substituted or unsubstituted quinolinylene;

$Ar^3$ is a group may be selected from a substituted pyrazinylene, a substituted pyrimidinylene, a substituted or unsubstituted benzoacridinylene, a substituted or unsubstituted dibenzoacridinylene, a substituted or unsubstituted benzoquinolinylene, a substituted or unsubstituted phenanthridinylene, a substituted or unsubstituted dinaphthofuranylene or a substituted or unsubstituted dinaphthothiophenylene;

$Ar^4$ is a group may be independently selected from H, an unsubstituted phenyl, an unsubstituted biphenyl, an unsubstituted naphthyl, an unsubstituted pyridyl or having the formula (II) or (III),

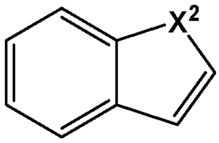
(II),
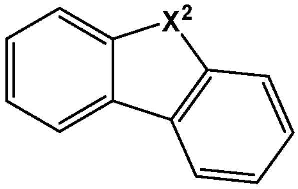
(III),

wherein

$X^2$ is selected from S or O;

n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

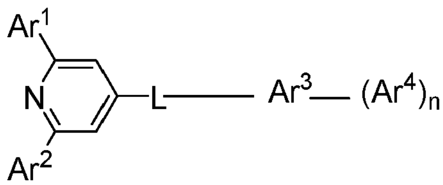
(I)

wherein,

$Ar^1$ is hydrogen and $Ar^2$ is a pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted pyrazinylene, a substituted pyrimidinylene, a substituted or unsubstituted benzoacridinylene, a substituted or unsubstituted dibenzoacridinylene, a substituted or unsubstituted benzoquinolinylene, a substituted or unsubstituted phenanthridinylene, a substituted or unsubstituted dinaphthofuranylene or a substituted or unsubstituted dinaphthothiophenylene;

$Ar^4$ is a group may be independently selected from H, an unsubstituted phenyl, an unsubstituted biphenyl, an unsubstituted naphthyl, an unsubstituted pyridyl or having the formula (II) or (III),

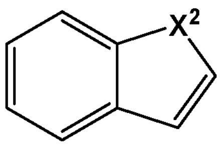
(II),
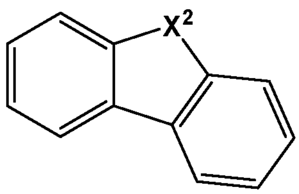
(III),

wherein

$X^2$ is selected from S or O;
n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

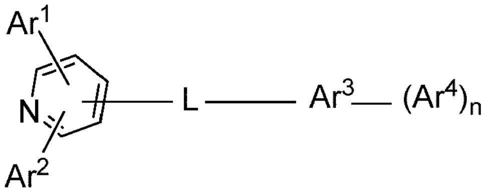

(I)

wherein,

$Ar^1$ and $Ar^2$ are pyridyl group;
L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;
$Ar^3$ is a group may be selected from a substituted pyrazinylene,
$Ar^4$ is a group may be independently selected from H, an unsubstituted phenyl, an unsubstituted biphenyl, an unsubstituted naphthyl, an unsubstituted pyridyl or having the formula (II) or (III),

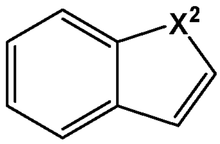

(II),

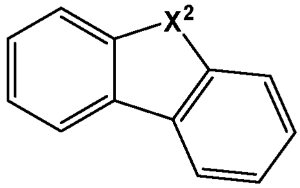

(III),

wherein
$X^2$ is selected from S or O;
n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

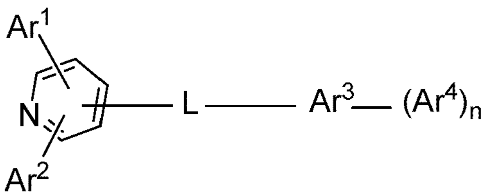

(I)

wherein,

$Ar^1$ is hydrogen and $Ar^2$ is a pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted pyrazinylene,

$Ar^4$ is a group may be independently selected from H, an unsubstituted phenyl, an unsubstituted biphenyl, an unsubstituted naphthyl, an unsubstituted pyridyl or having the formula (II) or (III),

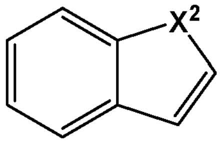 (II), 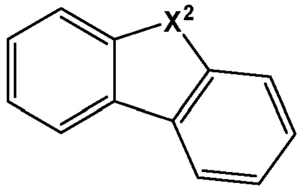 (III),

wherein

$X^2$ is selected from S or O;

n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

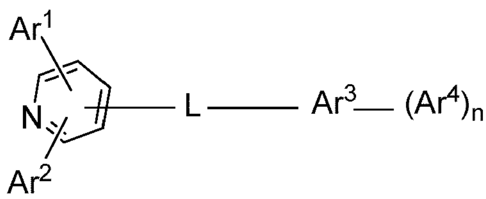

(I)

wherein,

$Ar^1$ and $Ar^2$ are pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted or unsubstituted benzoacridinylene, a substituted or unsubstituted dibenzoacridinylene;

$Ar^4$ is a group may be independently selected from H, an unsubstituted phenyl, an unsubstituted biphenyl, an unsubstituted naphthyl, an unsubstituted pyridyl or having the formula (II) or (III),

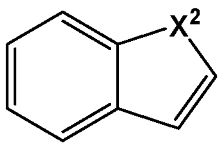 (II), 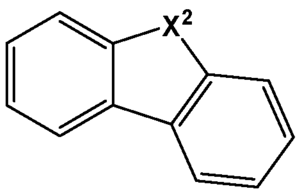 (III),

wherein

$X^2$ is selected from S or O;

n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

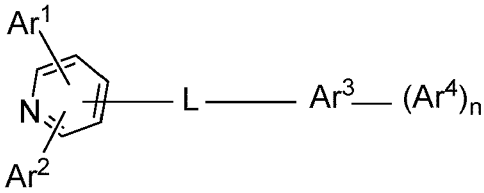

(I)

wherein,

$Ar^1$ is hydrogen and $Ar^2$ is a pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted or unsubstituted benzoacridinylene, a substituted or unsubstituted dibenzoacridinylene;

$Ar^4$ is a group may be independently selected from H, an unsubstituted phenyl, an unsubstituted biphenyl, an unsubstituted naphthyl, an unsubstituted pyridyl or having the formula (II) or (III),

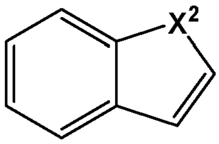

(II),

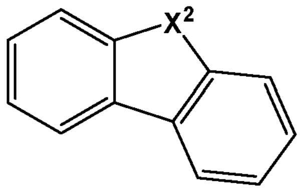

(III),

wherein

$X^2$ is selected from S or O;

n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

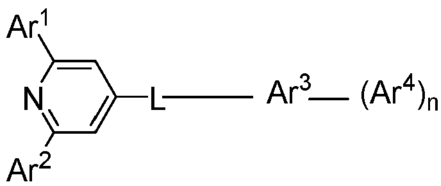

(I)

wherein,

$Ar^1$ and $Ar^2$ are pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted pyrazinylene,

$Ar^4$ is a group may be independently selected from H, an unsubstituted phenyl, an unsubstituted biphenyl, an unsubstituted naphthyl, an unsubstituted pyridyl or having the formula (II) or (III),

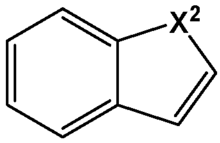
(II),
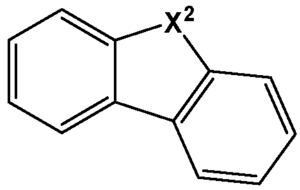
(III),

wherein $X^2$ is selected from S or O;

n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

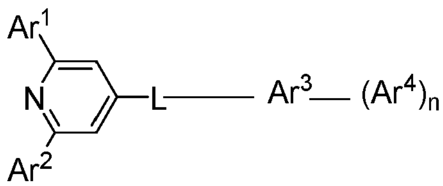
(I)

wherein,

$Ar^1$ is hydrogen and $Ar^2$ is a pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted pyrazinylene,

$Ar^4$ is a group may be independently selected from H, an unsubstituted phenyl, an unsubstituted biphenyl, an unsubstituted naphthyl, an unsubstituted pyridyl or having the formula (II) or (III),

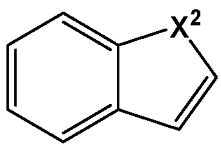
(II),
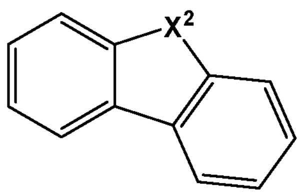
(III),

wherein $X^2$ is selected from S or O;

n may be 0, 1, 2 or 3.

According to a further preferred embodiment, wherein the organic semiconductor layer and/or the organic

semiconductor layer of the organic electronic device may comprises:

- a metal dopant, and
- a compound represented by the following Formula (I):

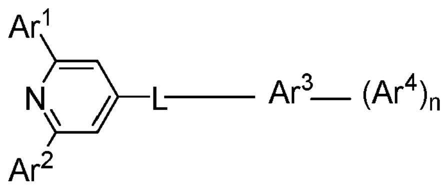

(I)

wherein,

$Ar^1$ and $Ar^2$ are pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted or unsubstituted benzoacridinylene, a substituted or unsubstituted dibenzoacridinylene;

$Ar^4$ is a group may be independently selected from H, an unsubstituted phenyl, an unsubstituted biphenyl, an unsubstituted naphthyl, an unsubstituted pyridyl or having the formula (II) or (III),

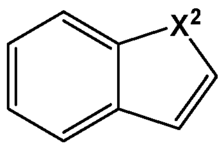

(II),

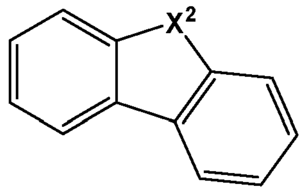

(III),

wherein $X^2$ is selected from S or O;

n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

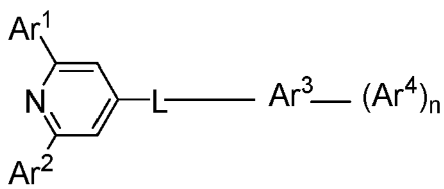

(I)

wherein,

$Ar^1$ is hydrogen and $Ar^2$ is a pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a

substituted or unsubstituted quinolinylene;

$Ar^3$ is a group may be selected from a substituted or unsubstituted benzoacridinylene, a substituted or unsubstituted dibenzoacridinylene;

$Ar^4$ is a group may be independently selected from H, an unsubstituted phenyl, an unsubstituted biphenyl, an unsubstituted naphthyl, an unsubstituted pyridyl or having the formula (II) or (III),

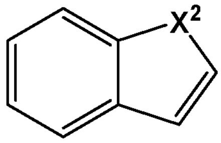
(II),
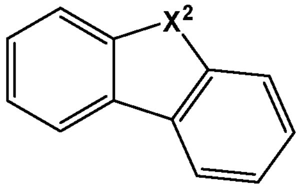
(III),

wherein

$X^2$ is selected from S or O;

n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

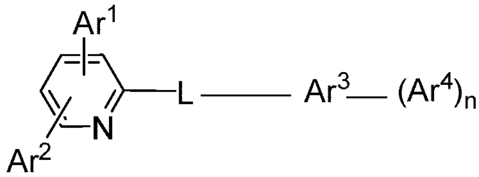
(I)

wherein,

$Ar^1$ and $Ar^2$ are independently selected from hydrogen and a pyridyl group; wherein at least one of $Ar^1$ and $Ar^2$ is a pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted pyrazinylene, a substituted or unsubstituted benzoacridinylene, a substituted or unsubstituted dibenzoacridinylene;

$Ar^4$ is a group may be independently selected from H, an unsubstituted phenyl, an unsubstituted biphenyl, an unsubstituted naphthyl, an unsubstituted pyridyl or having the formula (II) or (III),

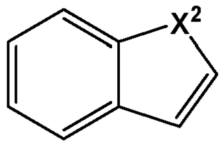
(II),
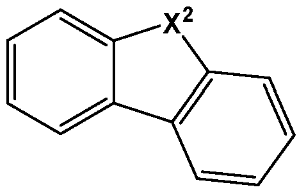
(III),

wherein $X^2$ is selected from S or O;

n may be 0, 1, 2 or 3.

According to a further preferred embodiment, wherein the organic semiconductor layer and/or the organic

semiconductor layer of the organic electronic device may comprises:

- a metal dopant, and
- a compound represented by the following Formula (I):

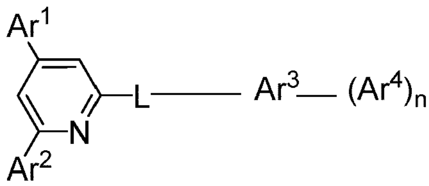

(I)

wherein,

| | |
|---|---|
| $Ar^1$ and $Ar^2$ | are pyridyl group; |
| L | is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; |
| $Ar^3$ | is a group may be selected from a substituted pyrazinylene; |
| $Ar^4$ | is a group may be independently selected from H, an unsubstituted phenyl, an unsubstituted biphenyl, an unsubstituted naphthyl, an unsubstituted pyridyl or having the formula (II) or (III), |

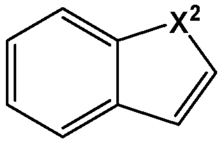

(II),

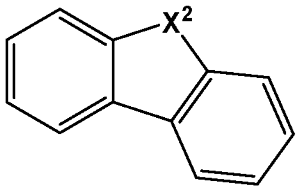

(III),

wherein

| | |
|---|---|
| $X^2$ | is selected from S or O; |
| n | may be 0, 1, 2 or 3. |

According to a further preferred embodiment, wherein the organic semiconductor layer and/or the organic semiconductor layer of the organic electronic device may comprises:

- a metal dopant, and
- a compound represented by the following Formula (I):

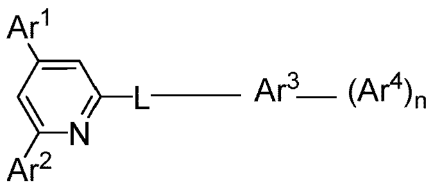

(I)

wherein,

| | |
|---|---|
| $Ar^1$ | is hydrogen and $Ar^2$ is a pyridyl group; |
| L | is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a |

substituted or unsubstituted quinolinylene;

$Ar^3$ is a group may be selected from a substituted pyrazinylene;

$Ar^4$ is a group may be independently selected from H, an unsubstituted phenyl, an unsubstituted biphenyl, an unsubstituted naphthyl, an unsubstituted pyridyl or having the formula (II) or (III),

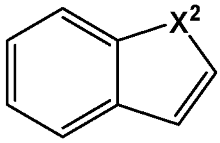
(II),
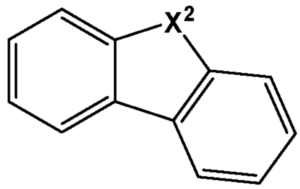
(III),

wherein $X^2$ is selected from S or O;

n may be 0, 1, 2 or 3.

According to a further preferred embodiment, wherein the organic semiconductor layer and/or the organic semiconductor layer of the organic electronic device may comprises:

- a metal dopant, and
- a compound represented by the following Formula (I):

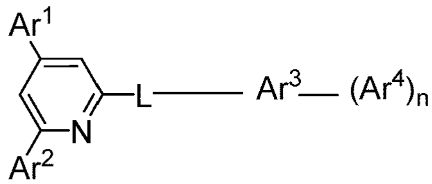
(I)

wherein,

$Ar^1$ and $Ar^2$ are pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted or unsubstituted benzoacridinylene or a substituted or unsubstituted dibenzoacridinylene;

$Ar^4$ is a group may be independently selected from H, an unsubstituted phenyl, an unsubstituted biphenyl, an unsubstituted naphthyl, an unsubstituted pyridyl or having the formula (II) or (III),

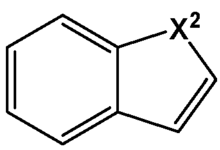
(II),
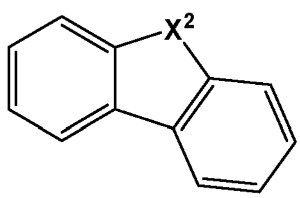
(III),

wherein $X^2$ is selected from S or O;

n may be 0, 1, 2 or 3.

According to a further preferred embodiment, wherein the organic semiconductor layer and/or the organic semiconductor layer of the organic electronic device may comprises:

- a metal dopant, and

- a compound represented by the following Formula (I):

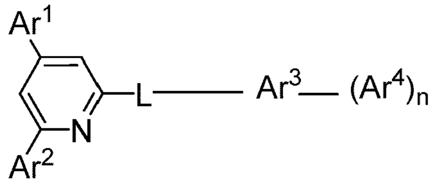

(I)

wherein,

| | |
|---|---|
| $Ar^1$ | is hydrogen and $Ar^2$ is a pyridyl group; |
| L | is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; |
| $Ar^3$ | is a group may be selected from a substituted or unsubstituted benzoacridinylene or a substituted or unsubstituted dibenzoacridinylene; |
| $Ar^4$ | is a group may be independently selected from H, an unsubstituted phenyl, an unsubstituted biphenyl, an unsubstituted naphthyl, an unsubstituted pyridyl or having the formula (II) or (III), |

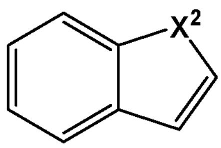

(II),

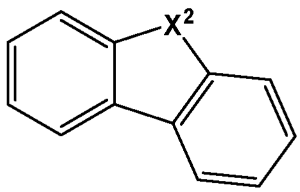

(III),

wherein

| | |
|---|---|
| $X^2$ | is selected from S or O; |
| n | may be 0, 1, 2 or 3. |

According to a further preferred embodiment, wherein a compound represented by the following Formula (I):

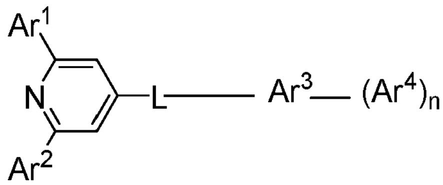

(I)

wherein,

| | |
|---|---|
| $Ar^1$ and $Ar^2$ | are pyridyl group; |
| L | is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; |
| $Ar^3$ | is a group may be selected from a substituted pyrazinylene, |
| $Ar^4$ | is a group may be independently selected from H, an unsubstituted phenyl, an unsubstituted biphenyl, an unsubstituted naphthyl, an unsubstituted pyridyl or having the formula (II) or (III), |

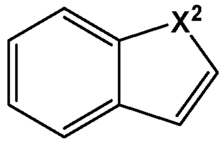
(II),
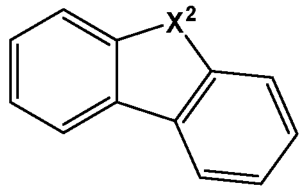
(III),

wherein $X^2$ is selected from S or O;

n may be 0, 1, 2 or 3.

According to a further preferred embodiment, wherein a compound represented by the following Formula (I):

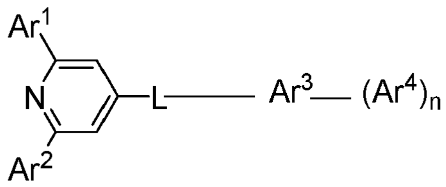
(I)

wherein,

$Ar^1$ is hydrogen and $Ar^2$ is a pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted pyrazinylene,

$Ar^4$ is a group may be independently selected from H, an unsubstituted phenyl, an unsubstituted biphenyl, an unsubstituted naphthyl, an unsubstituted pyridyl or having the formula (II) or (III),

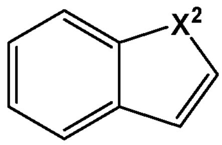
(II),
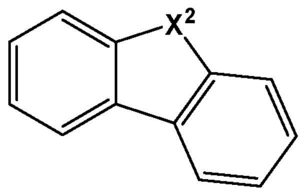
(III),

wherein $X^2$ is selected from S or O;

n may be 0, 1, 2 or 3.

According to a further preferred embodiment, wherein a compound represented by the following Formula (I):

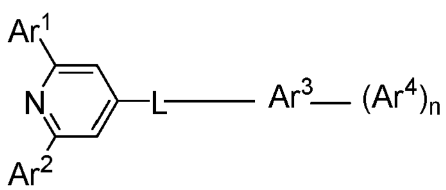
(I)

wherein,

$Ar^1$ and $Ar^2$ are pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted or unsubstituted benzoacridinylene, a substituted or unsubstituted dibenzoacridinylene;

$Ar^4$ is a group may be independently selected from H, an unsubstituted phenyl, an unsubstituted biphenyl, an unsubstituted naphthyl, an unsubstituted pyridyl or having the formula (II) or (III),

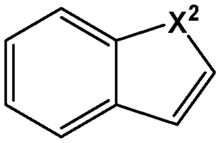 (II), 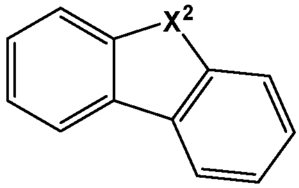 (III),

wherein $X^2$ is selected from S or O;

n may be 0, 1, 2 or 3.

According to a further preferred embodiment, wherein a compound represented by the following Formula (I):

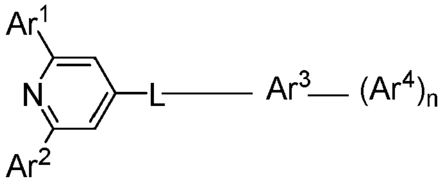

(I)

wherein,

$Ar^1$ is hydrogen and $Ar^2$ is a pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted or unsubstituted benzoacridinylene, a substituted or unsubstituted dibenzoacridinylene;

$Ar^4$ is a group may be independently selected from H, an unsubstituted phenyl, an unsubstituted biphenyl, an unsubstituted naphthyl, an unsubstituted pyridyl or having the formula (II) or (III),

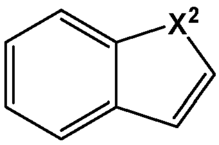 (II), 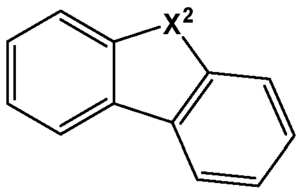 (III),

wherein

$X^2$ is selected from S or O;

n may be 0, 1, 2 or 3.

According to a further preferred embodiment, wherein a compound represented by the following Formula (I):

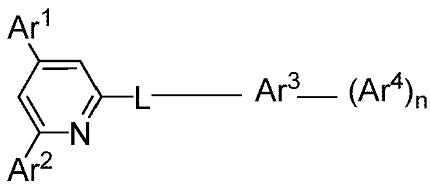

(I)

wherein,

Ar[1] and Ar[2] are pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

Ar[3] is a group may be selected from a substituted pyrazinylene;

Ar[4] is a group may be independently selected from H, an unsubstituted phenyl, an unsubstituted biphenyl, an unsubstituted naphthyl, an unsubstituted pyridyl or having the formula (II) or (III),

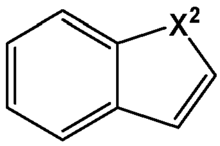

(II),

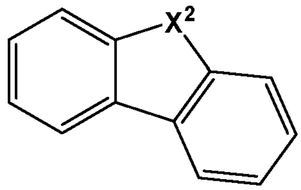

(III),

wherein

$X^2$ is selected from S or O;

n may be 0, 1, 2 or 3.

According to a further preferred embodiment, wherein a compound represented by the following Formula (I):

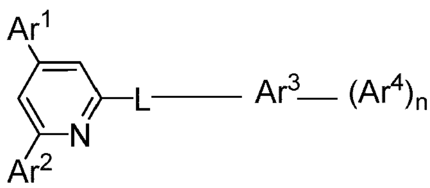

(I)

wherein,

Ar[1] is hydrogen and Ar[2] is a pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

Ar[3] is a group may be selected from a substituted pyrazinylene;

Ar[4] is a group may be independently selected from H, an unsubstituted phenyl, an unsubstituted biphenyl, an unsubstituted naphthyl, an unsubstituted pyridyl or having the formula (II) or (III),

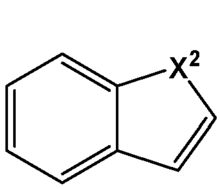 (II), 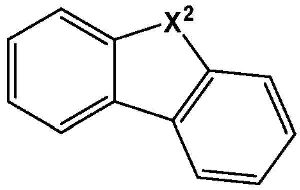 (III),

wherein

$X^2$ is selected from S or O;

n may be 0, 1, 2 or 3.

According to a further preferred embodiment, wherein a compound represented by the following Formula (I):

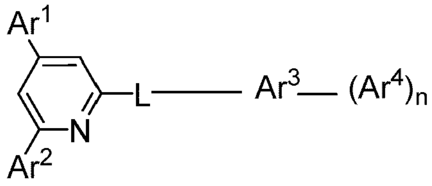

(I)

wherein,

$Ar^1$ and $Ar^2$ are pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted or unsubstituted benzoacridinylene or a substituted or unsubstituted dibenzoacridinylene;

$Ar^4$ is a group may be independently selected from H, an unsubstituted phenyl, an unsubstituted biphenyl, an unsubstituted naphthyl, an unsubstituted pyridyl or having the formula (II) or (III),

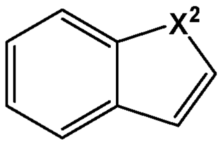 (II), 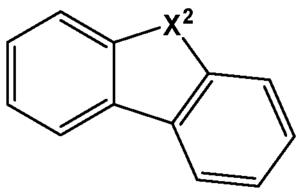 (III),

wherein

$X^2$ is selected from S or O;

n may be 0, 1, 2 or 3.

According to a further preferred embodiment, wherein a compound represented by the following Formula (I):

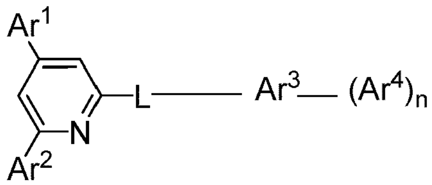

(I)

wherein,

$Ar^1$ is hydrogen and $Ar^2$ is a pyridyl group;

L is a bond, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;

$Ar^3$ is a group may be selected from a substituted or unsubstituted benzoacridinylene or a substituted or unsubstituted dibenzoacridinylene;

$Ar^4$ is a group may be independently selected from H, an unsubstituted phenyl, an unsubstituted biphenyl, an unsubstituted naphthyl, an unsubstituted pyridyl or having the formula (II) or (III),

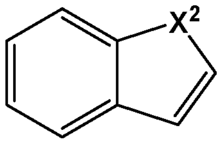 (II), 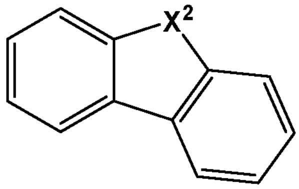 (III),

wherein $X^2$ is selected from S or O;

n may be 0, 1, 2 or 3.

According to another embodiment, the organic semiconductor layer and/or the organic semiconductor layer of the organic electronic device comprises the compound of Formula (I), wherein according to Formula (I) n may be selected 0, 1, 2, 3 or 4, or preferably n may be selected 0, 1, 2 or 3, or further preferred n may be selected 0, 1 or 2, or in addition preferred n may be selected 0 or 1, also preferred n may be selected 2 or 3.

According to another embodiment, the organic semiconductor layer and/or the organic semiconductor layer of the organic electronic device comprises the compound of Formula (I), wherein according to Formula (I) $R^1$, $R^2$ may be independently selected from substituted or unsubstituted $C_1$ to $C_{16}$ alkyl, substituted or unsubstituted $C_6$ to $C_{12}$ aryl, substituted or unsubstituted $C_3$ to $C_{17}$ heteroaryl.

According to another embodiment, the organic semiconductor layer and/or the organic semiconductor layer of the organic electronic device comprises the compound of Formula (I), wherein according to Formula (I) $R^1$, $R^2$ may be independently selected from unsubstituted $C_6$ to $C_{18}$ aryl, or unsubstituted $C_3$ to $C_{24}$ heteroaryl. According to another embodiment, the organic semiconductor layer and/or the organic semiconductor layer of the organic electronic device comprises the compound of Formula (1), wherein according to Formula (I) $R^2$ may be preferably independently selected from methyl.

According to another embodiment, the organic semiconductor layer and/or the organic semiconductor layer of the organic electronic device comprises the compound of Formula (I), wherein according to Formula (I) $Ar^4$ may be independently selected from phenyl, biphenyl, terphenyl, naphthyl, phenanthrenyl, pyridyl, quinolinyl, quinazolinyl.

According to another embodiment, the organic semiconductor layer and/or the organic semiconductor layer of the organic electronic device comprises the compound of Formula (I), wherein according to Formula (I) $Ar^4$ may be independently selected from phenyl, biphenyl, terphenyl, naphthyl, phenanthrenyl.

According to another embodiment, the organic semiconductor layer and/or the organic semiconductor layer of the organic electronic device comprises the compound of Formula (I), wherein according to Formula (I) $Ar^4$ may be independently selected from phenyl and biphenyl and more preferred from phenyl.

According to another embodiment, the organic semiconductor layer and/or the organic semiconductor layer of the organic electronic device comprises the compound of Formula (I), wherein according to Formula (I):

- $Ar^1$ and $Ar^2$ may be independently selected from a hydrogen, phenyl, pyridyl, phenyl pyridyl or quinolinyl group; or
- $Ar^1$ is hydrogen and $Ar^2$ is a pyridyl, phenyl pyridyl or quinolinyl group;
- $Ar^1$ is phenyl and $Ar^2$ is a pyridyl, phenyl pyridyl or quinolinyl group;
- $Ar^1$ is hydrogen and $Ar^2$ is a pyridyl group;
- $Ar^1$ is phenyl and $Ar^2$ is a pyridyl group; or
- $Ar^1$ and $Ar^2$ are pyridyl groups.

According to another embodiment, the organic semiconductor layer and/or the organic semiconductor layer of the organic electronic device comprises the compound of Formula (I), wherein according to Formula (I):
L may be a single bond or is selected from an unsubstituted phenylene, an unsubstituted biphenylene, an unsubstituted

terphenylene, an unsubstituted anthracenylene, an unsubstituted dibenzofuranylene, an unsubstituted dibenzothiophenylene, an unsubstituted pyridinylene, unsubstituted phenylpyridinylene, preferably L may be a single bond or is selected from unsubstituted phenylene or unsubstituted biphenylene.

According to another embodiment, the organic semiconductor layer and/or the organic semiconductor layer of the organic electronic device comprises the compound of Formula (I), wherein according to Formula (I):
L may be selected from unsubstituted phenylene or unsubstituted biphenylene preferably L may be selected from unsubstituted phenylene.

According to another embodiment, the organic semiconductor layer and/or the organic semiconductor layer of the organic electronic device comprises the compound of Formula (I), wherein according to Formula (I) $Ar^3$ is a group may be selected from a substituted pyrazinylene, a substituted pyrimidinylene, an unsubstituted benzoacridinylene or an unsubstituted dibenzoacridinylene.

According to another embodiment, the organic semiconductor layer and/or the organic semiconductor layer of the organic electronic device comprises the compound of Formula (I), wherein according to Formula (I):

- $Ar^4$ may be a group selected from H, an unsubstituted phenyl, an unsubstituted biphenyl, an unsubstituted pyridyl, an unsubstituted naphthyl, an unsubstituted dibenzofuranyl, an unsubstituted benzofuranyl, an unsubstituted dibenzothiophenyl, an unsubstituted benzothiophenyl; or
- H.

According to another embodiment, the organic semiconductor layer and/or the organic semiconductor layer of the organic electronic device comprises the compound of Formula (I), wherein according to Formula (I) L may be independently selected from B1 to B21:

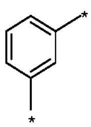, 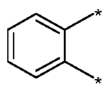, 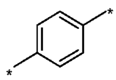, 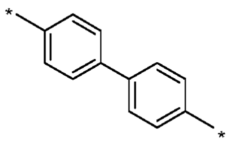, 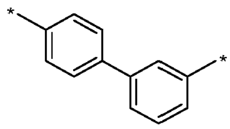, 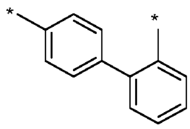,

(B1) (B2) (B3) (B4) (B5), (B6),

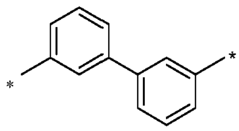, 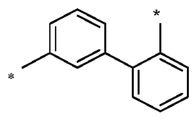, 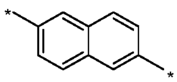, 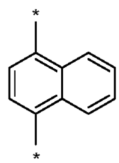, 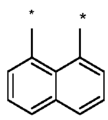,

(B7) (B8) (B9) (B10) (B11),

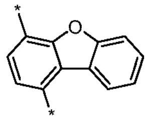, 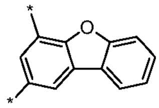, 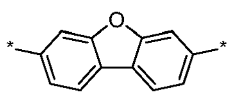, 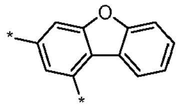, 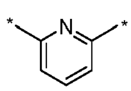, 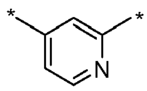,

(B12) (B13) (B14) (B15) (B16), (B17),

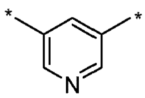, 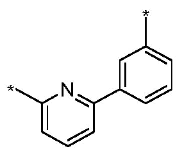, 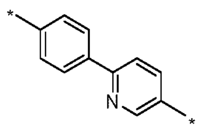, 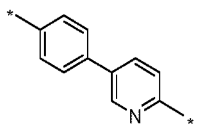,

(B18) (B19) (B20) (B21);

wherein the asterisk symbol "*"

represents the binding position of L.

According to another embodiment, the organic semiconductor layer and/or the organic semiconductor layer of the organic electronic device comprises the compound of Formula (I), wherein according to Formula (I) $Ar^3$ may be independently selected from E1 to E9:

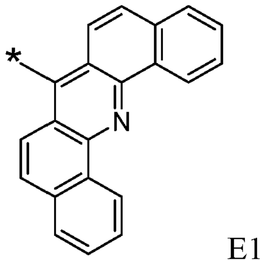
E1,
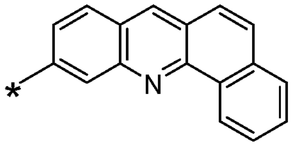
E2,
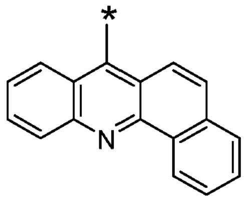
E3,
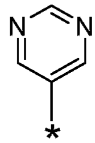
E4,

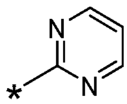
E5,

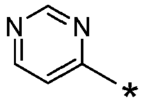
E6,
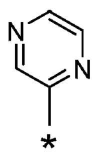
E7,
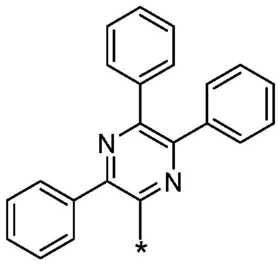
E8,
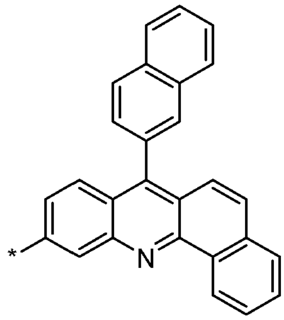
E9,

wherein the asterisk symbol "*" represents the binding position of $Ar^3$ to L and if n = 1, 2, 3 or 4 $Ar^4$ bonds to a carbon of $Ar^3$ excluding the binding position of $Ar^3$ to L.

According to another embodiment, the organic semiconductor layer and/or the organic semiconductor layer of the organic electronic device comprises the compound of Formula (I), wherein according to Formula (I) $Ar^4$ may be independently selected from hydrogen or F1 to F13:

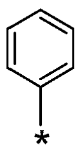
F1,
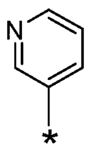
F2,
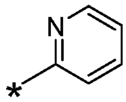
F3,
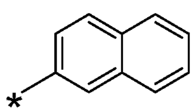
F4,
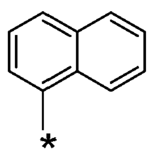
F5,
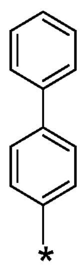
F5,

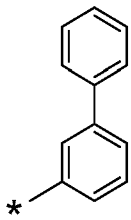 F6, 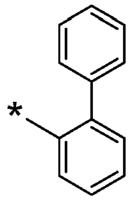 F7, 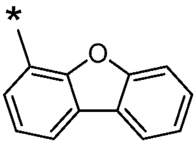 F8, 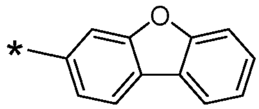 F8,

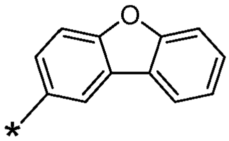 F8, 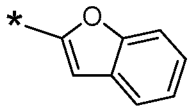 F9, 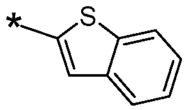 F10, 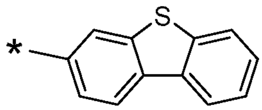 F11,

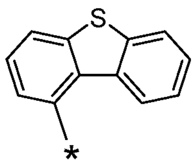 F12, 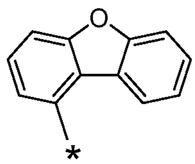 F13;

wherein the asterisk symbol "*" represents the binding position of $Ar^4$ to $Ar^3$, and $Ar^4$ bonds to a carbon of $Ar^3$ excluding the binding position of $Ar^3$ to L.

According to another embodiment, the organic semiconductor layer and/or the organic semiconductor layer of the organic electronic device comprises the compound of Formula (I), wherein according to Formula (I) $Ar^3$-$(Ar^4)_n$ may be independently selected from D1 to D12:

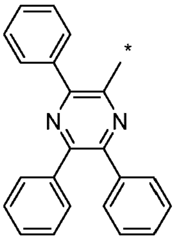 , 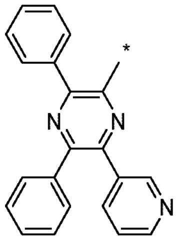 , 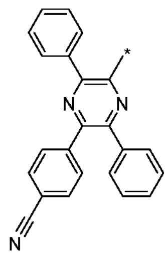 , 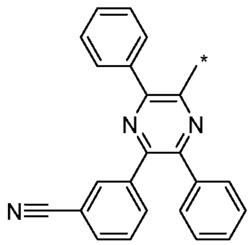 , 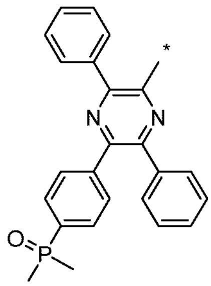 ,

D1, D2 D3 D4 D5

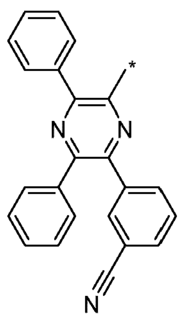 , 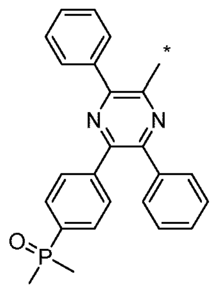 , 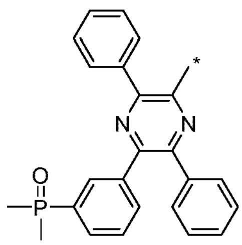 , 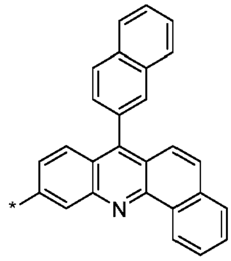 , 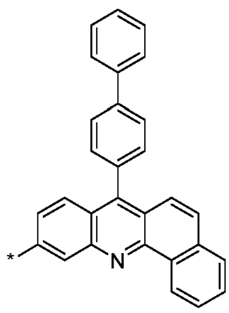

D6, D7, D8 D9 D10

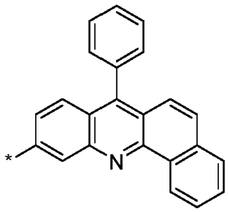,
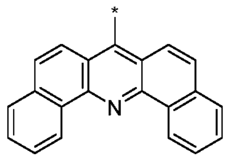

D11 D12;

wherein the asterisk symbol "*" represents the binding position of $Ar^3$ to L.

According to another embodiment, the organic semiconductor layer and/or the organic semiconductor layer of the organic electronic device comprises the compound of Formula (I), wherein the compound of Formula (I) may be selected from G1 to G36:

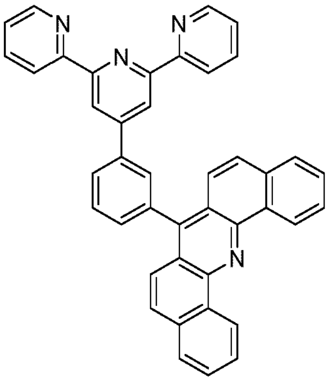,
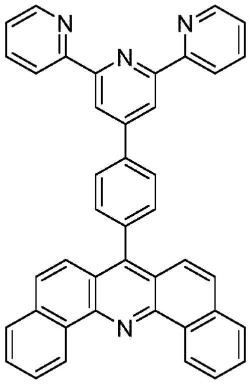,
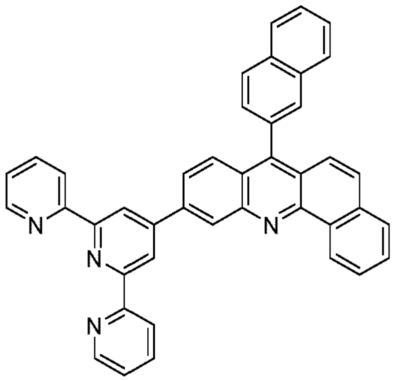

G1 G2 G3

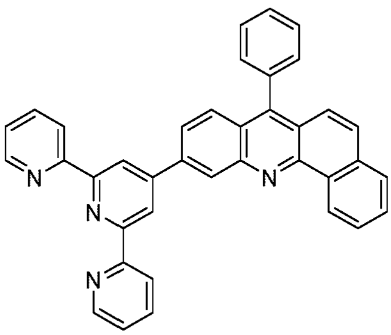,
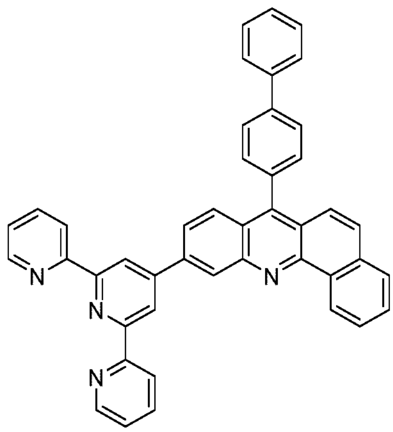,
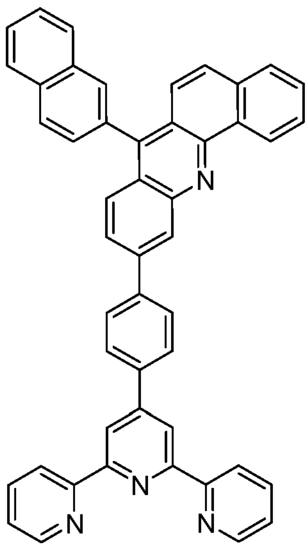,

G4 G5 G6

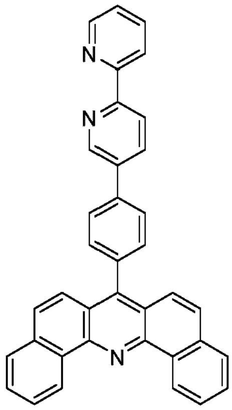
,

G7

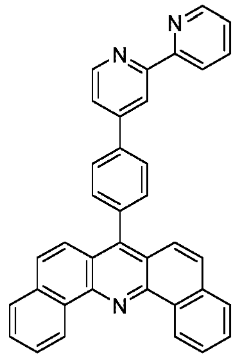
,

G8

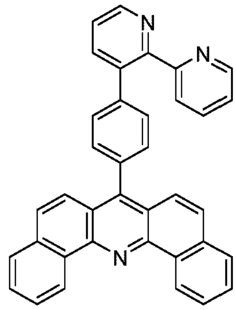
,

G9

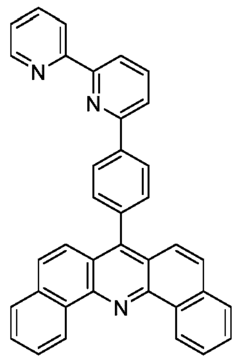
,

G10

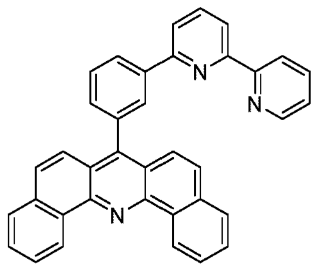
,

G11

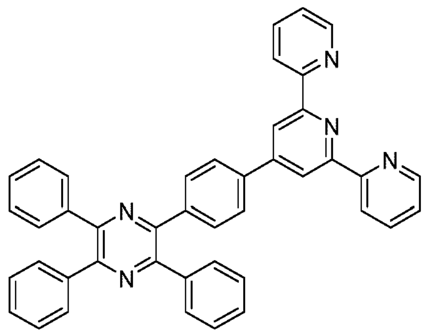
,

G12

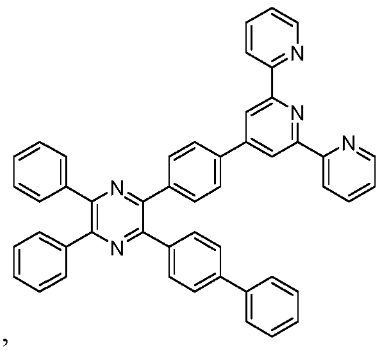
,

G13

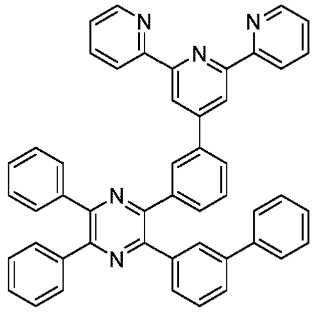
,

G14

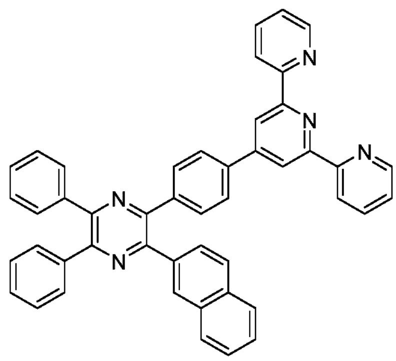
,

G15

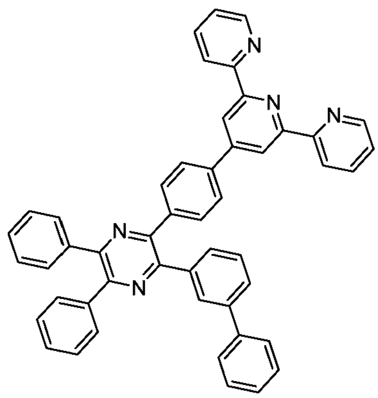
,

G16

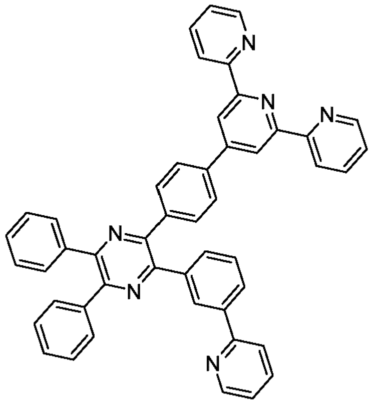,

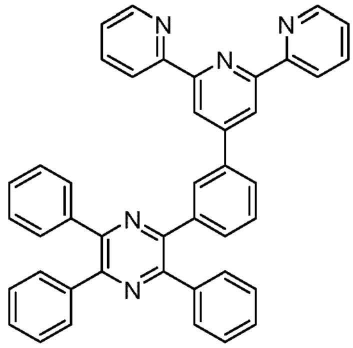,

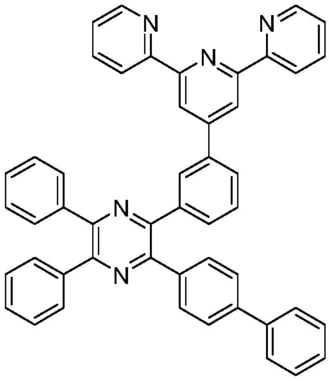,

G17 G18 G19

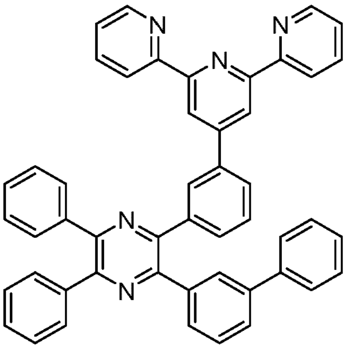,

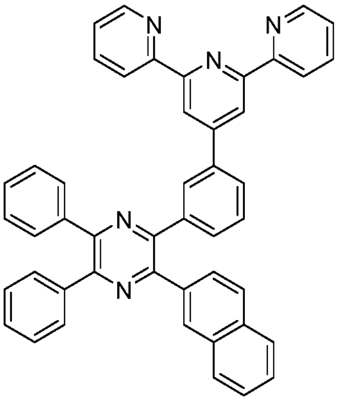,

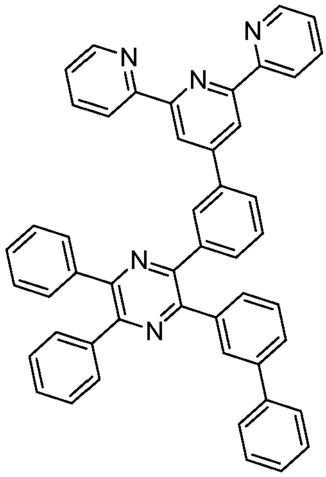,

G20 G21 G22

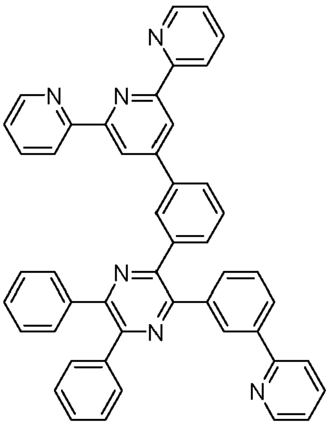,

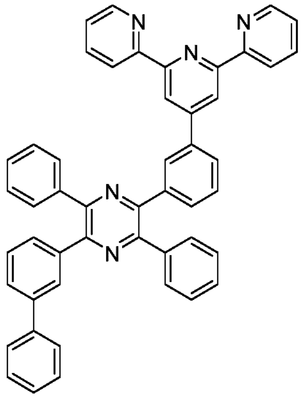,

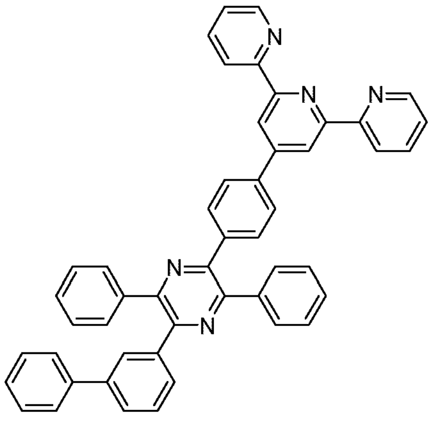

G23 G24 G25

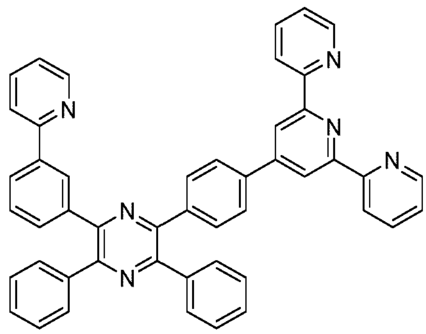

,

G26

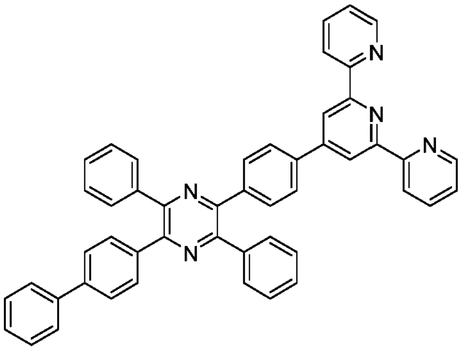

,

G27

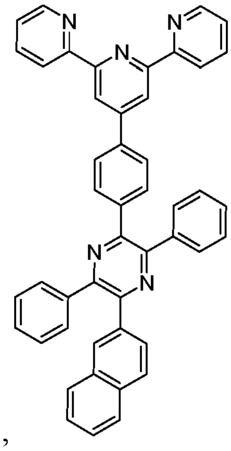

,

G28

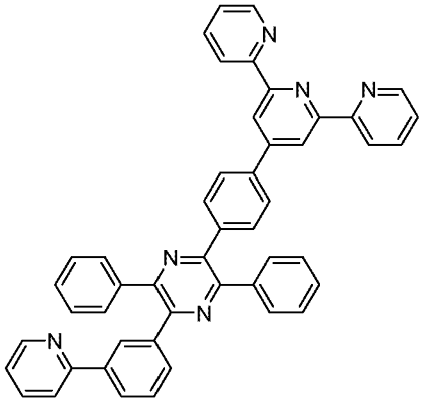

,

G29

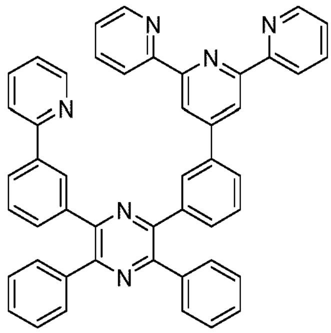

,

G30

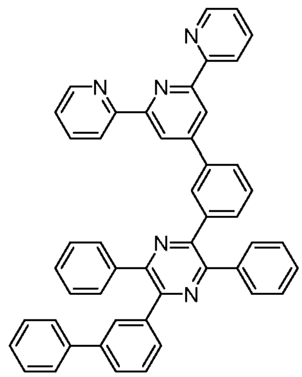

G31

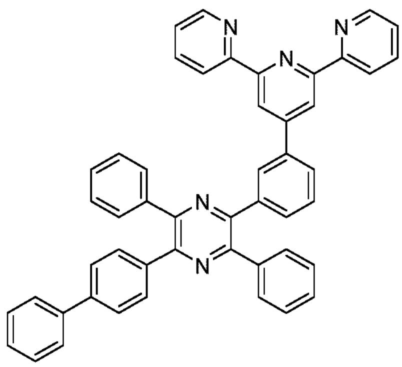

,

G32

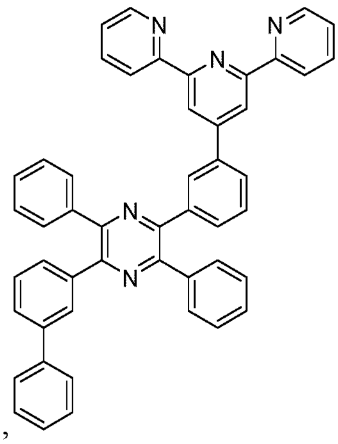

,

G33

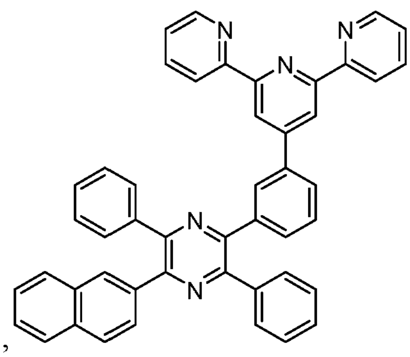

,

G34

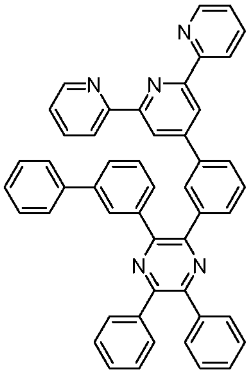,
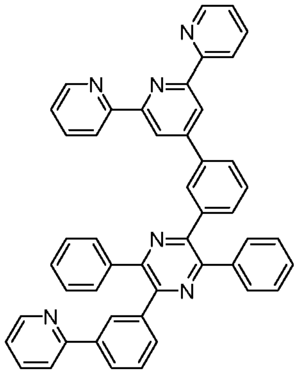

G35 G36.

According to another embodiment, the organic semiconductor layer and/or the organic semiconductor layer of the organic electronic device, wherein the metal of the metal dopant may have an electronegativity of about ≥ 0.7 to about ≤ 1.3 according to Pauling scale, and preferably the metal dopant has an electronegativity of about ≥ 0.9 to about ≤ 1.2, further preferred about ≥ 1 to about ≤ 1.1.

According to another embodiment, the organic semiconductor layer and/or the organic semiconductor layer of the organic electronic device may comprise a metal dopant, wherein the metal dopant is

- a metal selected from the group consisting of alkali metals, alkaline earth metals and rare earth metals;
- a metal selected from Li, Na, Cs, Mg, Ca, Sr, S or Yb;
- a metal selected from Li, Cs, Mg or Yb; or
- a metal selected from Li or Yb.

According to another embodiment, the organic semiconductor layer and/or the organic semiconductor layer of the organic electronic device may comprise a metal dopant, wherein the metal dopant is a metal selected from the group consisting of alkali metals, alkaline earth metals and rare earth metals preferably the metal is selected from Li, Na, Cs, Mg, Ca, Sr, S or Yb, and more preferably the metal is selected from Li, Cs, Mg or Yb. Li, Na, Cs, Mg, Ca, Sr, S or YbLi, Cs, Mg or Yb

According to another embodiment, the organic semiconductor layer and/or the organic semiconductor layer of the organic electronic device may comprise a metal dopant, wherein the metal dopant is a metal selected from Li or Yb.

According to one embodiment, wherein the organic semiconductor layer and/or the organic semiconductor layer of the organic electronic device may consists of a compound of Formula (I) and a metal dopant, wherein the metal dopant is a metal from the group consisting of alkali metals, alkaline earth metals and rare earth metals preferably the metal is selected from Li, Na, Cs, Mg, Ca, Sr, S or Yb, and more preferably the metal is selected from Li, Cs, Mg or Yb Li, Na, Cs, Mg, Ca, Sr, S or YbLi, Cs, Mg or Yb.

According to one embodiment, wherein the organic semiconductor layer and/or the organic semiconductor layer of the organic electronic device may consists of a compound of Formula (I) and a metal dopant, wherein the metal dopant is a metal selected from Li or Yb.

aThe compound of formula (1) and/or the metal diopant may be essentially non-emissive.

According to another aspect an organic semiconductor layer may comprises at least one composition of the present invention.

The organic semiconductor layer comprising the composition of the present invention may be essentially non-emissive.

The thickness of the organic semiconductor layer may be from about 0.5 nm to about 100 nm, for example about 2 nm to about 40 nm. When the thickness of the organic semiconductor layer is within these ranges, the organic semiconductor layer may have improved charge transport ability without a substantial increase in operating voltage.

The organic semiconductor layer comprising the composition of the present invention may have strong electron transport characteristics to increase charge mobility and/or stability.

Compound of Formula (I)

According to another aspect a compound may be represented by the following Formula (I):

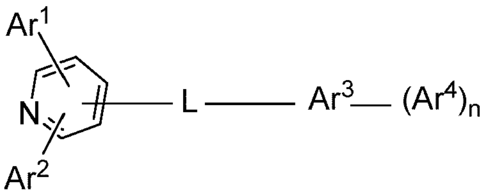

(I),

wherein

| | |
|---|---|
| $Ar^1$ and $Ar^2$ | are same or different and independently selected from hydrogen, substituted or unsubstituted $C_6$ to $C_{36}$ aryl, substituted or unsubstituted $C_3$ to $C_{36}$ heteroaryl, wherein, at least one of the $Ar^1$ and $Ar^2$ group comprises a substituted or unsubstituted $C_3$ to $C_{36}$ heteroaryl; |
| L | is a single bond, a substituted or unsubstituted $C_6$ to $C_{18}$ arylene, a substituted or unsubstituted $C_3$ to $C_{18}$ heteroarylene, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene; |
| $Ar^3$ | is a group selected from substituted pyrazinylene, substituted or unsubstituted dibenzoacridinylene; |
| $Ar^4$ | is a group independently selected from H, a substituted or unsubstituted $C_6$ to $C_{18}$ aryl, a substituted or unsubstituted $C_3$ to $C_{20}$ heteroaryl, an unsubstituted phenyl, an unsubstituted biphenyl, an unsubstituted naphthyl, unsubstituted anthracenyl, an unsubstituted phenanthridinyl, an unsubstituted pyridyl, an unsubstituted quinolinyl, an unsubstituted pryrimidyl, or having the formula (II) or (III), |

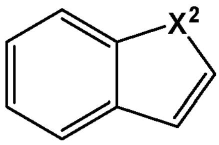

(II),

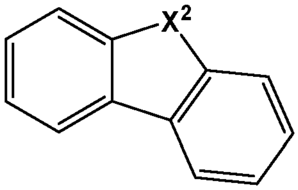

(III),

| | |
|---|---|
| | wherein the group of formula (II) and (III) are substituted or unsubstituted and $X^2$ is selected from N, S,O or $C(R^1)_2$; |
| n | is 0, 1, 2, 3 or 4; |

wherein the substituents of L, $Ar^1$, $Ar^2$, $Ar^3$, $Ar^4$ and/or N are independently selected from:

- $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $-PX^3(R^2)_2$, D, F or CN;
- $R^1$ and $R^2$ are independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy and
- two $R^1$ can be linked together to form a ring
- $X^3$ is selected from S or O.

According to one embodiment, wherein the compound may be represented by the following Formula (I):

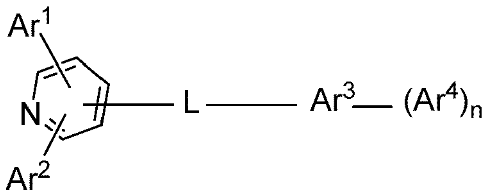

(I)

wherein,

- Ar$^1$ and Ar$^2$ are same or different and independently selected from hydrogen, substituted or unsubstituted $C_6$ to $C_{36}$ aryl, substituted or unsubstituted $C_3$ to $C_{36}$ heteroaryl, wherein, at least one of the Ar$^1$ and Ar$^2$ group comprises a substituted or unsubstituted $C_3$ to $C_{36}$ heteroaryl;
- L is a single bond, a substituted or unsubstituted $C_6$ to $C_{18}$ arylene, a substituted or unsubstituted $C_3$ to $C_{18}$ heteroarylene, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;
- Ar$^3$ is a group selected from, substituted pyrazinylene, substituted or unsubstituted dibenzoacridinylene;
- Ar$^4$ is a group independently selected from H, substituted or unsubstituted $C_6$ to $C_{18}$ aryl, unsubstituted phenyl, unsubstituted biphenyl, unsubstituted naphthyl, unsubstituted anthracenyl,
- n is 0, 1, 2, 3 or 4;

wherein the substituents of L, Ar$^1$, Ar$^2$, Ar$^4$ and/or N are independently selected from:

- $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $-PX^3(R^2)_2$, D, F or CN;
- $R^2$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy
- $X^3$ is selected from S and O;

wherein the substituents of Ar$^3$ and/or N are independently selected from:

$C_6$ to $C_{18}$ aryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl,
partially or perdeuterated $C_1$ to $C_{16}$ alkoxy, $-PX^3(R^2)_2$, D, F or CN.

According to one embodiment, wherein the compound may be represented by the Formula (I), wherein L is selected from an unsubstituted phenylene or an unsubstituted biphenylene.

According to one embodiment, wherein the compound may be represented by the Formula (1), wherein L is selected from an unsubstituted phenylene .

## Electronic device

According to another aspect an electronic device may comprises at least one organic semiconductor layer of the present invention.

According to another aspect an electronic device may comprises at least one anode and at least one cathode, preferably the organic semiconductor layer is arranged between the anode and the cathode.

According to another aspect an electronic device may comprises an anode, a cathode and at least one organic semiconductor layer, wherein the organic electronic device further comprises at least one emission layer and the organic semiconductor layer is arranged between the at least one emission layer and the cathode.

According to another aspect the at least one organic semiconductor layer may be arranged between the at least one emission layer and the cathode, wherein the organic semiconductor layer is in direct contact with the cathode. According to another aspect the organic electronic device may comprises in addition a first and a second emission layer and the organic semiconductor layer is arranged between the at least one emission layer and the cathode, and between a first and a second emission layer. According to another aspect the organic electronic device may comprises in addition a p-type charge generation layer (CGL) and the organic semiconductor layer is arranged between the at least one emission layer and the cathode, and the organic semiconductor layer is in direct contact with a p-type charge generation layer (CGL).

The organic semiconductor layer comprising a compound of Formula (I) and a metal dopant of the present invention may have strong electron transport characteristics to increase charge mobility and/or stability and thereby to

improve luminance efficiency, voltage characteristics, and/or lifetime characteristics of an electronic device.

The electronic device of the present invention may further comprise a photoactive layer, wherein the organic semiconductor layer of the present invention is arranged between the photoactive layer and the cathode layer, preferably between an emission layer or light-absorbing layer and the cathode layer, preferably the organic semiconductor layer is an electron transport layer.

An organic electronic device according to one embodiment comprises the organic semiconductor layer of the present invention, at least one anode layer, at least one cathode layer and at least one emission layer, wherein the organic semiconductor layer is preferably arranged between the emission layer and the cathode layer.

An organic electronic device according to one embodiment can be a light emitting device, thin film transistor, a battery, a display device or a photovoltaic cell, and preferably a light emitting device. A light emitting device can be an OLED.

According to one embodiment the OLED may have the following layer structure, wherein the layers having the following order:

an anode layer, a hole injection layer, optional a first hole transport layer, optional a second hole transport layer, an emission layer, an electron transport layer comprising the compound of Formula (I) and a metal dopant according to the invention, an electron injection layer, and
a cathode layer.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of Formula (I) according to the invention, and
- a second deposition source to release the at least one metal dopant;

the method comprising the steps of forming the electron transport layer stack; whereby for an organic light-emitting diode (OLED):

- the electron transport layer is formed by releasing the compound of Formula (I) from the first deposition source and the metal dopant from the second deposition source.

According to various embodiments of the present invention, the method may further include forming on the anode electrode an emission layer and at least one layer selected from the group consisting of forming a hole injection layer, forming a hole transport layer, or forming a hole blocking layer, between the anode electrode and the organic semiconductor layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, preferably a first electron transport layer is formed on the emission layer and a second electron transport layer is formed on the first electron transport layer and the second electron transport layer comprises the compound of Formula (I) and a metal dopant according to the invention,
- and finally a cathode electrode is formed,
- optional a hole injection layer, a hole transport layer, and a hole blocking layer, formed in that order between the first anode electrode and the emission layer,
- optional an electron injection layer is formed between the electron transport layer stack and the cathode electrode.

According to various embodiments of the present invention, the method may further include forming an electron injection layer on a first electron transport layer. However, according to various embodiments of the OLED of the present invention, the OLED may not comprise an electron injection layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

The term "organic metal complex" means a compound which comprises one or more metal and one or more organic groups. The metal may be bound to the organic group via covalent or ionic bond. The organic group means a group comprising mainly covalently bound carbon and hydrogen atoms. The organic group may further comprise heteroatoms selected from N, O, S, B, Si, P, Se, preferably from B, N, O and S.

In the context of the present specification the term "essentially non-emissive" or "non-emitting" means that the visible emission spectrum from the composition or a layer of the compound of Formula (I) and at least one metal dopant. The visible emission spectrum is an emission spectrum with a wavelength of about ≥ 380 nm to about ≤ 780 nm. Preferably, an organic semiconductor layer or a device comprising a layer, which comprises the compound of Formula (I) and at least one a metal selected from Li, Na, Cs, Mg, Ca, Sr, S or Yb, preferably Li, Cs, Mg or Yb and more preferably Li and Yb.

The term "free of', "does not contain", "does not comprise" does not exclude impurities which may be present in the compounds prior to deposition. Impurities have no technical effect with respect to the object achieved by the present invention.

The operating voltage, also named U, is measured in Volt (V) at 10 milliAmpere per square centimeter (mA/cm2).

The candela per Ampere efficiency, also named cd/A efficiency, is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The rate onset temperature is measured in °C and describes the VTE source temperature at which measurable evaporation of a compound commences at a pressure of less than $10^{-5}$ mbar.

The term "OLED", "organic light emitting diode", "organic light emitting device", "organic optoelectronic device" and "organic light-emitting diode" are simultaneously used and have the same meaning.

The term "transition metal" means and comprises any element in the d-block of the periodic table, which comprises groups 3 to 12 elements on the periodic table.

The term "group III to VI metal" means and comprises any metal in groups III to VI of the periodic table.

As used herein, "weight percent", "wt.-%", "percent by weight", "% by weight", and variations thereof refer to a composition, component, substance or agent as the weight of that composition, component, substance or agent of the respective electron transport layer divided by the total weight of the composition thereof and multiplied by 100. It is understood that the total weight percent amount of all components, substances or agents of the respective electron transport layer are selected such that it does not exceed 100 wt.-%.

As used herein, "volume percent", "vol.-%", "percent by volume", "% by volume", and variations thereof refer to an elemental metal, a composition, component, substance or agent as the volume of that elemental metal, component, substance or agent of the respective electron transport layer divided by the total volume of the respective electron transport layer thereof and multiplied by 100. It is understood that the total volume percent amount of all elemental metal, components, substances or agents of the respective cathode electrode layer are selected such that it does not exceed 100 vol.-%.

All numeric values are herein assumed to be modified by the term "about", whether or not explicitly indicated. As used herein, the term "about" refers to variation in the numerical quantity that can occur.

Whether or not modified by the term "about", the claims include equivalents to the quantities.

It should be noted that, as used in this specification and the appended claims, the singular forms "a", "an", and "the" include plural referents unless the content clearly dictates otherwise.

It should be noted that, as used in this specification and the appended claims, "*" if not otherwise defined indicates the chemical bonding position.

The anode electrode and cathode electrode may be described as anode electrode / cathode electrode or anode electrode / cathode electrode or anode electrode layer / cathode electrode layer.

In the present specification, when a definition is not otherwise provided, an "alkyl group" may refer to an aliphatic hydrocarbon group. The alkyl group may refer to "a saturated alkyl group" without any double bond or triple bond. The alkyl group may be a linear, cyclic or branched alkyl group.

The alkyl group may be a $C_1$ to $C_{16}$ alkyl group, or preferably a $C_1$ to $C_{12}$ alkyl group. More specifically, the alkyl group may be a $C_1$ to $C_{14}$ alkyl group, or preferably a $C_1$ to $C_{10}$ alkyl group or a $C_1$ to $C_6$ alkyl group. For example, a $C_1$ to $C_4$ alkyl group comprises 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a tert-butyl group, a pentyl group, a hexyl group, a cyclopropyl group, a cyclobutyl group, a cyclopentyl group, a cyclohexyl group, and the like.

In the present specification, "aryl" and "arylene group" may refer to a group comprising at least one hydrocarbon aromatic moiety, and all the elements of the hydrocarbon aromatic moiety may have p-orbitals which form conjugation, for example a phenyl group, a naphthyl group, an anthracenyl group, a phenanthrenyl group, a pyrenyl group, a fluorenyl group and the like.

The term "heteroaryl" and "heteroarylene" may refer to aromatic heterocycles with at least one heteroatom, and all the elements of the aromatic heterocycle may have p-orbitals which form conjugation, for example a pyridyl, pyrimidyl, pyrazinyl, triazinyl, pyrrolyl, carbazolyl, furanyl, benzofuranyl, dibenzofuranyl, thiophenyl, benzothiophenyl, dibenzothiophenyl group and the like. Preferably, the aromatic heterocycles are free of $sp^3$-hybridised carbon atoms.

The term "substituted or unsubstituted heteroaryl", "substituted or unsubstituted $C_5$ to $C_{24}$ heteroaryl", "substituted or unsubstituted $C_3$ to $C_{24}$ heteroaryl", "substituted or unsubstituted $C_5$ to $C_{18}$ heteroaryl", "substituted or unsubstituted Cs to $C_{17}$ heteroaryl", "substituted or unsubstituted $C_3$ to $C_{17}$ heteroaryl", "substituted or unsubstituted $C_3$ to $C_{12}$ heteroarylene" and the like means that the substituted or unsubstituted heteroaryl comprises at least one heteroaryl ring; or at least one heteroaryl ring and at least one non-heteroaryl ring; or at least two heteroaryl rings and at least one non-heteroaryl ring; or at least three heteroaryl rings and at least one non-heteroaryl ring; or at least one heteroaryl ring and at least two non-heteroaryl rings. The rings of the substituted or unsubstituted heteroaryl can be a fused.

The term "hetero-fluorene ring" refers to a dibenzo[d,d]furanyl, dibenzo[b,d]thiophenyl or dibenzo[b,d]selenophenyl group.

The heteroatom may be selected from N, O, S, B, Si, P, Se, preferably from N, O and S.

A heteroarylene ring may comprise at least 1 to 3 heteroatoms. Preferably a heteroarylene ring may comprise at least 1 to 3 heteroatoms individually selected from N, S and/or O.

Further preferred in addition to the compounds of Formula (I) at least one additional heteroaryl/ene ring may comprise at least 1 to 3 N-atoms, or at least 1 to 2-N atoms or at least one N-atom.

Further preferred in addition to the compounds of Formula (I) at least one additional heteroaryl/ene ring may comprise at least 1 to 3 O-atoms, or at least 1 to 2 O-atoms or at least one O-atom.

Further preferred in addition to the compounds of Formula (I) at least one additional heteroaryl/ene ring may comprise at least 1 to 3 S-atoms, or at least 1 to 2 S-atoms or at least one S-atom.

According to another preferred embodiment the compound according to Formula (I) may comprise:

- at least 6 to 25 aromatic rings, preferably at least 7 to 22 aromatic rings, further preferred at least 8 to 20 aromatic rings, in addition preferred at least 9 to 15 aromatic rings and more preferred at least 10 to 14 aromatic rings; wherein
- at least 2 to 5, preferably 3 to 4 or 2 to 3 are heteroaromatic rings.

According to one embodiment the compound according to Formula (I):

- comprises at least about 6 to about 20 aromatic rings, preferably at least about 7 to about 18 aromatic rings, further preferred at least about 9 to about 16 aromatic rings, in addition preferred at least about 10 to about 15 aromatic rings and more preferred at least about 11 to about 14 aromatic rings; and/or
- the compound of Formula (I) comprises at least about 2 to about 6, preferably about 3 to about 5 or about 2 to about 4, hetero aromatic rings, wherein the hetero atoms can be selected from N, O, S.

According to one embodiment the compound according to Formula (I) can be free of a fluorene ring and free of a hetero-fluorene ring.

According to one embodiment the compound according to Formula (I) can be free of of a spiro-group.

According to a further preferred embodiment the compound of Formula (I) comprises at least 2 to 7, preferably 2 to 5, or 2 to 3 hetero aromatic rings.

According to a further preferred embodiment the compound of Formula (I) comprises at least 2 to 7, preferably 2 to 5, or 2 to 3 hetero aromatic rings, wherein at least one of the aromatic rings is a five member hetero aromatic ring.

According to a further preferred embodiment the compound of Formula (I) comprises at least 3 to 7, preferably 3 to 6, or 3 to 5 hetero aromatic rings, wherein at least two of the hetero aromatic rings are five member hetero-aromatic-rings.

According to one embodiment the compound according to Formula (I) may comprise at least 6 to 12 non-hetero

aromatic rings and 2 to 3 hetero aromatic rings.

According to one preferred embodiment the compound according to Formula (I) may comprise at least 7 to 12 non-hetero aromatic rings and 2 to 5 hetero aromatic rings.

According to one preferred embodiment the compound according to Formula (I) may comprise at least 7 to 11 non-hetero aromatic rings and 2 to 3 hetero aromatic rings.

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

According to another embodiment the compound of Formula (I) may have a melting point of about ≥ 220° C and about ≤ 380° C, preferably about ≥ 260° C and about ≤ 370° C, further preferred about ≥ 265° C and about ≤ 360° C.

Glass transition temperature

The glass transition temperature is measured under nitrogen and using a heating rate of 10 K per min in a Mettler Toledo DSC 822e differential scanning calorimeter as described in DIN EN ISO 11357, published in March 2010.

According to another embodiment the compound of Formula (I) may have a glass transition temperature Tg of about ≥ 105° C and about ≤ 380° C, preferably about ≥ 110° C and about ≤ 350° C.

Rate onset temperature

The rate onset temperature is determined by loading 100 mg compound into a VTE source. As VTE source a point source for organic materials is used as supplied by Kurt J. Lesker Company (www.lesker.com) or CreaPhys GmbH (http://www.creaphvs.com). The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Ångstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low takt time and decomposition of the organic compound in VTE source may occur due to prolonged exposure to elevated temperatures.

The rate onset temperature is an indirect measure of the volatility of a compound. The higher the rate onset temperature the lower is the volatility of a compound.

According to another embodiment the compound of Formula (I) may have a rate onset temperature $T_{RO}$ of about ≥ 200° C and about ≤ 260° C, preferably about ≥ 220° C and about ≤ 260° C, further preferred about ≥ 220° C and about ≤ 260° C, in addition preferred about ≥ 230° C and about ≤ 255° C.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

According to one embodiment the compounds according to Formula (I) may have a dipole moment (Debye) in the range from about ≥ 0.4 to about ≤ 4, preferably from about ≥ 1.3 to about ≤ 3.8, further preferred from about ≥ 1.4 to about ≤ 3.6.

Calculated HOMO and LUMO

The HOMO and LUMO are calculated with the program package TURBOMOLE V6.5. The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

According to one embodiment the compounds according to Formula (I) may have a LUMO energy level (eV) in the range from about - 2.20 eV to about - 1.50 eV, preferably from about - 2.1 eV to about - 1.70 eV, further preferred from about - 2.08 eV to about - 1.90 eV, also preferred from about - 2.06 eV to about - 1.95 eV.

Technical effect

Surprisingly, it was found that the organic electronic device comprising organic semiconductor layer according to the present invention solve the problem underlying the present invention by being superior over the organic electronic device known in the art, in particular with respect to operating voltage, which is important for reducing power consumption and increasing battery life, for example of a mobile display device. At the same time the cd/A efficiency, also referred to as current efficiency is kept at a similar or even improved level. Long life time at high current density is important for the longevity of a device which is run at high brightness.

Likewise, some compounds falling within the scope of the broadest definition of the present invention have surprisingly be found to be particularly well performing with respect to the mentioned property of glass transition temperature, rate onset temperature and/ or operating voltage in organic electronic devices. These compounds are discussed herein to be particularly preferred.

The beneficial effect of the invention on the performance of organic electronic devices can be seen in Table 3 and Table 4.

Anode

A material for the anode may be a metal or a metal oxide, or an organic material, preferably a material with work function above about 4.8 eV, more preferably above about 5.1 eV, most preferably above about 5.3 eV. Preferred metals are noble metals like Pt, Au or Ag, preferred metal oxides are transparent metal oxides like ITO or IZO which may be advantageously used in bottom-emitting OLEDs having a reflective cathode.

In devices comprising a transparent metal oxide anode or a reflective metal anode, the anode may have a thickness from about 50 nm to about 100 nm, whereas semitransparent metal anodes may be as thin as from about 5 nm to about 15 nm, and non-transparent metal anodes may have a thickness from about 15 nm to about 150nm.

Hole injection layer (HIL)

The hole injection layer may improve interface properties between the anode and an organic material used for the hole transport layer, and is applied on a non-planarized anode and thus may planarize the surface of the anode. For example, the hole injection layer may include a material having a median value of the energy level of its highest occupied molecular orbital (HOMO) between the work function of the anode material and the energy level of the HOMO of the hole transport layer, in order to adjust a difference between the work function of the anode and the energy level of the HOMO of the hole transport layer.

When the hole transport region comprises a hole injection layer 36, the hole injection layer may be formed on the anode by any of a variety of methods, for example, vacuum deposition, spin coating, casting, Langmuir-Blodgett (LB) method, or the like.

When hole injection layer is formed using vacuum deposition, vacuum deposition conditions may vary depending on the material that is used to form the hole injection layer, and the desired structure and thermal properties of the hole injection layer to be formed and for example, vacuum deposition may be performed at a temperature of about 100 °C to about 500 °C, a pressure of about $10^{-6}$ Pa to about $10^{-1}$ Pa, and a deposition rate of about 0.1 to about 10 nm/sec, but the deposition conditions are not limited thereto.

When the hole injection layer is formed using spin coating, the coating conditions may vary depending on the material that is used to form the hole injection layer, and the desired structure and thermal properties of the hole injection layer to be formed. For example, the coating rate may be in the range of about 2000 rpm to about 5000 rpm, and a temperature at which heat treatment is performed to remove a solvent after coating may be in a range of about 80 °C to about 200 °C, but the coating conditions are not limited thereto.

The hole injection layer may further comprise a p-dopant to improve conductivity and/or hole injection from the anode.

p-dopant

In another aspect, the p-dopant may be homogeneously dispersed in the hole injection layer.

In another aspect, the p-dopant may be present in the hole injection layer in a higher concentration closer to the anode and in a lower concentration closer to the cathode.

The p-dopant may be one of a quinone derivative or a radialene compound but not limited thereto. Non-limiting examples of the p-dopant are quinone derivatives such as tetracyanoquinonedimethane (TCNQ), 2,3,5,6-tetrafluoro-tetracyano-1,4-benzoquinonedimethane (F4-TCNQ), 4,4',4"-((1E,1'E,1"E)-cyclopropane-1,2,3-triylidenetris(cyanomethanylylidene))-tris(2,3,5,6-tetrafluorobenzonitrile).

According to another embodiment, an organic electronic device comprising an organic semiconductor layer comprising a composition according to invention may additional comprise a layer comprising a radialene compound and/or a quinodimethane compound.

In another embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have formula (XX) and/or the quinodimethane compound may have formula (XXIa) or (XXIb):

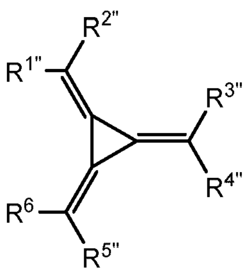
(XX)

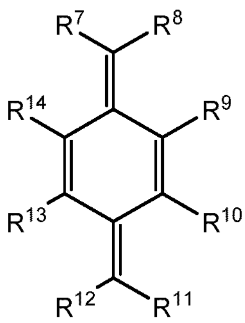
(XXIa)

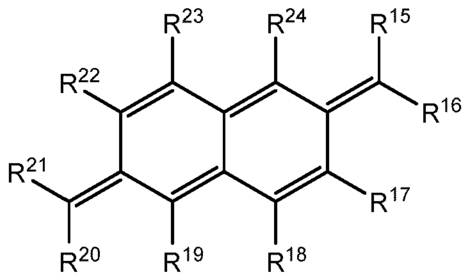

(XXIb),

wherein R1", R2", R3", R4", R5", $R^6$, $R^7$, $R^8$, $R^{11}$, $R^{12}$, $R^{15}$, $R^{16}$, $R^{20}$, $R^{21}$ may be independently selected from an electron withdrawing groups and $R^9$, $R^{10}$, $R^{13}$, $R^{14}$, $R^{17}$, $R^{18}$, $R^{19}$, $R^{22}$, $R^{23}$ and $R^{24}$ may be independently selected from H, halogen and electron withdrawing groups. Electron withdrawing group/s that can be suitable used are above mentioned.

Hole transport layer (HTL)

Conditions for forming the hole transport layer and the electron blocking layer may be defined based on the above-described formation conditions for the hole injection layer.

A thickness of the hole transport part of the charge transport region may be from about 10 nm to about 1000 nm, for example, about 10 nm to about 100 nm. When the hole transport part of the charge transport region comprises the hole injection layer and the hole transport layer, a thickness of the hole injection layer may be from about 10 nm to about 1000 nm, for example about 10 nm to about 100 nm and a thickness of the hole transport layer may be from about 5 nm to about 200 nm, for example about 10 nm to about 150 nm. When the thicknesses of the hole transport part of the charge transport region, the HIL, and the HTL are within these ranges, satisfactory hole transport characteristics may be obtained without a substantial increase in operating voltage.

Hole transport matrix materials used in the hole transport region are not particularly limited. Preferred are covalent compounds comprising a conjugated system of at least 6 delocalized electrons, preferably organic compounds comprising at least one aromatic ring, more preferably organic compounds comprising at least two aromatic rings, even more preferably organic compounds comprising at least three aromatic rings, most preferably organic compounds comprising at least four aromatic rings. Typical examples of hole transport matrix materials which are widely used in hole transport layers are polycyclic aromatic hydrocarbons, triarylene amine compounds and heterocyclic aromatic compounds. Suitable ranges of frontier orbital energy levels of hole transport matrices useful in various layer of the hole transport region are well-known. In terms of the redox potential of the redox couple HTL matrix/ cation radical of the HTL matrix, the preferred values (if measured for example by cyclic voltammetry against ferrocene/ferrocenium redox couple as reference) may be in the range 0.0 - 1.0 V, more preferably in the range 0.2 - 0.7 V, even more preferably in the range 0.3 - 0.5 V.

Buffer layer

The hole transport part of the charge transport region may further include a buffer layer.

Buffer layer that can be suitable used are disclosed in US 6 140 763, US 6 614 176 and in US2016/248022.

The buffer layer may compensate for an optical resonance distance of light according to a wavelength of the light emitted from the EML, and thus may increase efficiency.

Emission layer (EML)

The emission layer may be formed on the hole transport region by using vacuum deposition, spin coating, casting, LB method, or the like. When the emission layer is formed using vacuum deposition or spin coating, the conditions for deposition and coating may be similar to those for the formation of the hole injection layer, though the conditions for the deposition and coating may vary depending on the material that is used to form the emission layer. The emission layer may include an emitter host (EML host) and an emitter dopant (further only emitter).

A thickness of the emission layer may be about 100Å to about 1000Å, for example about 200Å to about 600Å. When the thickness of the emission layer is within these ranges, the emission layer may have improved emission characteristics without a substantial increase in operating voltage.

Emitter host

According to another embodiment, the emission layer comprises compound of Formula (I) as emitter host.

The emitter host compound has at least three aromatic rings, which may be independently selected from carbocyclic rings and heterocyclic rings.

Other compounds that can be used as the emitter host is an anthracene matrix compound represented by formula 400 below:

Formula 400

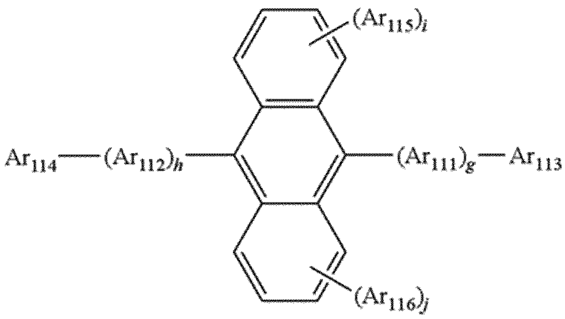

In formula 400, $Ar_{111}$ and $Ar_{112}$ may be each independently a substituted or unsubstituted $C_6$-$C_{60}$ arylene group; $Ar_{113}$ to $Ar_{116}$ may be each independently a substituted or unsubstituted $C_1$-$C_{10}$ alkyl group or a substituted or unsubstituted $C_6$-$C_{60}$ arylene group; and g, h, i, and j may be each independently an integer from 0 to 4.

In some embodiments, Arm and $Ar_{112}$ in formula 400 may be each independently one of a phenylene group, a naphthalene group, a phenanthrenylene group, or a pyrenylene group; or

a phenylene group, a naphthalene group, a phenanthrenylene group, a fluorenyl group, or a pyrenylene group, each substituted with at least one of a phenyl group, a naphthyl group, or an anthryl group.

In formula 400, g, h, i, and j may be each independently an integer of 0, 1, or 2.

In formula 400, $r_{113}$ to $Ar_{116}$ may be each independently one of

- a $C_1$-$C_{10}$ alkyl group substituted with at least one of a phenyl group, a naphthyl group, or an anthryl group;
- a phenyl group, a naphthyl group, an anthryl group, a pyrenyl group, a phenanthrenyl group, or a fluorenyl group;
- a phenyl group, a naphthyl group, an anthryl group, a pyrenyl group, a phenanthrenyl group, or a fluorenyl group, each substituted with at least one of a deuterium atom, a halogen atom, a hydroxyl group, a cyano group, a nitro group, an amino group, an amidino group, a hydrazine group, a hydrazone group, a carboxyl group or a salt thereof,
- a sulfonic acid group or a salt thereof, a phosphoric acid group or a salt thereof,
- a $C_1$-$C_{60}$ alkyl group, a $C_2$-$C_{60}$ alkenyl group, a $C_2$-$C_{60}$ alkynyl group, a $C_1$-$C_{60}$ alkoxy group, a phenyl group, a naphthyl group, an anthryl group, a pyrenyl group, a phenanthrenyl group, or
- a fluorenyl group

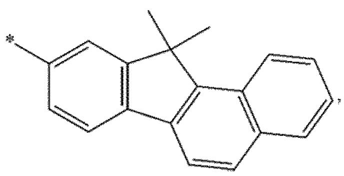

;

or

- formulas 7 or 8

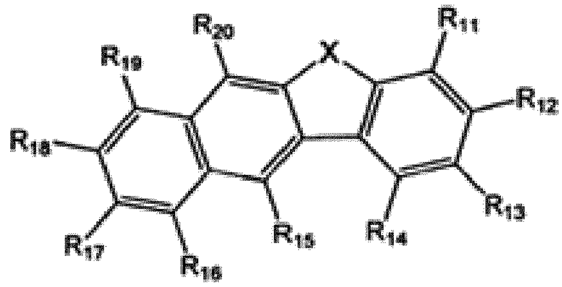

(7),

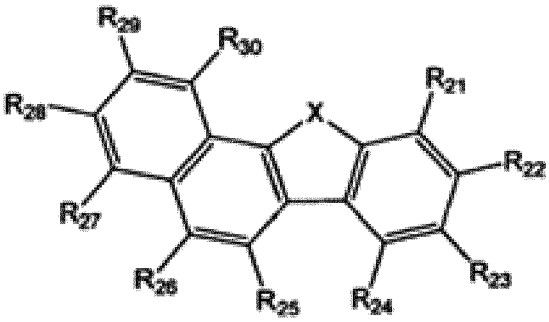

(8).

Wherein in the formulas 7 and 8, X is selected form an oxygen atom and a sulfur atom.

In the formula 7, any one of $R_{11}$ to $R_{14}$ is used for bonding to Arm. $R_{11}$ to $R_{14}$ that are not used for bonding to Arm and $R_{15}$ to $R_{20}$ are the same as $R_1$ to $R_8$.

In the formula 8, any one of $R_{21}$ to $R_{24}$ is used for bonding to Arm. $R_{21}$ to $R_{24}$ that are not used for bonding to Arm and $R_{25}$ to $R_{30}$ are the same as $R_1$ to $R_8$.

Preferably, the EML host comprises between one and three heteroatoms selected from the group consisting of N, O or S. More preferred the EML host comprises one heteroatom selected from S or O.

Emitter dopant

The dopant is mixed in a small amount to cause light emission, and may be generally a material such as a metal complex that emits light by multiple excitation into a triplet or more. The dopant may be, for example an inorganic, organic, or organic/inorganic compound, and one or more kinds thereof may be used.

The emitter may be a red, green, or blue emitter.

The dopant may be a fluorescent dopant, for example ter-fluorene, the structures are shown below. 4.4'-bis(4-diphenyl amiostyryl)biphenyl (DPAVBI, 2,5,8,11-tetra-tert-butyl perylene (TBPe), and Compound 8 below are examples of fluorescent blue dopants.

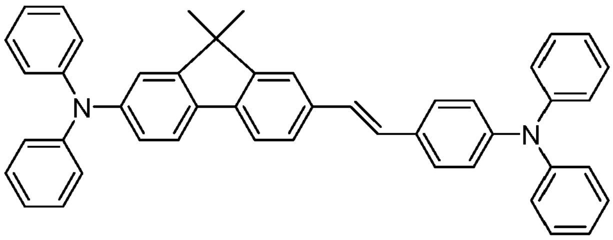

**Compound 8**

The dopant may be a phosphorescent dopant, and examples of the phosphorescent dopant may be an organic metal compound comprising Ir, Pt, Os, Ti, Zr, Hf, Eu, Tb, Tm, Fe, Co, Ni, Ru, Rh, Pd, or a combination thereof. The phosphorescent dopant may be, for example a compound represented by formula Z, but is not limited thereto:

$J_2MX$ (Z).

In formula Z, M is a metal, and J and X are the same or different, and are a ligand to form a complex compound with M.

The M may be, for example Ir, Pt, Os, Ti, Zr, Hf, Eu, Tb, Tm, Fe, Co, Ni, Ru, Rh, Pd or a combination thereof, and the J and X may be, for example a bidendate ligand.

One or more emission layers may be arranged between the anode and the cathode. To increase overall performance, two or more emission layers may be present.

Charge generation layer

A charge generation layer (also named CGL) may be arranged between the first and the second emission layer, and second and third emission layer, if present. Typically, the CGL comprises a n-type charge generation layer (also named n-CGL or electron generation layer) and a p-type charge generation layer (also named p-CGL or hole generation layer). An interlayer may be arranged between the n-type CGL and the p-type CGL.
According to another embodiment, the organic semiconductor layer that comprises the compound of Formula (I) and a metal dopant is an n-type charge generation layer. In another embodiment the n-type charge generation layer may consist of the compound of Formula (I) and a metal dopant according to the invention.
In another aspect, the at least n-type charge generation layer may comprise a compound of Formula (I) and a metal dopant, wherein the metal dopant may be a metal selected from Li, Na, Cs, Mg, Ca, Sr, S or Yb, preferably from Li, Cs, Mg or Yb.
In another aspect, the at least one n-type charge generation layer may comprise a compound of Formula (I) and a metal dopant, wherein the metal dopant may be a metal selected from Li or Yb.
The p-type CGL may comprise a dipyrazino[2,3-f:2',3'-h]quinoxaline, a quinone compound or a radialene compound, preferably dipyrazino[2,3-f:2',3'-h]quinoxaline-2,3,6,7,10,11-hexacarbonitrile or a compound or formula (XX) and/or a compound of formula (XXIa) or (XXIb).

Electron transport layer (ETL)

According to another embodiment, the organic semiconductor layer that comprises the compound of Formula (I) and a metal dopant is an electron transport layer. In another embodiment the electron transport layer may consist of the compound of Formula (I) and a metal dopant according to the invention.
In another embodiment, the organic electronic device comprises an electron transport region of a stack of organic layers formed by two or more electron transport layers, wherein at least one electron transport layer comprises the compound of Formula (I) and a metal dopant according to the present invention.
In another aspect, the at least one electron transport layer may comprise a compound of Formula (I) and a metal dopant, wherein the metal dopant may be a metal selected from Li, Na, Cs, Mg, Ca, Sr, S or Yb, preferably from Li, Cs, Mg or Yb Li, Na, Cs, Mg, Ca, Sr, S or Yb Li, Cs, Mg or Yb.
In another aspect, the at least one electron transport layer may comprise a compound of Formula (I) and a metal dopant, wherein the metal dopant may be a metal selected from Li or Yb.
The thickness of the electron transport layer may be from about 0.5 nm to about 100 nm, for example about 2 nm to about 40 nm. When the thickness of the electron transport layer is within these ranges, the electron transport layer may have improved electron transport ability without a substantial increase in operating voltage.

Electron injection layer (EIL)

According to another aspect of the invention, the organic electronic device may further comprise an electron injection layer between the electron transport layer (first-ETL) and the cathode.
The electron injection layer (EIL) may facilitate injection of electrons from the cathode.
According to another aspect of the invention, the electron injection layer comprises:

(i) an electropositive metal selected from alkali metals, alkaline earth metals and rare earth metals in substantially elemental form, preferably selected from Li, Na, K, Rb, Cs, Mg, Ca, Sr, Ba, Eu and Yb, more preferably from Li, Na, Mg, Ca, Sr and Yb, even more preferably from Li or Yb, most preferably Yb; and/or
(ii) an alkali metal complex and/or alkali metal salt, preferably the Li complex and/or salt, more preferably a Li quinolinolate, even more preferably a lithium 8-hydroxyquino-linolate.

The electron injection layer may include at least one selected from LiF, NaCl, CsF, $Li_2O$, and BaO.
A thickness of the EIL may be from about 0.1 nm to about 10 nm, or about 0.3 nm to about 9 nm. When the thickness of the electron injection layer is within these ranges, the electron injection layer may have satisfactory electron injection ability without a substantial increase in operating voltage.
The electron injection layer may comprise or consist of the compound of Formula (I) and a metal dopant according to the invention .

Cathode

A material for the cathode may be a metal, an alloy, or an electrically conductive compound that have a low

work function, or a combination thereof. Specific examples of the material for the cathode may be lithium (Li), magnesium (Mg), aluminum (Al), aluminum-lithium (Al-Li), calcium (Ca), magnesium-indium (Mg-In), magnesium-silver (Mg-Ag), silver (Ag) etc. In order to manufacture a top-emission light-emitting device having a reflective anode deposited on a substrate, the cathode may be formed as a light-transmissive electrode from, for example, indium tin oxide (ITO), indium zinc oxide (IZO) or silver (Ag).

In devices comprising a transparent metal oxide cathode or a reflective metal cathode, the cathode may have a thickness from about 50 nm to about 100 nm, whereas semitransparent metal cathodes may be as thin as from about 5 nm to about 15 nm.

Substrate

A substrate may be further disposed under the anode or on the cathode. The substrate may be a substrate that is used in a general organic light emitting diode and may be a glass substrate or a transparent plastic substrate with strong mechanical strength, thermal stability, transparency, surface smoothness, ease of handling, and water resistance.

Hereinafter, the embodiments are illustrated in more detail with reference to examples.

BRIEF DESCRIPTION OF THE DRAWINGS

These and/or other aspects and advantages of the present invention will become apparent and more readily appreciated from the following description of the exemplary embodiments, taken in conjunction with the accompanying drawings, of which:

FIG. 1 is a schematic sectional view of an organic light-emitting diode (OLED), according to an exemplary embodiment of the present invention;

FIG. 2 is a schematic sectional view of an OLED, according to an exemplary embodiment of the present invention.

FIG. 3 is a schematic sectional view of a tandem OLED comprising a charge generation layer, according to an exemplary embodiment of the present invention.

FIG. 4 is a schematic sectional view of a tandem OLED comprising a charge generation layer, according to an exemplary embodiment of the present invention.

DETAILED DESCRIPTION

Reference will now be made in detail to the exemplary embodiments of the present invention, examples of which are illustrated in the accompanying drawings, wherein like reference numerals refer to the like elements throughout. The exemplary embodiments are described below, in order to explain the aspects of the present invention, by referring to the figures.

Herein, when a first element is referred to as being formed or disposed "on" a second element, the first element can be disposed directly on the second element, or one or more other elements may be disposed there between. When a first element is referred to as being formed or disposed "directly on" a second element, no other elements are disposed there between.

FIG. 1 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160, an electron injection layer (EIL) 180 and a cathode 190. The electron transport layer (ETL) 160 comprises or consists of the compound of Formula (I) and a metal dopant.

Instead of a single electron transport layer 160, optional an electron transport layer stack (ETL) can be used. The electron transport layer stack (ETL) comprises a first electron transport layer and a second electron transport layer, wherein the first electron transport layer is arranged near to EML and the second electron transport layer is arranged near to the cathode (190). The first and/or the second electron transport layer comprise the compound of Formula (I) and a metal dopant according to the invention.

Fig. 2 is a schematic sectional view of an OLED 100, according to another exemplary embodiment of the present invention. Fig. 2 differs from Fig. 1 in that the OLED 100 of Fig. 2 comprises an electron blocking layer (EBL) 145 and a hole blocking layer (HBL) 155. Referring to Fig. 2 the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode electrode 190. The electron transport layer (ETL) 160 and/or the electron injection layer (EIL) 180 comprise or consist of the compound of Formula (I) and metal dopant.

In the description above the method of manufacture an OLED 100 of the present invention is started with a

substrate 110 onto which an anode 120 is formed, on the anode electrode 120, an hole injection layer 130, hole transport layer 140, optional an electron blocking layer 145, an emission layer 150, optional a hole blocking layer 155, optional at least one electron transport layer 160, optional at least one electron injection layer 180, and a cathode 190 are formed, in that order or the other way around.

Fig. 3 is a schematic sectional view of an OLED 200, according to another exemplary embodiment of the present invention. Fig. 3 differs from Fig. 2 in that the OLED 100 of Fig. 3 further comprises a charge generation layer and a second hole transport layer (HTL) 141. Referring to Fig. 3 the OLED 200 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185 which may comprise compound of Formula (I) and a metal dopant, a p-type charge generation layer (p-type GCL) 135, a second hole transport layer (HTL) 141 and a cathode 190. The electron transport layers (ETL) 160 and 161 and/or the electron injection layer (EIL) 180 and/or the n-type charge generation layer (n-type CGL) 185 comprise or consist of the compound of Formula (I) and a metal dopant.

Fig. 4 is a schematic sectional view of a tandem OLED 200, according to another exemplary embodiment of the present invention. Fig. 4 differs from Fig. 2 in that the OLED 100 of Fig. 3 further comprises a charge generation layer and a second emission layer. Referring to Fig. 4 the OLED 200 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185 which may comprise compound of Formula (I) and a metal dopant, a p-type charge generation layer (p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190. The electron transport layers (ETL) 160 and 161 and/or the electron injection layer (EIL) 180 and/or the n-type charge generation layer (n-type CGL) 185 comprise or consist of the compound of Formula (I) and a metal dopant.

In the description above the method of manufacture an OLED 200 of the present invention is started with a substrate 110 onto which an anode 120 is formed, on the anode electrode 120, a first hole injection layer 130, first hole transport layer 140, optional a first electron blocking layer 145, a first emission layer 150, optional a first hole blocking layer 155, optional at least one first electron transport layer 160, an n-type CGL 185, a p-type CGL 135, a second hole transport layer 141, optional a second electron blocking layer 146, a second emission layer 151, an optional second hole blocking layer 156, an optional at least one second electron transport layer 161, an optional a second electron injection layer (EIL) 181 and a cathode 190 are formed, in that order or the other way around.

Embodiments

Hereinafter, the embodiments are illustrated in more detail with reference to examples. Reference will now be made in detail to the exemplary aspects.

Preparation of compounds of Formula (I)

General procedure 1 for the Synthesis of compound of Formula (I)

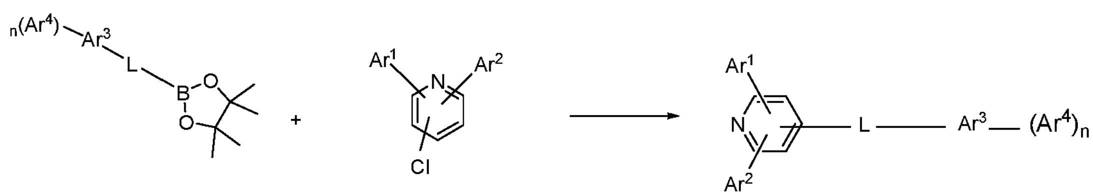

Reagent 1

Reagent 2

Compound of Formula (I)

To synthesize compound of Formula (I) a flask was flushed with nitrogen and charged with reagent 1 (1.1 equiv.), reagent 2 (1 equiv.), $K_2CO_3$ (2 equiv.) dissolved in deaerated water), [1,1'-Bis(diphenylphosphino)ferrocene]dichloropalladium(II) (2 mol%). A deaerated mixture of toluene and ethanol (5:1) was added and the reaction mixture was heated to reflux under a nitrogen atmosphere while monitoring with TLC. After cooling down to room temperature, the resulting precipitate was isolated by suction filtration and washed with water (100 mL) and methanol (30mL). For further purification the crude product was then dissolved in dichloromethane and filtered through a pad of

silicagel. After rinsing with additional dichloromethane, the filtrate was concentrated under reduced pressure and precipitated from methanol (150 mL). The resulting precipitate was recrystallized from 100mL toluene. The formed precipitate was collected by suction filtration and washed with small portion of toluene to give compound of Formula (I). An additional crop is isolated by precipitation by concentration of the toluene mother liquor Final purification was achieved by sublimation.

**General procedure 2 for the Synthesis of compound of Formula (I)**

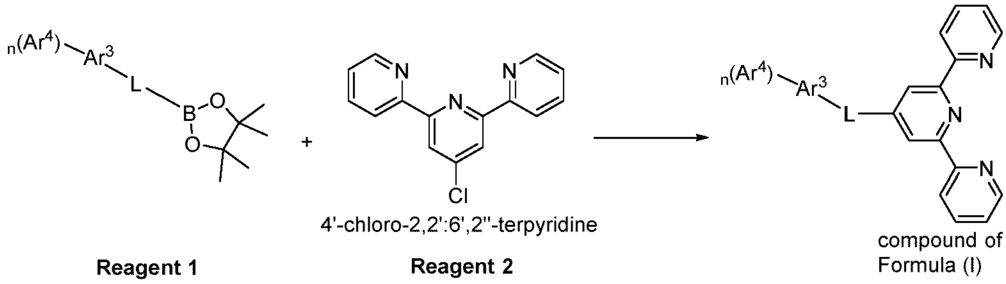

4'-chloro-2,2':6',2"-terpyridine

Reagent 1 Reagent 2 compound of Formula (I)

To synthesize compound of Formula (I) a flask was flushed with nitrogen and charged with reagent (1) (1.1 equiv.), 4'-chloro-2,2':6',2"-terpyridine (2) (1 equiv.), $K_2CO_3$ (2 equiv.) dissolved in deaerated water), [1,1'-Bis(diphenylphosphino)ferrocene]dichloropalladium(II) (2 mol%). A deaerated mixture of toluene and ethanol (5:1) was added and the reaction mixture was heated to reflux under a nitrogen atmosphere while monitoring with TLC. After cooling down to room temperature, the resulting precipitate was isolated by suction filtration and washed with water (100 mL) and methanol (30mL). For further purification the crude product was then dissolved in dichloromethane and filtered through a pad of silicagel. After rinsing with additional dichloromethane, the filtrate was concentrated under reduced pressure and precipitated from methanol (150 mL). The resulting precipitate was recrystallized from 100mL toluene. The formed precipitate was collected by suction filtration and washed with small portion of toluene to give compound of Formula (I). An additional crop is isolated by precipitation by concentration of the toluene mother liquor Final purification was achieved by sublimation.

**General procedure 3 for the Synthesis of compound of Formula (I)**

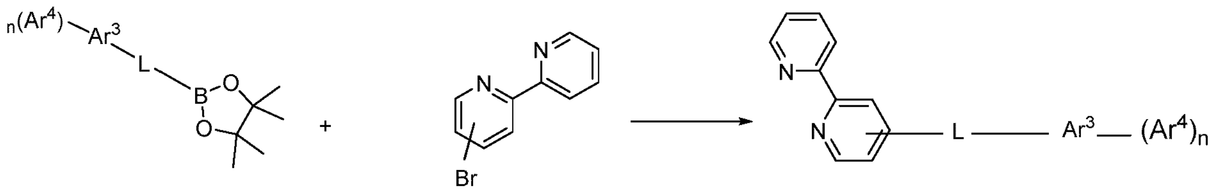

Compound of Formula (I)

Reagent 1 Reagent 2

To synthesize compound of Formula (I) a flask was flushed with nitrogen and charged with reagent (1) (1.1 equiv.), Reagent 2 (1 equiv.), $K_2CO_3$ (2 equiv.) dissolved in deaerated water), [1,1'-Bis(diphenylphosphino)ferrocene]dichloropalladium(II) (2 mol%). A deaerated mixture of toluene and ethanol (5:1) was added and the reaction mixture was heated to reflux under a nitrogen atmosphere while monitoring with TLC. After cooling down to room temperature, the resulting precipitate was isolated by suction filtration and washed with water (100 mL) and methanol (30mL). For further purification the crude product was then dissolved in dichloromethane and filtered through a pad of silicagel. After rinsing with additional dichloromethane, the filtrate was concentrated under reduced pressure and precipitated from methanol (150 mL). The resulting precipitate was recrystallized from 100mL toluene. The formed precipitate was collected by suction filtration and washed with small portion of toluene to give compound of Formula (I). An additional crop is isolated by precipitation by concentration of the toluene mother liquor Final purification was achieved by sublimation.

**General procedure 4 for the Synthesis of compound of Formula (I)**

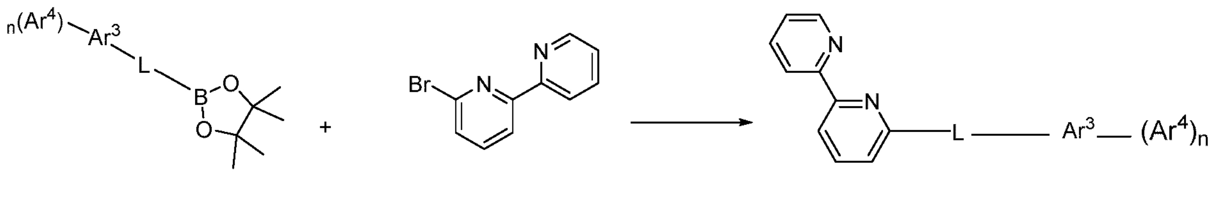

Reagent 1 Reagent 2 Compound of Formula (I)

To synthesize compound of Formula (I) a flask was flushed with nitrogen and charged with reagent (1) (1.1 equiv.), Reagent 2 (1 equiv.), $K_2CO_3$ (2 equiv.) dissolved in deaerated water), [1,1'-Bis(diphenylphosphino)ferrocene]dichloropalladium(II) (2 mol%). A deaerated mixture of toluene and ethanol (5:1) was added and the reaction mixture was heated to reflux under a nitrogen atmosphere while monitoring with TLC. After cooling down to room temperature, the resulting precipitate was isolated by suction filtration and washed with water (100 mL) and methanol (30mL). For further purification the crude product was then dissolved in dichloromethane and filtered through a pad of silicagel. After rinsing with additional dichloromethane, the filtrate was concentrated under reduced pressure and precipitated from methanol (150 mL). The resulting precipitate was recrystallized from 100mL toluene. The formed precipitate was collected by suction filtration and washed with small portion of toluene to give compound of Formula (I). An additional crop is isolated by precipitation by concentration of the toluene mother liquor Final purification was achieved by sublimation.

Building block preparation

**Synthesis of 2,3,5-triphenyl-6-(4-(4,4,5,5-tetramethyl-1,3,2-dioxaborolan-2-yl)phenyl)pyrazine**

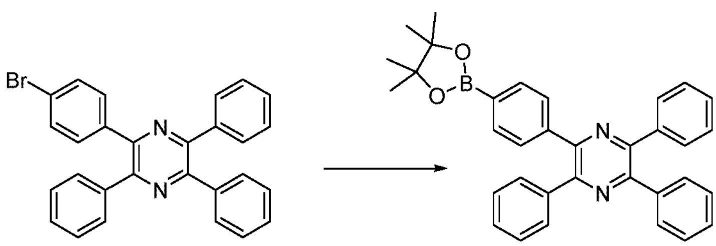

943442-81-7

A flask was flushed with nitrogen and charged with 2-(4-bromophenyl)-3,5,6-triphenylpyrazine (15.0 g, 32.4 mmol), bis(pinacolato)diboron (9.04 g, 35.6 mmol), Pd(dppf)Cl2 (0.71 g, 0.97 mmol), and potassium acetate (9.5 g, 97.2 mmol). Dry and deaerated DMF (90 mL) was added and the reaction mixture was heated to 100 °C under a nitrogen atmosphere overnight. Subsequently, all volatiles were removed in vacuo, water (400 mL) and dichloromethane (1 L) were added and the organic phase was washed with water (3 x 400 mL). After drying over MgSO4, the organic phase was filtered through a pad of silica gel. After rinsing with additional dichloromethane (1 L), the filtrate was concentrated under reduced pressure to a minimal volume. Methanol (350 mL) was added and the suspension was left stirring at room temperature overnight. The solid was collected by suction filtration to yield 15.9 g (96%) of 2,3,5-triphenyl-6-(4-(4,4,5,5-tetramethyl-1,3,2-dioxaborolan-2-yl)phenyl)pyrazine after drying.

Synthesis of 2,3,5-triphenyl-6-(3-(4,4,5,5-tetramethyl-1,3,2-dioxaborolan-2-**yl)phenyl)pyrazine**

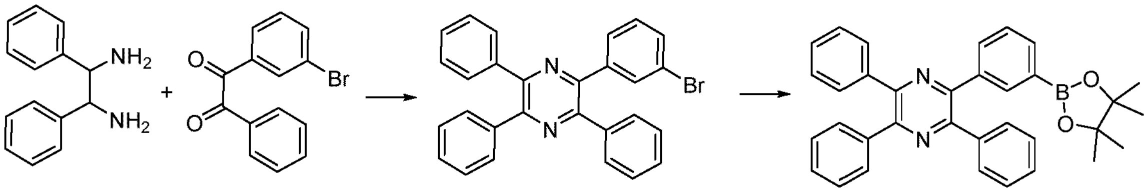

5700-60-7 40396-54-1

A flask was charged with 1-(3-bromophenyl)-2-phenylethane-1,2-dione (36.6 g, 126.5 mmol), 1,2-diphenylethane-1,2-diamine (38.7 g, 182.1 mmol), and acetic acid (320) mL. The mixture was heated to 75 °C for 24 h. Subsequently, the reaction mixture was concentrated under reduced pressure to approx. 100 mL and then carefully poured into sat. aq. K2CO3 (700 mL). After extraction with dichloromethane three times, the combined organic phases were washed with brine, dried over MgSO4, and concentrated under reduced pressure. The crude product was purified by column chromatography (silica, n-hexane/dichloromethane 8:2) and trituration with n-hexane to afford 38.8 g (66%) of 2,3,5-triphenyl-6-(3-(4,4,5,5-tetramethyl-1,3,2-dioxaborolan-2-yl)phenyl)pyrazineafter drying.

**Representative synthesis of 4'-(3-(3,5,6-triphenylpyrazin-2-yl)phenyl)-2,2':6',2"-terpyridine**

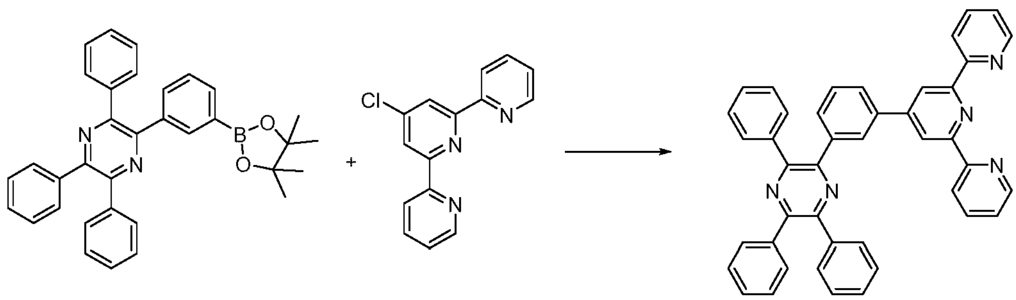

**Reagent 1** **Reagent 2** **compound G18**

To synthesize 4'-(3-(3,5,6-triphenylpyrazin-2-yl)phenyl)-2,2':6',2"-terpyridine (compound MX2) a flask was flushed with nitrogen and charged with 2,3,5-triphenyl-6-(3-(4,4,5,5-tetramethyl-1,3,2-dioxaborolan-2-yl)phenyl)pyrazine (1) (17.0 g, 33.0 mmol), 4'-chloro-2,2':6',2"-terpyridine (2) (8.1 g, 30.0 mmol), K2CO3 (8.29 g, 60 mmol, dissolved in 30mL deaerated water), [1,1'-Bis(diphenylphosphino)ferrocene]dichloropalladium(II) (0.44 g, 0.6 mmol). A deaerated mixture of toluene and ethanol (5:1, 180 mL) was added and the reaction mixture was heated to reflux under a nitrogen atmosphere while monitoring with TLC. After cooling down to room temperature, the resulting precipitate was isolated by suction filtration and washed with water (100 mL) and methanol (30mL). For further purification the crude product was then dissolved in dichloromethane and filtered through a pad of silicagel. After rinsing with additional dichloromethane, the filtrate was concentrated under reduced pressure and precipitated from methanol (150 mL). The resulting precipitate was recrystallized from 100mL toluene. The formed precipitate was collected by suction filtration and washed with small portion of toluene to give 7.8 g of compound 1. An additional crop is isolated by precipitation by concentration of the toluene mother liquor. Combined 11.6 g of Compound G18 were obtained after drying. Final purification was achieved by sublimation. HPLC: 99.96%, m/z = 616 ([M+H]+).

**Synthesis of 7-(3-([2,2'-bipyridin]-6-yl)phenyl)dibenzo[c,h]acridine**

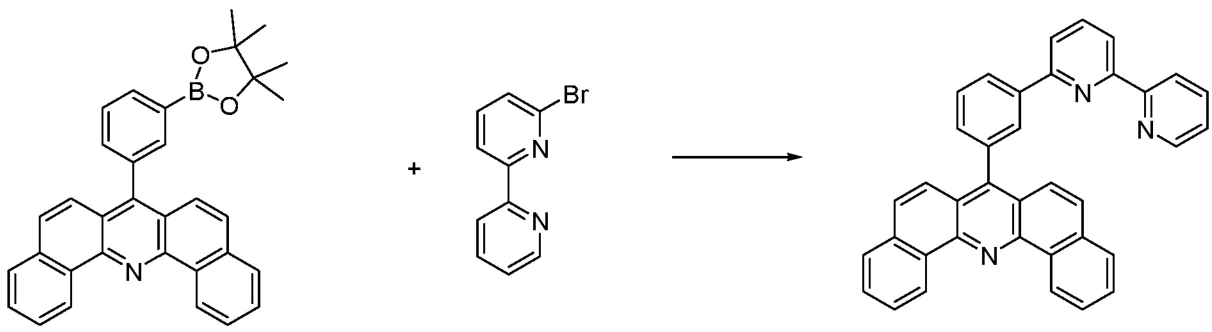

Reagent 1 Reagent 2 G11

To synthesize 7-(3-([2,2'-bipyridin]-6-yl)phenyl)dibenzo[c,h]acridine (compound 1) a flask was flushed with nitrogen and charged with 7-(3-(4,4,5,5-tetramethyl-1,3,2-dioxaborolan-2-yl)phenyl)dibenzo[c,h]acridine (30 g, 62.3 mmol), 6-bromo-2,2'-bipyridine (16.1 g, 68.5 mmol), K3PO4 (31.8 g, 150 mmol, dissolved in 75mL deaerated water), chloro(crotyl)(2-dicyclohexylphosphino-2',6'-dimethoxybiphenyl)palladium(II) (0.76 g, 1.24 mmol). Deaerated THF (310 mL) was added and the reaction mixture was heated to 60°C under a nitrogen atmosphere while monitoring with TLC. After cooling down to room temperature the solvent was distilled of. The resulting solid was extracted with CHCl3/H2O. The CHCl3 solution was dried over MgSO4 and filtered through a pad of florisil, additional rinsing is done with THF. The THF-solution is concentrated under reduced pressure. The precipitate is collected by suction filtration. After drying 12.5 g of G11 were obtained. Final purification was achieved by sublimation. HPLC 100%; m/z = 510.2 ([M+H]$^+$).

Further examples (compound G1-G3 and G12) were obtained by following this exemplary protocol, varying reagent 1, using the reagents and conditions and purification adaption as given in the Table 1 below:

Table 1

| Compund | Regent1 | Catalyst | Base | Solvent<br>Temp. | Further purification method | Amount (Yield) HPLC |
|---|---|---|---|---|---|---|
| G1 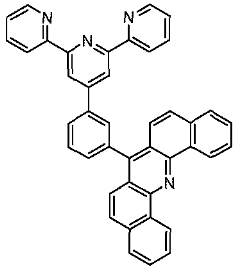 | 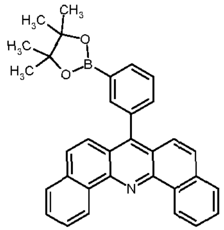 | 1798781-99-3 | 7778-53-2 | THF/H2O 7/2<br>60°C | product precipitates, crude treated with Pd(dba) 2/PtBu3 in Xylene at reflux for 24h, then filtered through SiO2, rinse CH2Cl2/MeOH, organic phase concentrated & precip. | 7,1 g (58%) 100% m/z = 562 $[M+H]^+$ |
| G2 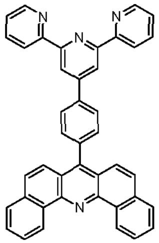 | 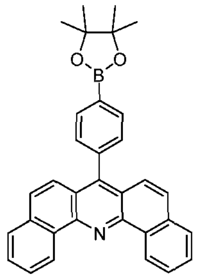 | 1798781-99-3 | 7778-53-2 | THF/H2O 4/1<br>60°C | product precipitates, soxhlet extraction (chlorobenzene), crystallization from chlorobenzene | 18,2 g (67 %) 100 % m/z = 587 $[M+H]^+$ |
| G3 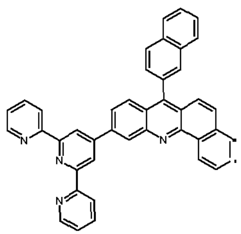 | 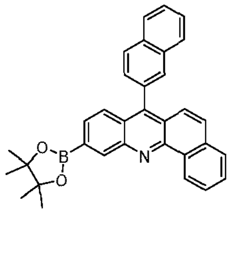 | 1798781-99-3 | 7778-53-2 | THF/H2O 4/1<br>60°C | product precipitates, soxhlet extraction (chlorobenzene), crystallization from chlorobenzene | 8,5 g (63%) 100 % m/z = 587 $[M+H]^+$ |

(continued)

| Compund | Regent1 | Catalyst | Base | Solvent<br>Temp. | Further purification method | Amount (Yield) HPLC |
|---|---|---|---|---|---|---|
| G12 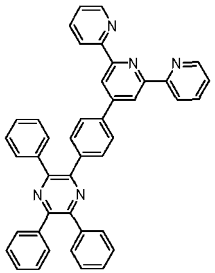 | 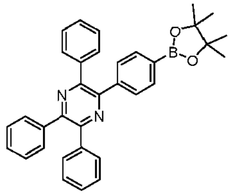 | 1798781-99-3 | 7778-53-2 | dioxane/H2O 10/1<br>60°C | product precipitates, filtration through SiO2 (CHCl3), concentrated and precipitation with iso-propylacetate | 30 g (84%)<br>100 %<br>m/z = 616 $[M+H]^+$ |

In Table 2 below the properties of inventive compounds of Formula (I) are listed.

Table 2: Properties of inventive compounds of Formula (I)

| Referred to as: | Structure | mp (°C) | Tg (°C) | $T_{RO}$ (°C) | HOMO (eV) | LUMO (eV) | Diplole moment (Debye) |
|---|---|---|---|---|---|---|---|
| G1 | 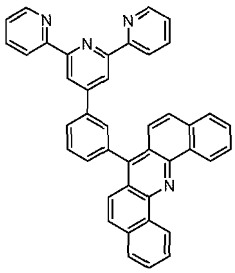 | 287 | 148 | 250 | -5.70 | -1.79 | 0.47 |
| G2 | 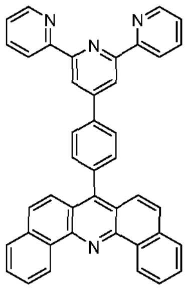 | 343 | 156 | -- | -5.66 | -1.82 | 2.03 |
| G3 | 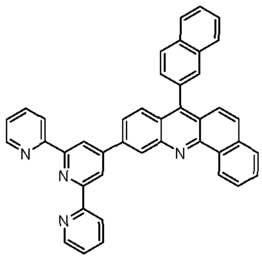 | 339 | 156 | 288 | -5.60 | -2.02 | 4.0 |
| G11 | 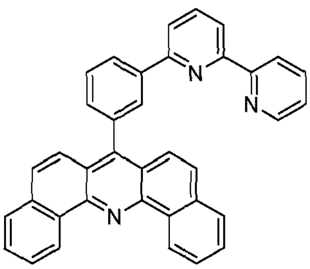 | 236 | 109 | 208 | -5.48 | -1.59 | -- |
| G12 | 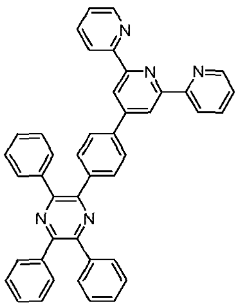 | 280 | 131 | 238 | -5.79 | -1.71 | 1.91 |

(continued)

| Referred to as: | Structure | mp (°C) | Tg (°C) | $T_{RO}$ (°C) | HOMO (eV) | LUMO (eV) | Diplole moment (Debye) |
|---|---|---|---|---|---|---|---|
| G18 | 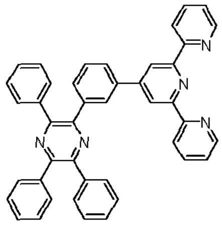 | -- | 118 | 225 | -5.64 | -1.64 | 1.61 |

General procedure 1 the for fabrication of OLEDs

For bottom emission devices, Examples 1 to 4 and comparative examples 1 to 2, a glass substrate was cut to a size of 150 mm x 150 mm x 0.7 mm, ultrasonically cleaned with isopropyl alcohol for 5 minutes and then with pure water for 5 minutes, and cleaned again with UV ozone for 30 minutes, to prepare a first electrode. 90 nm ITO were deposited on the glass substrate at a pressure of $10^{-5}$ to $10^{-7}$ mbar to form the anode.

Then, 92 vol.-% Biphenyl-4-yl(9,9-diphenyl-9H-fluoren-2-yl)-[4-(9-phenyl-9H-carbazol-3-yl)phenyl]-amine (CAS 1242056-42-3) with 8 vol.-% 2,2',2"-(cyclopropane-1,2,3-triylidene)tris(2-(p-cyanotetrafluorophenyl)acetonitrile) was vacuum deposited on the anode, to form a HIL having a thickness of 10 nm.

Then, Biphenyl-4-yl(9,9-diphenyl-9H-fluoren-2-yl)-[4-(9-phenyl-9H-carbazol-3-yl) phenyl]-amine was vacuum deposited on the HIL, to form a HTL having a thickness of 128 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then 97 vol.-% H09 (Sun Fine Chemicals, South Korea) as EML host and 3 vol.-% BD200 (Sun Fine Chemicals, South Korea) as fluorescent blue dopant were deposited on the EBL, to form a blue-emitting EML with a thickness of 20 nm.

Then the hole blocking layer is formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer.

Then, the electron transporting layer is formed on the hole blocking layer according to Examples 1 to 4 and comparative examples 1 to 2 with a the thickness of 31 nm. The electron transport layer comprises 99 wt.-% matrix compound of Formula 1 and 1 wt.-% of Li as metal dopant or electron transport layer comprises 97 wt.-% matrix compound of Formula 1 and 3 wt.-% of Yb, see Table 3.

Al is evaporated at a rate of 0.01 to 1 Å/s at $10^{-7}$ mbar to form a cathode with a thickness of 100 nm.

The OLED stack is protected from ambient conditions by encapsulation of the device with a glass slide. Thereby, a cavity is formed, which includes a getter material for further protection.

General procedure 2 for the fabrication of OLEDs

For bottom emission devices, Examples 4 to 8 and comparative examples 3 to 5, a glass substrate was cut to a size of 150 mm x 150 mm x 0.7 mm, ultrasonically cleaned with isopropyl alcohol for 5 minutes and then with pure water for 5 minutes, and cleaned again with UV ozone for 30 minutes, to prepare a first electrode. 90 nm ITO were deposited on the glass substrate at a pressure of $10^{-5}$ to $10^{-7}$ mbar to form the anode.

Then, 97 vol.-% Biphenyl-4-yl(9,9-diphenyl-9H-fluoren-2-yl)-[4-(9-phenyl-9H-carbazol-3-yl)phenyl]-amine (CAS 1242056-42-3) with 3 vol.-% 2,2',2"-(cyclopropane-1,2,3-triylidene)tris(2-(p-cyanotetrafluorophenyl)acetonitrile) was vacuum deposited on the anode, to form a HIL having a thickness of 10 nm.

Then, Biphenyl-4-yl(9,9-diphenyl-9H-fluoren-2-yl)-[4-(9-phenyl-9H-carbazol-3-yl) phenyl]-amine was vacuum deposited on the HIL, to form a first HTL having a thickness of 128 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then 97 vol.-% H09 (Sun Fine Chemicals, South Korea) as EML host and 3 vol.-% BD200 (Sun Fine Chemicals, South Korea) as fluorescent blue dopant were deposited on the EBL, to form a first blue-emitting EML with a thickness of 20 nm.

Then the first hole blocking layer is formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer.

Then, the electron transporting layer having a thickness of 25 nm is formed on the hole blocking layer by depositing 4'-(4-(4-(4,6-diphenyl-1,3,5-triazin-2-yl)phenyl)naphthalen-1-yl)-[1,1'-biphenyl]-4-carbonitrile. The electron transport layer (ETL) comprises 50 wt.-% matrix compound and 50 wt.-% of LiQ.

Then the n-CGL was formed on ETL with a thickness of 15 nm. The n-CGL comprises 99 wt.-% matrix compound of Formula 1 and 1 wt.-% of Li as metal dopant or electron transport layer comprises 97 wt.-% matrix compound of Formula 1 and 3 wt.-% of Yb, see Table 4. Then the p-CGL was formed on n-CGL with a thickness of 10 nm on n-CGL by depositing Biphenyl-4-yl(9,9-diphenyl-9H-fluoren-2-yl)-[4-(9-phenyl-9H-carbazol-3-yl) phenyl]-amine.

Then, Biphenyl-4-yl(9,9-diphenyl-9H-fluoren-2-yl)-[4-(9-phenyl-9H-carbazol-3-yl) phenyl]-amine was vacuum deposited on the p-CGL, to form a second HTL having a thickness of 10 nm.

Al is evaporated at a rate of 0.01 to 1 Å/s at $10^{-7}$ mbar to form a cathode with a thickness of 100 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m$^2$ using an Instrument Systems CAS-140CT array spectrometer (calibrated by Deutsche Akkreditierungsstelle (DAkkS)) for each of the voltage values. The cd/A efficiency at 10 mA/cm$^2$ is determined by interpolating the luminance-voltage and current-voltage characteristics, respectively.

Lifetime LT of the device is measured at ambient conditions (20°C) and 30 mA/cm$^2$, using a Keithley 2400 sourcemeter, and recorded in hours.

The brightness of the device is measured using a calibrated photo diode. The lifetime LT is defined as the time till the brightness of the device is reduced to 97 % of its initial value.

In Table 3 is shown the performance of an organic electronic device comprising an electron transport layer comprising a compound of Formula (I) and a metal dopant.

In comparative example 1, compound ETM-1 was used as matrix compound:

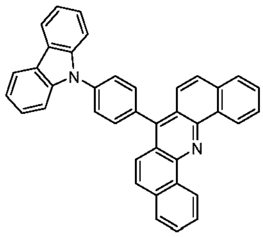

ETM-1.

Compound ETM-1 is free of a terpyridyl-group. The organic semiconductor layer comprises 99 vol.-% ETM-1 and 1 vol.-% Li. The operating voltage at 15 mA/cm$^2$ is 8.4 V and the cd/A efficiency is 3.3 cd/A.

In comparative example 2, compound ETM-1 was used as matrix compound:

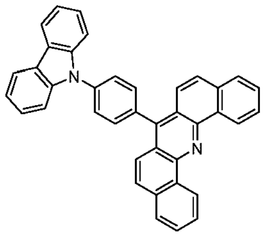

ETM-1.

Compound ETM-1 is free of a terpyridyl-group. The ETL comprises 97 vol.-% ETM-2 and 3 vol.-% Yb. The operating voltage at 15 mA/cm$^2$ is 6.3 V and the cd/A efficiency is 3.0 cd/A.

In Example 1, the ETL comprises 99 vol.-% compound of Formula (I) of G1

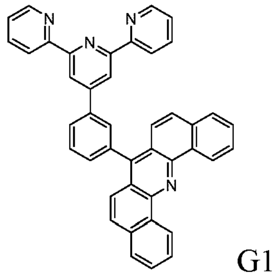

G1

and 1 vol.-% Li. The operating voltage at 15 mA/$cm^2$ is 3.7 V and the cd/A efficiency is 8.5 cd/A.

In Example 2, the ETL comprises 97 vol.-% compound of Formula (I) of G1

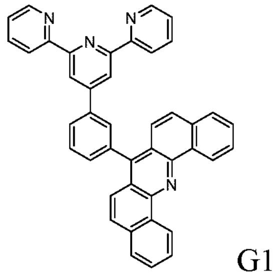

G1

and 3 vol.-% Yb. The operating voltage at 15 mA/$cm^2$ is 4.6 V and the cd/A efficiency is 4.8 cd/A.

In Example 3, the ETL comprises 99 vol.-% compound of Formula (I) of MX2

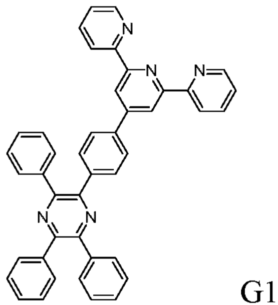

G12

and 1 vol.-% Li. The operating voltage at 15 mA/$cm^2$ is 3.8 V and the cd/A efficiency is 7.8 cd/A.

In Example 4, the ETL comprises 97 vol.-% compound of Formula (I) of G12

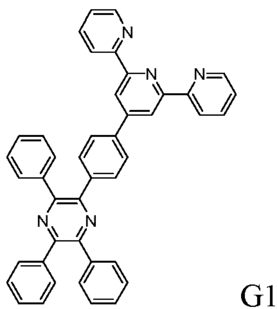

G12

and 3 vol.-% Yb. The operating voltage at 15 mA/$cm^2$ is 4.6 V and the cd/A efficiency is 4.5 cd/A.

In Table 3 it is shown the performance of an organic electronic device comprising an n-CGL comprising a compound of Formula (I) and a metal dopant.

In comparative example 3, compound ETM-1 was used as matrix compound:

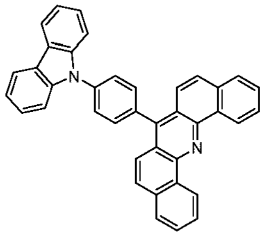

ETM-1.

Compound ETM-1 is free of a terpyridyl-group. The n-CGL comprises 99 vol.-% ETM-1 and 1 vol.-% Li. The operating voltage at 15 mA/cm$^2$ is 8.4 V and the cd/A efficiency is-cd/A.

In comparative example 4, compound ETM-2 was used as matrix compound:

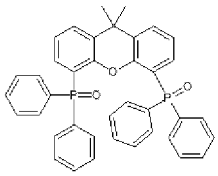

ETM-2.

Compound ETM-2 is free of a terpyridyl-group. The n-CGL comprises 99 vol.-% ETM-1 and 1 vol.-% Li. The operating voltage at 15 mA/cm$^2$ is 8.4 V and the cd/A efficiency is-cd/A.

In comparative example 5, compound ETM-2 was used as matrix compound:

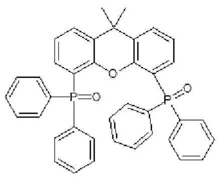

ETM-2.

Compound ETM-2 is free of a terpyridyl-group. The n-CGL comprises 97 vol.-% ETM-2 and 3 vol.-% Yb. The operating voltage at 15 mA/cm$^2$ is 6.3 V and the cd/A efficiency is 7.3 cd/A.

In comparative example 5, compound ETM-1 was used as matrix compound:

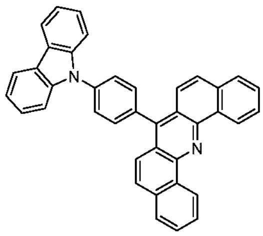

ETM-1.

Compound ETM-1 is free of a terpyridyl-group. The ETL comprises 97 vol.-% ETM-2 and 3 vol.-% Yb. The operating voltage at 15 mA/cm$^2$ is 6.3 V and the cd/A efficiency is 3.0 cd/A.

In Example 5, the n-CGL comprises 99 vol.-% compound of Formula (I) of G1

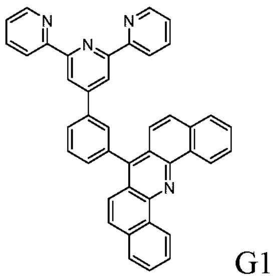

G1

and 1 vol.-% Li. The operating voltage at 15 mA/cm$^2$ is 5.2 V and the cd/A efficiency is 6.8 cd/A.

In Example 6, the n-CGL comprises 97 vol.-% compound of Formula (I) of G1

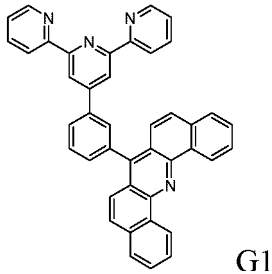

G1

and 3 vol.-% Yb. The operating voltage at 15 mA/cm$^2$ is 5.1 V and the cd/A efficiency is 6.7 cd/A.

In Example 7, the n-CGL comprises 99 vol.-% compound of Formula (I) of G12

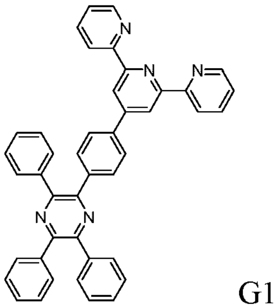

G12

and 1 vol.-% Li. The operating voltage at 15 mA/cm$^2$ is 5.2 V and the cd/A efficiency is 6.8 cd/A.

In Example 8, the n-CGL comprises 97 vol.-% compound of Formula (I) of G12

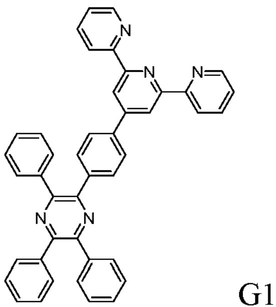

G12

and 3 vol.-% Yb. The operating voltage at 15 mA/cm$^2$ is 5.1 V and the cd/A efficiency is 6.9 cd/A.

Therefore, it can be seen the material for ETL (Example 1-4) and n-CGL (Example 5-8) can secure low driving voltage and high efficiency of an organic electronic device, when used in an organic electronic device.

Table 3: Performance of an organic electronic device comprising an electron transport layer comprising a compound of Formula (I) and a metal dopant

| Example | Matrix compound | Concentration of matrix compound in ETL (vol.-%) | Metal dopant | Concentration of Metal dopant (vol.-%) | Thickness electron transport layer (nm) | Operating voltage at 15 mA/cm$^2$ (V) |
|---|---|---|---|---|---|---|
| Example 1 (Comparative) | 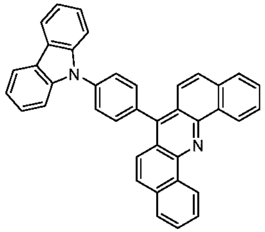 | 99 | Li | 1 | 31 | 8.4 |
| Example 2 (Comparative) | 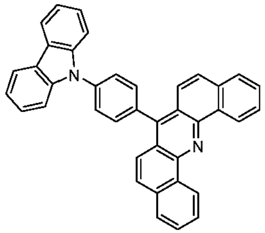 | 97 | Yb | 3 | 31 | 6.3 |
| Example 1 | 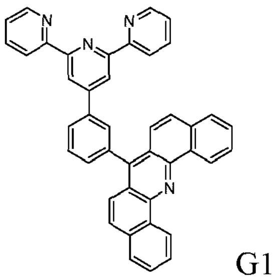 G1 | 99 | Li | 1 | 31 | 3.7 |
| Example 2 | 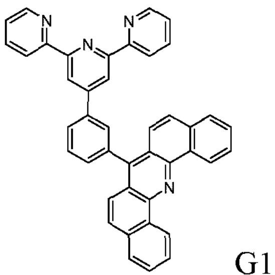 G1 | 97 | Yb | 3 | 31 | 4.6 |

(continued)

| Example | Matrix compound | Concentration of matrix compound in ETL (vol.-%) | Metal dopant | Concentration of Metal dopant (vol.-%) | Thickness electron transport layer (nm) | Operating voltage at 15 mA/cm$^2$ (V) |
|---|---|---|---|---|---|---|
| Example 3 | 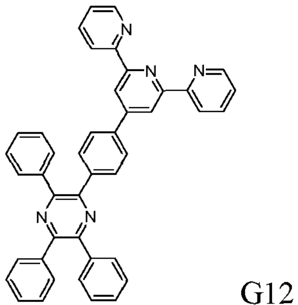 G12 | 99 | Li | 1 | 31 | 3.8 |
| Example 4 | 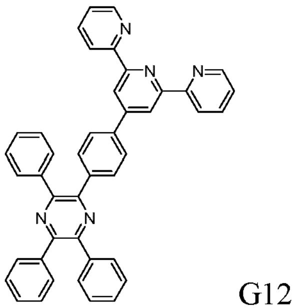 G12 | 97 | Yb | 3 | 31 | 4.6 |

Table 4: Performance of an organic electronic device comprising an n-CGL comprising a compound of Formula (I) and a metal dopant

| Example | Matrix compound | n-CGL (vol.-%) | Metal dopant | Concentratio n of Metal d opant (vol.-%) | Thickness n-CGL (nm) | Operating voltage at 15 $mA/cm^2$ (V) |
|---|---|---|---|---|---|---|
| Example 3 Comparative | 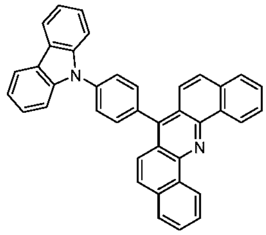 | 99 | Li | 1 | 15 | 11.9 |
| Example 4 Comparative | 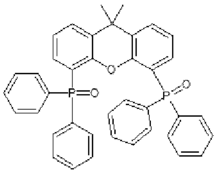 | 99 | Li | 1 | 15 | 10.4 |
| Example 5 Comparative | 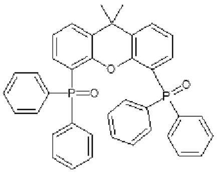 | 97 | Yb | 3 | 15 | 9.8 |
| Example 5 | 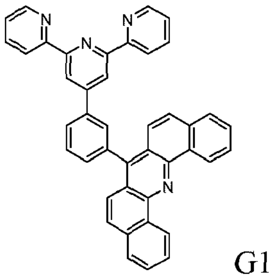 G1 | 99 | Li | 1 | 15 | 5.2 |
| Example 6 | 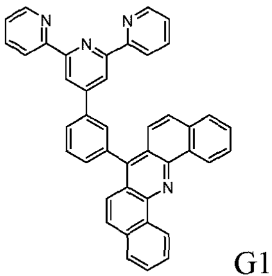 G1 | 97 | Yb | 3 | 15 | 5.1 |

(continued)

| Example | Matrix compound | n-CGL (vol.-%) | Metal dopant | Concentratio n of Metal d opant (vol.-%) | Thickness n-CGL (nm) | Operating voltage at 15 mA/cm2 (V) |
|---|---|---|---|---|---|---|
| Example 7 | 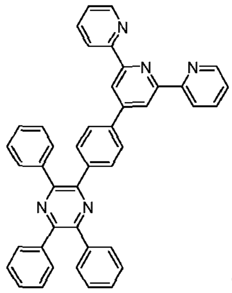 G12 | 99 | Li | 1 | 15 | 5.2 |
| Example 8 | 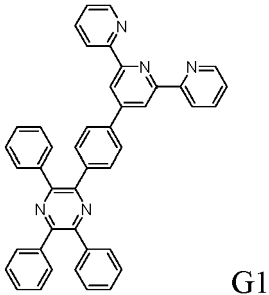 G12 | 97 | Yb | 3 | 15 | 5.1 |

In summary, low operating voltage and improved lifetime may be achieved when the organic semiconductor layer comprises a compound of Formula (I) and metal dopant.

## Claims

1. Organic electronic device comprising an anode, a cathode and at least one organic semiconductor layer, wherein the organic electronic device further comprises at least one emission layer and the at least one organic semiconductor layer is arranged between the at least one emission layer and the cathode, wherein the at least one organic semiconductor layer comprises:

   - a metal dopant, and
   - a compound represented by the following formula (I):

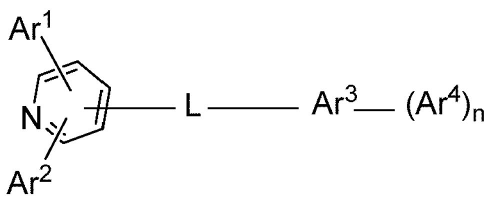

(I)

wherein,

Ar1 and Ar2 are same or different and independently selected from hydrogen, substituted or unsubstituted $C_6$ to $C_{36}$ aryl, substituted or unsubstituted $C_3$ to $C_{36}$ heteroaryl, wherein, at least one of the $Ar^1$ and $Ar^2$ group comprises a substituted or unsubstituted $C_3$ to $C_{36}$ heteroaryl;

L is a single bond, a substituted or unsubstituted $C_6$ to $C_{18}$ arylene, a substituted or unsubstituted $C_3$ to $C_{18}$ heteroarylene, a substituted or unsubstituted phenylene, a substituted or unsubstituted biphenylene, a substituted or unsubstituted terphenylene, a substituted or unsubstituted anthracenylene, a substituted

or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;
$Ar^3$ is a group selected from a substituted $C_4$ N-heteroarylene, a substituted or unsubstituted $C_{12}$ heteroarylene comprising at least three fused rings and one hetero atom selected from O, S or N, a substituted pyrazinylene, a substituted pyrimidinylene, a substituted or unsubstituted acridinylene, a substituted or unsubstituted benzoacridinylene, a substituted or unsubstituted dibenzoacridinylene, a substituted or unsubstituted dibenzofuranylene, a substituted or unsubstituted carbazolylene, a substituted or unsubstituted benzoquinolinylene, a substituted or unsubstituted phenanthridinylene, a substituted or unsubstituted dinaphthofuranylene or a substituted or unsubstituted dinaphthothiophenylene;
$Ar^4$ is a group independently selected from H, a substituted or unsubstituted $C_6$ to $C_{18}$ aryl, a substituted or unsubstituted $C_3$ to $C_{20}$ heteroaryl, an unsubstituted phenyl, an unsubstituted biphenyl, an unsubstituted naphthyl, an unsubstituted anthracenyl, an unsubstituted phenanthridinyl, an unsubstituted pyridyl, an unsubstituted quinolinyl, an unsubstituted pryrimidyl, or having the formula (II) or (III),

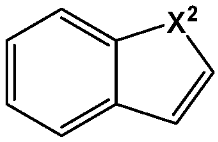 (II), 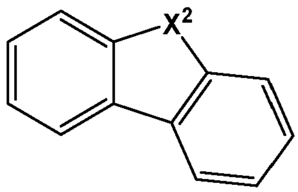 (III),

wherein the group of formula (II) and (III) are substituted or unsubstituted and $X^2$ is selected from N, S,O or $C(R^1)_2$;
n is 0, 1, 2, 3 or 4;

wherein the substituents of L, $Ar^1$, $Ar^2$, $Ar^3$, $Ar^4$ and/or N are independently selected from:

- $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, - $PX^3(R^2)_2$, D, F or CN;
- $R^1$ and $R^2$ are independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy and
- two $R^1$ can be linked together to form a ring
- $X^3$ is selected from S or O.

**2.** The organic electronic device according to claim 1, wherein

- $Ar^1$ and $Ar^2$ are independently selected from a hydrogen, phenyl, pyridyl, phenyl pyridyl or quinolinyl group; or
- $Ar^1$ is hydrogen and $Ar^2$ is a pyridyl, phenyl pyridyl or quinolinyl group;
- $Ar^1$ is phenyl and $Ar^2$ is a pyridyl, phenyl pyridyl or quinolinyl group;
- $Ar^1$ is hydrogen and $Ar^2$ is a pyridyl group;
- $Ar^1$ is phenyl and $Ar^2$ is a pyridyl group; or
- $Ar^1$ and $Ar^2$ are pyridyl groups.

**3.** The organic electronic device according to claim 1 or 2, wherein
L is a single bond or is selected from an unsubstituted phenylene, an unsubstituted biphenylene, an unsubstituted terphenylene, an unsubstituted anthracenylene, an unsubstituted dibenzofuranylene, an unsubstituted dibenzothiophenylene, an unsubstituted an unsubstituted pyridinylene, a substituted or unsubstituted phenylpyridinylene, preferably L is a single bond or is selected from unsubstituted phenylene or unsubstituted biphenylene.

**4.** The organic electronic device according to any of the preceding claims 1 to 3, wherein $Ar^3$ is a group selected from a substituted pyrazinylene, a substituted pyrimidinylene, an unsubstituted benzoacridinylene or an unsubstituted dibenzoacridinylene.

**5.** The organic electronic device according to any of the preceding claims 1 to 4, wherein

- $Ar^4$ is a group selected from H, an unsubstituted phenyl, an unsubstituted biphenyl, an unsubstituted pyridyl, an unsubstituted naphthyl, an unsubstituted dibenzofuranyl, an unsubstituted benzofuranyl, an unsubstituted dibenzothiophenyl, an unsubstituted benzothiophenyl;or
- H.

**6.** The organic electronic device according to any of the preceding claims 1 to 5, wherein the metal dopant is:

- a metal selected from the group consisting of alkali metals, alkaline earth metals and rare earth metals;
- a metal selected from Li, Na, Cs, Mg, Ca, Sr, Sm or Yb;
- a metal selected from Li, Cs, Mg or Yb; or
- a metal selected from Li or Yb.

**7.** The organic electronic device according to any of the preceding claims 1 to 6, wherein L is independently selected from B1 to B21:

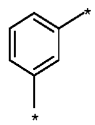 , 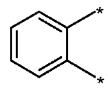 , 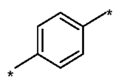 , 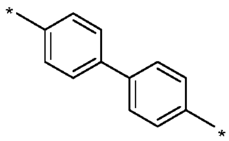 , 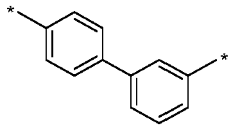 , 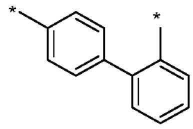 ,

(B1) (B2) (B3) (B4) (B5), (B6),

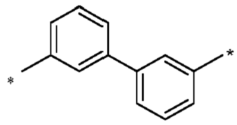 , 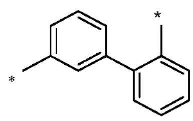 , 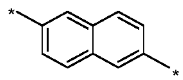 , 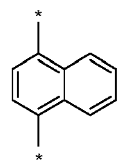 , 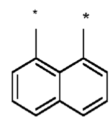 ,

(B7) (B8) (B9) (B10) (B11),

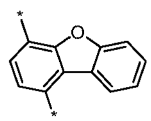 , 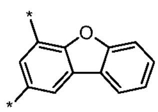 , 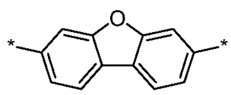 , 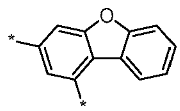 , 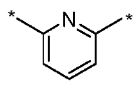 , 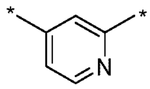

(B12) (B13) (B14) (B15) (B16), (B17),

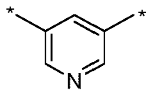 , 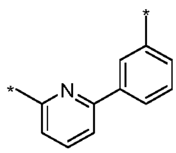 , 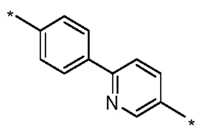 , 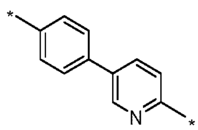 ,

(B18) (B19) (B20) (B21);

wherein the asterisk symbol "*" represents the binding position of L.

**8.** The organic electronic device according to any of the preceding claims 1 to 7, wherein the metal of the metal dopant has an electronegativity of about ≥ 0.7 to about ≤ 1.3 according to Pauling scale, and preferably the metal dopant has an electronegativity of about ≥ 0.9 to about ≤ 1.2, further preferred about ≥ 1 to about ≤ 1.1.

**9.** An organic electronic device according to any of the preceding claims 1 to 8, wherein $Ar^3$ is independently selected from E1 to E9:

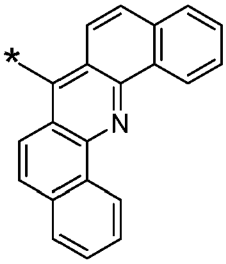 (E1), 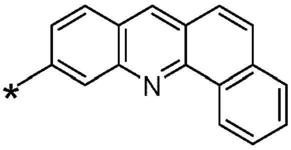 (E2), 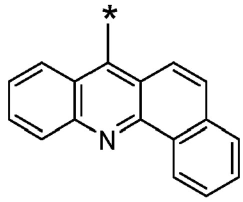 (E3), 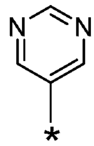 (E4),

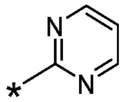 (E5),

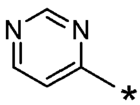 (E6), 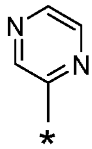 (E7), 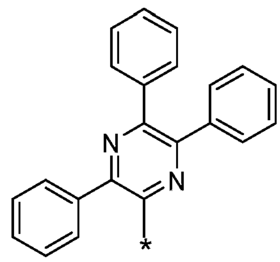 E8, 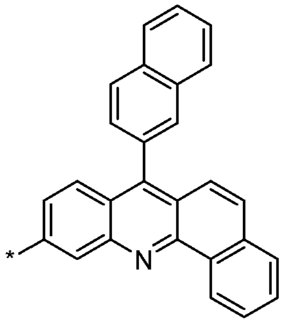 E9,

wherein the asterisk symbol "*" represents the binding position of $Ar^3$ to L and if n = 1, 2, 3 or 4 $Ar^4$ bonds to a carbon of $Ar^3$ excluding the binding position of $Ar^3$ to L.

**10.** An organic electronic device according to any of the preceding claims 1 to 9, wherein $Ar^4$ is independently selected from hydrogen or F1 to F13:

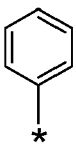 (F1), 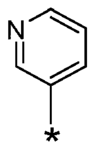 (F2), 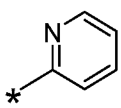 (F3), 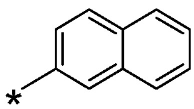 (F4), 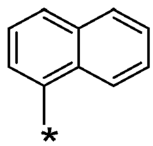 (F5), 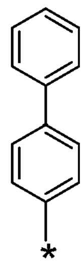 (F5),

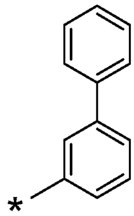 (F6), 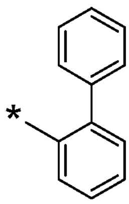 (F7), 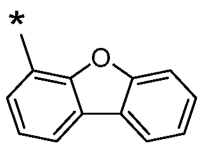 (F8), 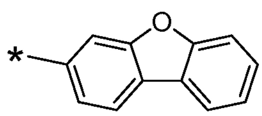 (F8),

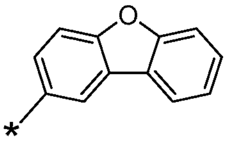 (F8), 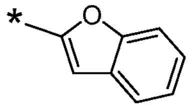 (F9), 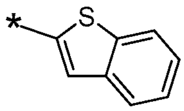 (F10), 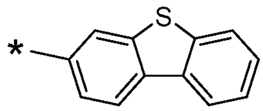 (F11),

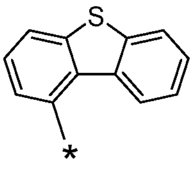 (F12), 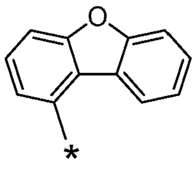 (F13);

wherein the asterisk symbol "*" represents the binding position of $Ar^4$ to $Ar^3$, and $Ar^4$ bonds to a carbon of $Ar^3$ excluding the binding position of $Ar^3$ to L.

**11.** The organic electronic device according to any of the preceding claim 1 to 10, wherein according formula (I) $Ar^3$-$(Ar^4)_n$ is independently selected from D1 to D12:

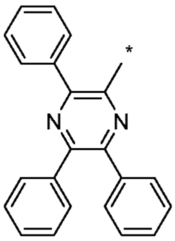, 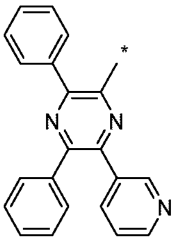, 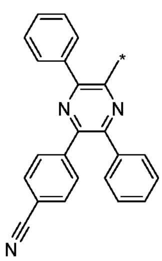, 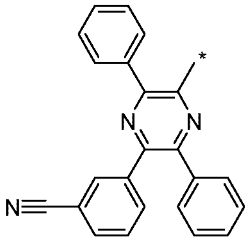, 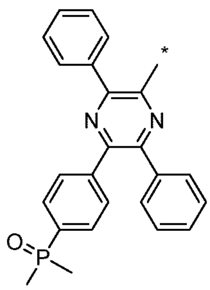,

D1, D2 D3 D4 D5

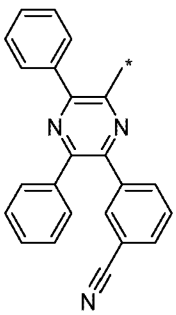, 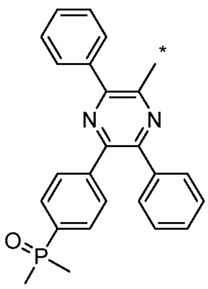, 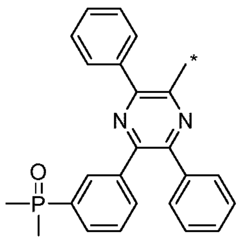, 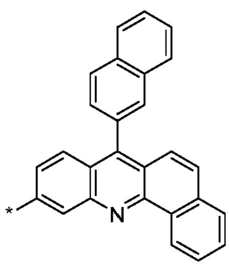, 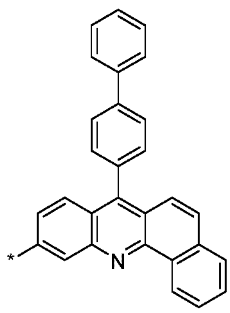

D6, D7, D8 D9 D10

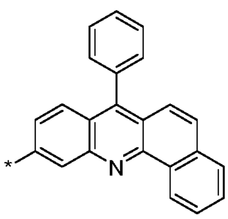, 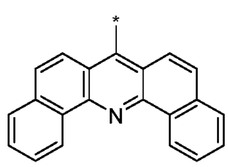

D11 D12;

wherein the asterisk symbol "*" represents the binding position of $Ar^3$ to L.

**12.** The organic electronic device according to any of the preceding claims 1 to 11, wherein the compound of formula (I) are selected from G1 to G36:

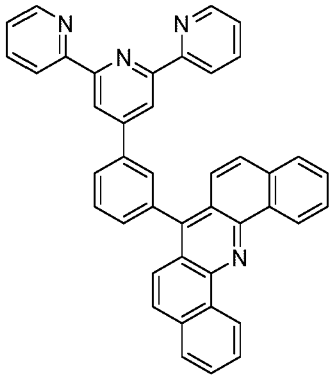

,

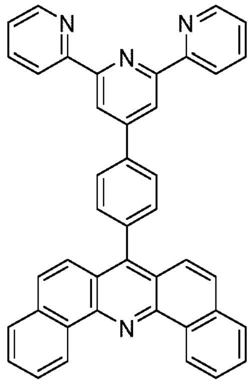

,

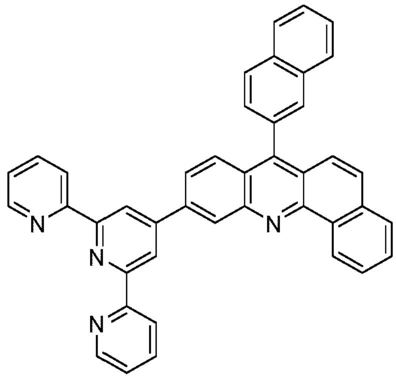

(G1) (G2) (G3)

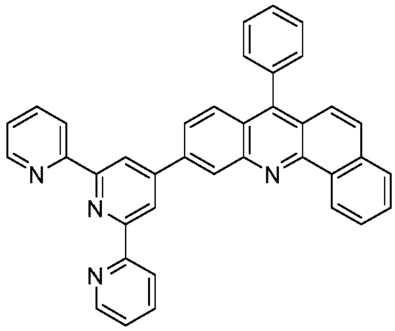

,

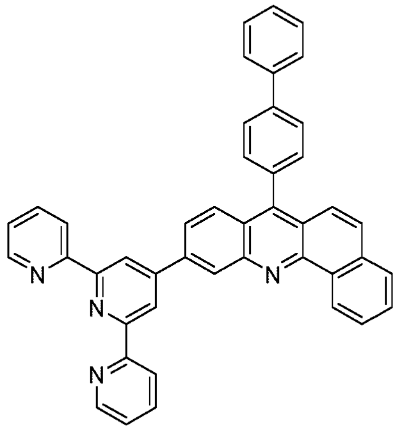

,

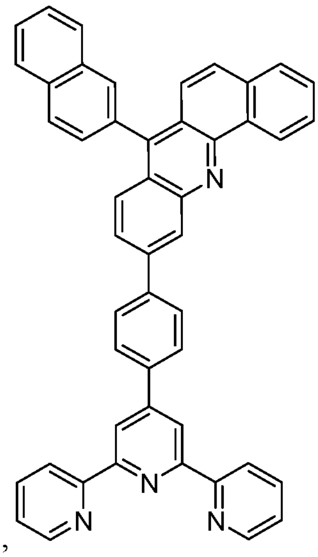

,

(G4) (G5) (G6)

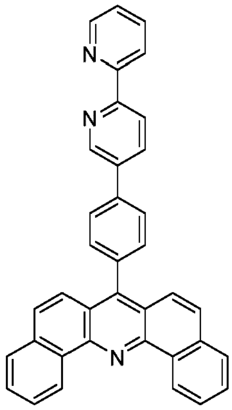

,

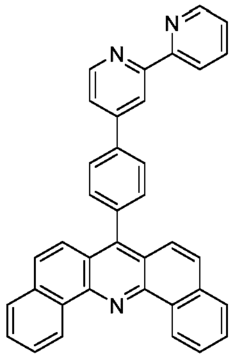

,

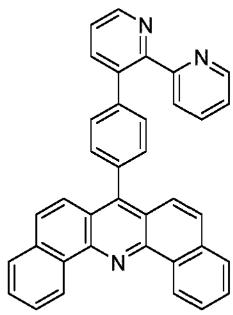

,

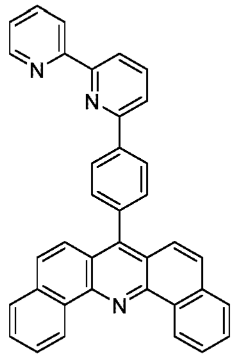

,

(G7) (G8) (G9) (G10)

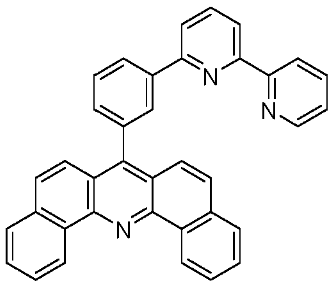
,
(G11)

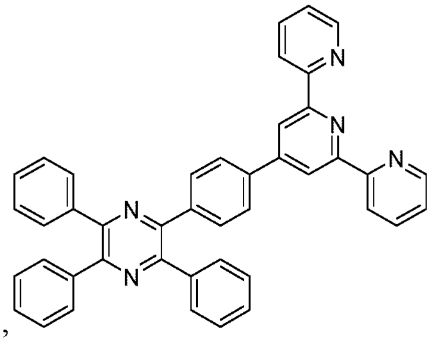
,
(G12)

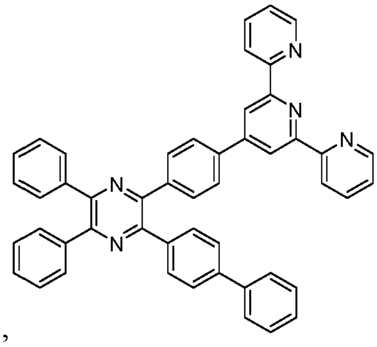
,
(G13)

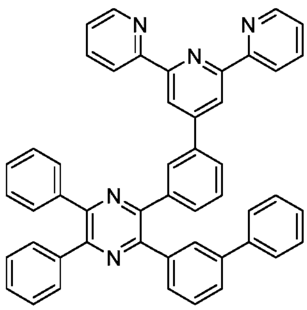
,
(G14)

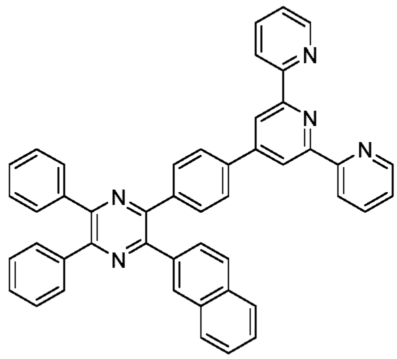
,
(G15)

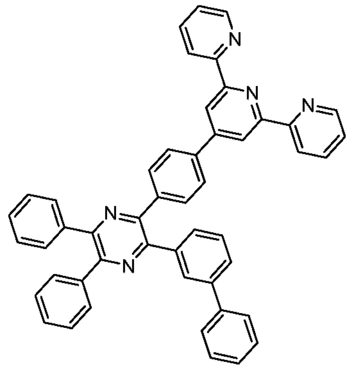
,
(G16)

,
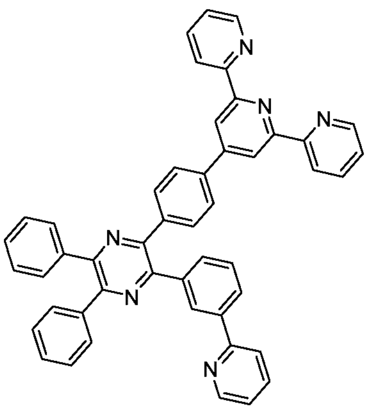
,
(G17)

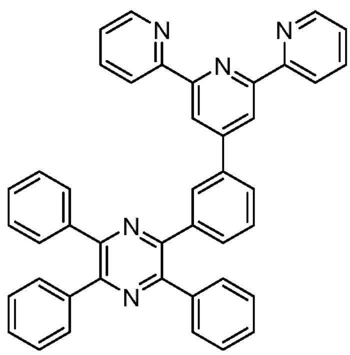
,
(G18)

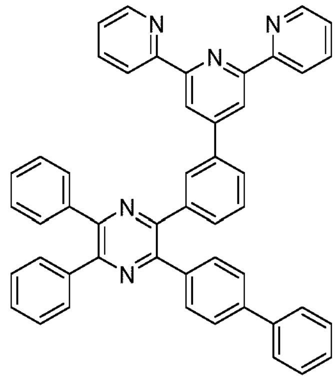
,
(G19)

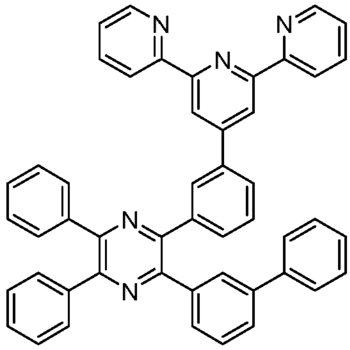
,
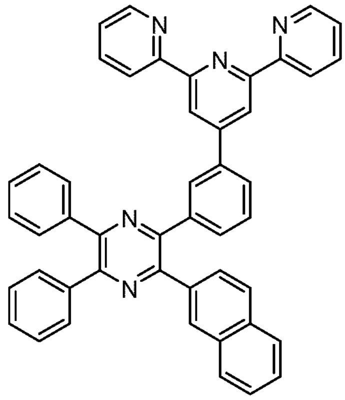
,
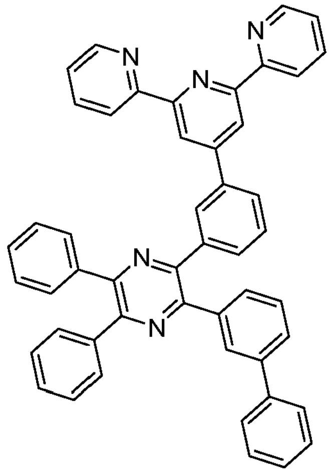
,

(G20) (G21) (G22)

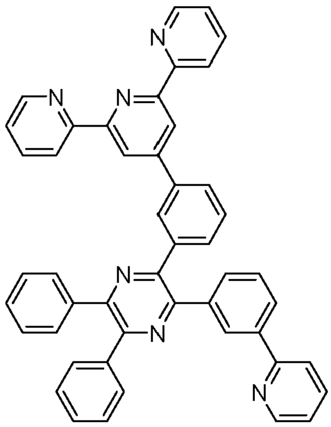
,
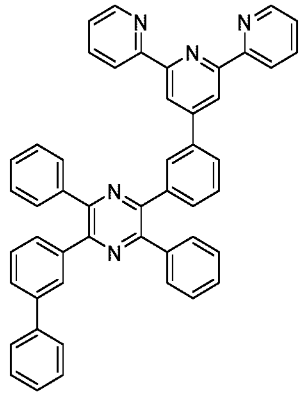
,
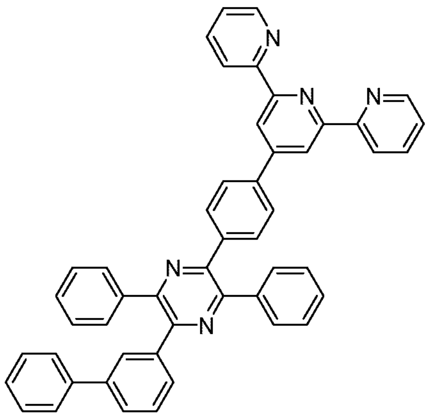

(G23) (G24) (G25)

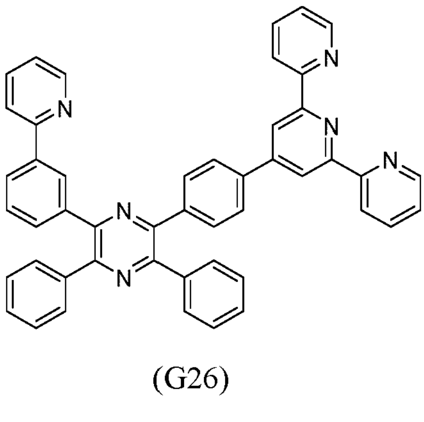
,
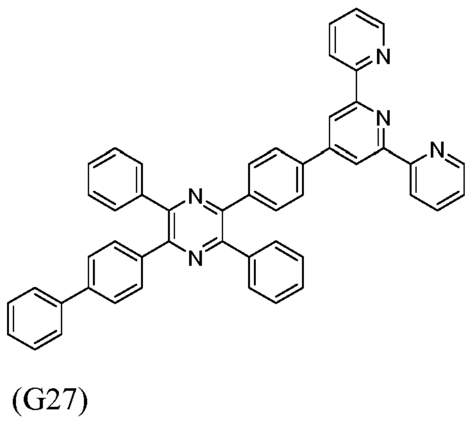
,
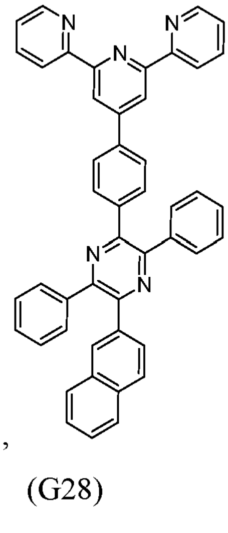
,

(G26) (G27) (G28)

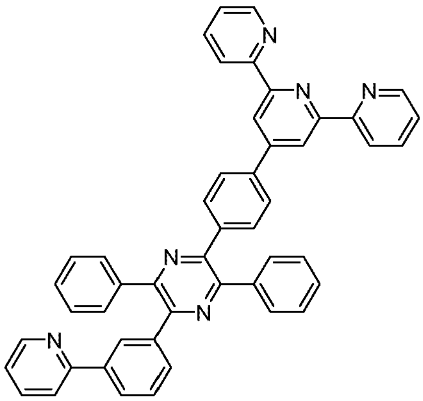

, 

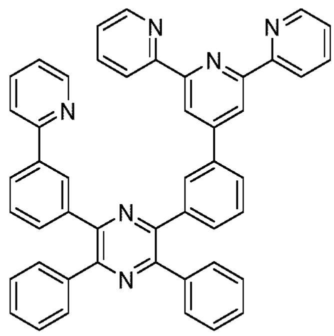

, 

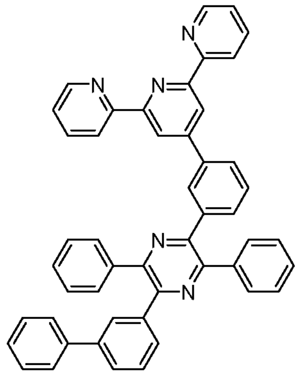

(G29) (G30) (G31)

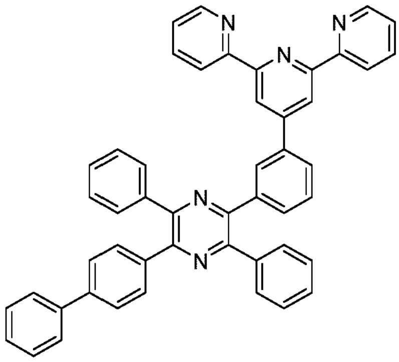

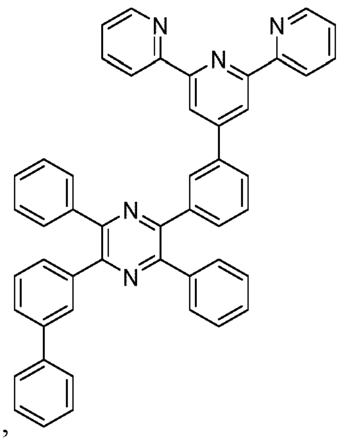

, 

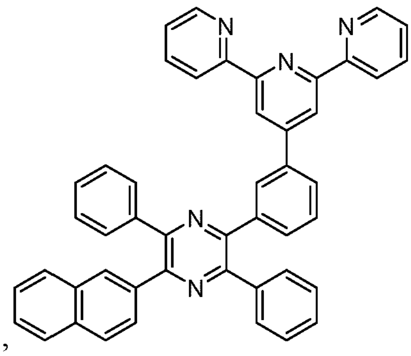

, 

,

(G32) (G33) (G34)

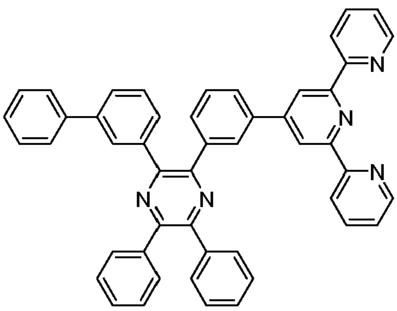

, 

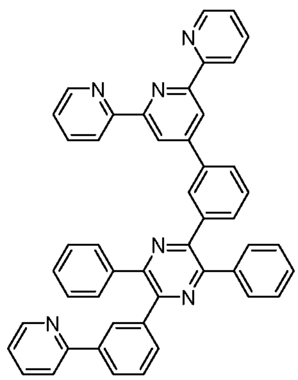

(G35) (G36).

13. The organic electronic device according to claims 1 to 12, wherein the electronic device is a light emitting device, thin film transistor, a battery, a display device or a photovoltaic cell, and preferably a light emitting device.

14. A compound represented by the following formula (I):

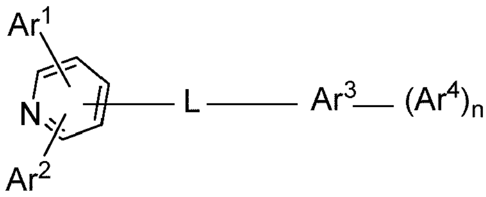

(I)

wherein,

Ar$^1$ and Ar$^2$ are same or different and independently selected from hydrogen, substituted or unsubstituted $C_6$ to $C_{36}$ aryl, substituted or unsubstituted $C_3$ to $C_{36}$ heteroaryl, wherein, at least one of the Ar$^1$ and Ar$^2$ group comprises a substituted or unsubstituted $C_3$ to $C_{36}$ heteroaryl;
L is a single bond, a substituted or unsubstituted $C_6$ to $C_{18}$ arylene, a substituted or unsubstituted $C_3$ to $C_{18}$ heteroarylene, a substituted or unsubstituted phenylene, a substituted or unsubstituted biphenylene, a substituted or unsubstituted terphenylene, a substituted or unsubstituted anthracenylene, a substituted or unsubstituted dibenzofuranylene, a substituted or unsubstituted dibenzothiophenylene, a substituted or unsubstituted carbazolylene, a substituted or unsubstituted pyridinylene, a substituted or unsubstituted phenylpyridinylene, a substituted or unsubstituted quinolinylene;
Ar$^3$ is a group selected from, substituted pyrazinylene, substituted or unsubstituted dibenzoacridinylene;
Ar$^4$ is a group independently selected from H, a substituted or unsubstituted $C_6$ to $C_{18}$ aryl, a substituted or unsubstituted $C_3$ to $C_{20}$ heteroaryl, an unsubstituted phenyl, an unsubstituted biphenyl, an unsubstituted naphthyl, unsubstituted anthracenyl, an unsubstituted phenanthridinyl, an unsubstituted pyridyl, an unsubstituted quinolinyl, an unsubstituted pryrimidyl, or having the formula (II) or (III),

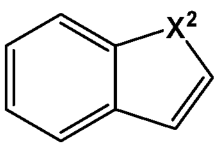

(II),

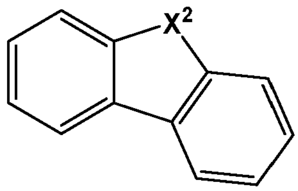

(III),

wherein the group of formula (II) and (III) are substituted or unsubstituted and X$^2$ is selected from N, S,O or C(R$^1$)$_2$;
n is 0, 1, 2, 3 or 4;

wherein the substituents of L, Ar$^1$, Ar$^2$, Ar$^3$, Ar$^4$ and/or N are independently selected from:

- $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, - PX$^3$(R$^2$)$_2$, D, F or CN;
- R$^1$ and R$^2$ are independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy and
- two R$^1$ can be linked together to form a ring
- X$^3$ is selected from S or O.

**15.** Compound according to claim 14, wherein L is selected from an unsubstituted phenylene or an unsubstituted biphenylene.

**Patentansprüche**

**1.** Organische elektronische Vorrichtung, umfassend eine Anode, eine Kathode und mindestens eine organische Halbleiterschicht, wobei die organische elektronische Vorrichtung ferner mindestens eine Emissionsschicht umfasst und die mindestens eine organische Halbleiterschicht zwischen der mindestens einen Emissionsschicht und der Kathode angeordnet ist, wobei die mindestens eine organische Halbleiterschicht Folgendes umfasst:

- einen Metall-Dotanden und
- eine durch die folgende Formel (I) wiedergegebene Verbindung:

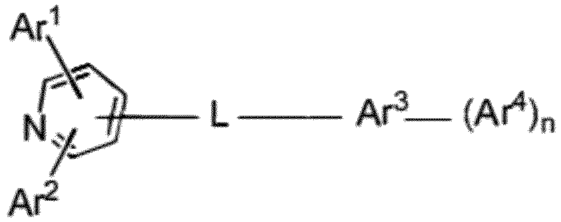

(I)

wobei

Ar[1] und Ar[2] gleich oder verschieden sind und unabhängig aus Wasserstoff, substituiertem oder unsubstituiertem $C_6$-bis $C_{36}$-Aryl, substituiertem oder unsubstituiertem $C_3$- bis $C_{36}$-Heteroaryl ausgewählt sind, wobei mindestens eine der Ar[1]- und Ar[2]-Gruppen ein substituiertes oder unsubstituiertes $C_3$-bis $C_{36}$-Heteroaryl umfasst;
L für eine Einfachbindung, ein substituiertes oder unsubstituiertes $C_6$-bis $C_{18}$-Arylen, ein substituiertes oder unsubstituiertes $C_3$- bis $C_{18}$-Heteroarylen, ein substituiertes oder unsubstituiertes Phenylen, ein substituiertes oder unsubstituiertes Biphenylen, ein substituiertes oder unsubstituiertes Terphenylen, ein substituiertes oder unsubstituiertes Antracenylen, ein substituiertes oder unsubstituiertes Dibenzofuranylen, ein substituiertes oder unsubstituiertes Dibenzothiophenylen, ein substituiertes oder unsubstituiertes Carbazolylen, ein substituiertes oder unsubstituiertes Pyridinylen, ein substituiertes oder unsubstituiertes Phenylpyridinylen, ein substituiertes oder unsubstituiertes Chinolinylen steht;
Ar[3] für eine Gruppe steht, die aus einem substituiertem $C_4$-N-Heteroarylen, einem substituierten oder unsubstituierten $C_{12}$-Heteroarylen mit mindestens drei anellierten Ringen und einem aus O, S oder N ausgewählten Heteroatom, einem substituierten Pyrazinylen, einem substituierten Pyrimidinylen, einem substituierten oder unsubstituierten Acrylidinylen, einem substituierten oder unsubstituierten Benzoacridinylen, einem substituierten oder unsubstituierten Dibenzoacridinylen, einem substituierten oder unsubstituierten Dibenzofuranylen, einem substituierten oder unsubstituierten Carbazolylen, einem substituierten oder unsubstituierten Benzochinolinylen, einem substituierten oder unsubstituierten Phenanthridinylen, einem substituierten oder unsubstituierten Dinaphthofuranylen oder einem substituierten oder unsubstituierten Dinaphthothiophenylen ausgewählt ist;
Ar[4] für eine Gruppe steht, die unabhängig aus H, einem substituierten oder unsubstituierten $C_6$- bis $C_{18}$-Aryl, einem substituierten oder unsubstituierten $C_3$-bis $C_{20}$-Heteroaryl, einem unsubstituierten Phenyl, einem unsubstituierten Biphenyl, einem unsubstituierten Naphthyl, einem unsubstituierten Anthracenyl, einem unsubstituierten Phenanthridinyl, einem unsubstituierten Pyridyl, einem unsubstituierten Chinolinyl, einem unsubstituierten Pyrimidinyl ausgewählt ist, oder die Formel (II) oder (III) aufweist,

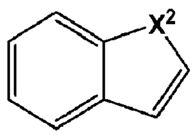

(II),

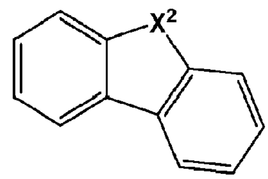

(III),

wobei die Gruppen der Formel (II) und (III) substituiert oder unsubstituiert sind und $X^2$ aus N, S, O oder $C(R^1)_2$ ausgewählt ist;
n für 0, 1, 2, 3 oder 4 steht;

wobei die Substituenten von L, Ar[1], Ar[2], Ar[3], Ar[4] und/oder N unabhängig aus:

- $C_6$- bis $C_{18}$-Aryl, $C_3$- bis $C_{20}$-Heteroaryl, $C_1$- bis $C_{16}$-Alkyl, $C_1$- bis $C_{16}$-Alkoxy, verzweigtem $C_3$- bis $C_{16}$-Alkyl, cyclischem $C_3$- bis $C_{16}$-Alkyl, verzweigtem $C_3$- bis $C_{16}$-Alkoxy, cyclischem $C_3$- bis $C_{16}$-Alkoxy, teil- oder perfluoriertem $C_1$- bis $C_{16}$-Alkyl, teil- oder perfluoriertem $C_1$- bis $C_{16}$-Alkoxy, teil- oder perdeuteriertem $C_1$- bis $C_{16}$-Alkyl, teil- oder perdeuteriertem $C_1$- bis $C_{16}$-Alkoxy, $PX^3(R^2)_2$, D, F oder CN ausgewählt sind;

- $R^1$ und $R^2$ unabhängig aus $C_6$- bis $C_{12}$-Aryl, $C_3$- bis $C_{12}$-Heteroaryl, $C_1$- bis $C_{16}$-Alkyl, $C_1$- bis $C_{16}$-Alkoxy, teil- oder perfluoriertem $C_1$- bis $C_{16}$-Alkyl, teil- oder perfluoriertem $C_1$- bis $C_{16}$-Alkoxy, teil- oder perdeuteriertem $C_1$- bis $C_{16}$-Alkyl, teil- oder perdeuteriertem $C_1$- bis $C_{16}$-Alkoxy ausgewählt sind und
- zwei $R^1$ miteinander zu einem Ring verknüpft sein können;
- $X^3$ aus S oder O ausgewählt ist.

**2.** Organische elektronische Vorrichtung nach Anspruch 1, wobei

- $Ar^1$ und $Ar^2$ unabhängig aus einer Wasserstoff-, Phenyl-, Pyridyl-, Phenylpyridyl- oder Chinolinylgruppe ausgewählt sind; oder
- $Ar^1$ für Wasserstoff steht und $Ar^2$ für eine Pyridyl-, Phenylpyridyl- oder Chinolinylgruppe steht;
- $Ar^1$ für Phenyl steht und $Ar^2$ für eine Pyridyl-, Phenylpyridyl- oder Chinolinylgruppe steht;
- $Ar^1$ für Wasserstoff steht und $Ar^2$ für eine Pyridylgruppe steht;
- $Ar^1$ für Phenyl steht und $Ar^2$ für eine Pyridylgruppe steht; oder
- $Ar^1$ und $Ar^2$ für Pyridylgruppen stehen.

**3.** Organische elektronische Vorrichtung nach Anspruch 1 oder 2, wobei
L für eine Einfachbindung steht oder aus einem unsubstituierten Phenylen, einem unsubstituierten Biphenylen, einem unsubstituierten Terphenylen, einem unsubstituierten Anthracenylen, einem unsubstituierten Dibenzofuranylen, einem unsubstituierten Dibenzothiophenylen, einem unsubstituierten Pyridinylen, einem substituierten oder unsubstituierten Phenylpyridinylen ausgewählt ist, vorzugsweise L für eine Einfachbindung steht oder aus unsubstituiertem Phenylen oder unsubstituiertem Biphenylen ausgewählt ist.

**4.** Organische elektronische Vorrichtung nach einem der vorhergehenden Ansprüche 1 bis 3, wobei $Ar^3$ für eine Gruppe steht, die aus einem substituierten Pyrazinylen, einem substituierten Pyrimidinylen, einem unsubstituierten Benzoacridinylen oder einem unsubstituierten Dibenzoacridinylen ausgewählt ist.

**5.** Organische elektronische Vorrichtung nach einem der vorhergehenden Ansprüche 1 bis 4, wobei

- $Ar^4$ für eine Gruppe, die aus H, einem unsubstituierten Phenyl, einem unsubstituierten Biphenyl, einem unsubstituiertem Pyridyl, einem unsubstituierten Naphthyl, einem unsubstituierten Dibenzofuranyl, einem unsubstituierten Benzofuranyl, einem unsubstituierten Dibenzothiophenyl, einem unsubstituierten Benzothiophenyl ausgewählt ist; oder
- H

steht.

**6.** Organische elektronische Vorrichtung nach einem der vorhergehenden Ansprüche 1 bis 5, wobei es sich bei dem Metall-Dotanden um:

- ein Metall, das aus der Gruppe bestehend aus Alkalimetallen, Erdalkalimetallen und Seltenerdmetallen ausgewählt ist;
- ein Metall, das aus Li, Na, Cs, Mg, Ca, Sr, Sm oder Yb ausgewählt ist;
- ein Metall, das aus Li, Cs, Mg oder Yb ausgewählt ist; oder
- ein Metall, das aus Li oder Yb ausgewählt ist; handelt.

**7.** Organische elektronische Vorrichtung nach einem der vorhergehenden Ansprüche 1 bis 6, wobei L unabhängig aus B1 bis B21 ausgewählt ist:

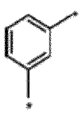, (B1)
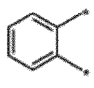, (B2)
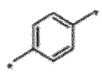, (B3)
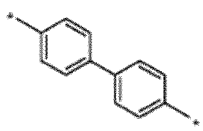, (B4)
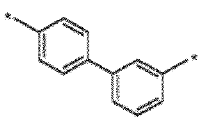, (B5),
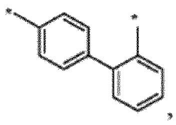, (B6),

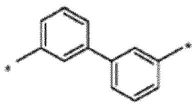, 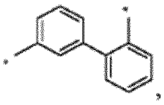, 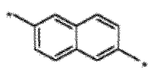, 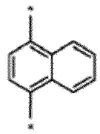, 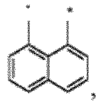,

(B7) (B8) (B9) (B10) (B11),

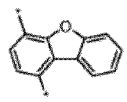, 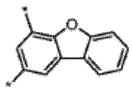, 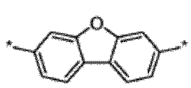, 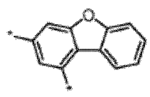, 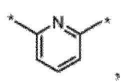, 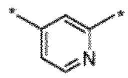,

(B12) (B13) (B14) (B15) (B16), (B17),

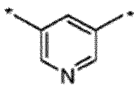, 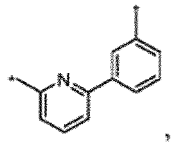, 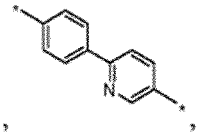, 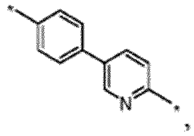,

(B18) (B19) (B20) (B21);

wobei das Sternchen-Symbol "*" die Anbindungsstelle von L repräsentiert.

**8.** Organische elektronische Vorrichtung nach einem der vorhergehenden Ansprüche 1 bis 7, wobei das Metall des Metall-Dotanden eine Elektronegativität von etwa ≥ 0,7 bis etwa ≤ 1,3 gemäß der Pauling-Skala aufweist und wobei das Metall des Metall-Dotanden vorzugsweise eine Elektronegativität von etwa ≥ 0,9 bis etwa ≤ 1,2, weiter bevorzugt etwa ≥ 1 bis etwa ≤ 1,1, aufweist.

**9.** Organische elektronische Vorrichtung nach einem der vorhergehenden Ansprüche 1 bis 8, wobei $Ar^3$ unabhängig aus E1 bis E9 ausgewählt ist:

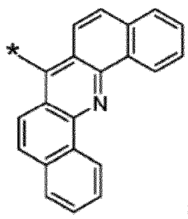 (E1), 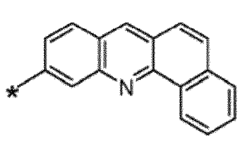 (E2), 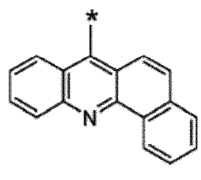 (E3), 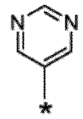 (E4),

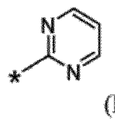

(E5),

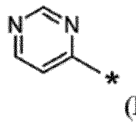 (E6), 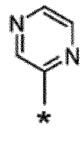 (E7), 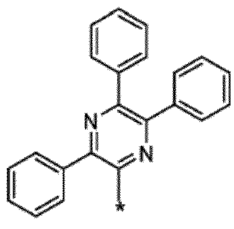 E8, 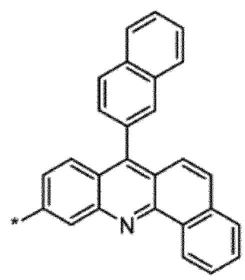 E9,

wobei das Sternchen-Symbol "*" die Anbindungsstelle von $Ar^3$ an L repräsentiert und dann, wenn n = 1, 2, 3 oder 4, $Ar^4$ an einen Kohlenstoff von $Ar^3$ mit Ausnahme der Anbindungsstelle von $Ar^3$ an L gebunden ist.

**10.** Organische elektronische Vorrichtung nach einem der vorhergehenden Ansprüche 1 bis 9, wobei $Ar^4$ unabhängig aus Wasserstoff oder F1 bis F13 ausgewählt ist:

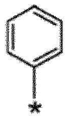 (F1), 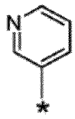 (F2), 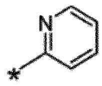 (F3), 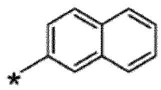 (F4), 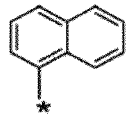 (F5), 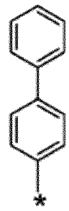 (F5),

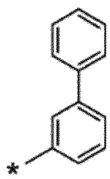 (F6), 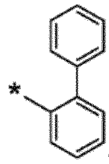 (F7), 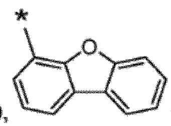 (F8), 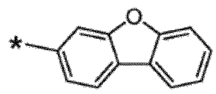 (F8),

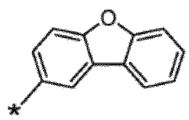 (F8), 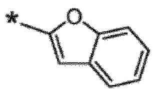 (F9), 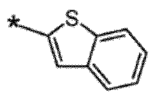 (F10), 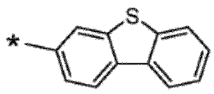 (F11),

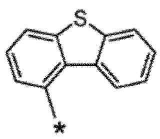 (F12), 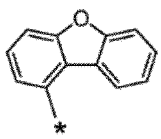 (F13);

wobei das Sternchen-Symbol "*" die Anbindungsstelle von $Ar^4$ an $Ar^3$ repräsentiert und $Ar^4$ an einen Kohlenstoff von $Ar^3$ mit Ausnahme der Anbindungsstelle von $Ar^3$ an L gebunden ist.

**11.** Organische elektronische Vorrichtung nach einem der vorhergehenden Ansprüche 1 bis 10, wobei gemäß Formel (I) $Ar^3$-$(Ar^4)_n$ unabhängig aus D1 bis D12 ausgewählt ist:

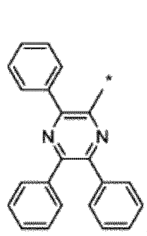, 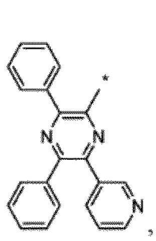, 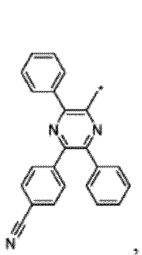, 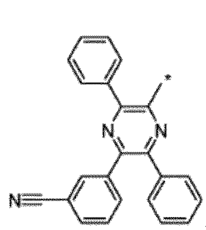, 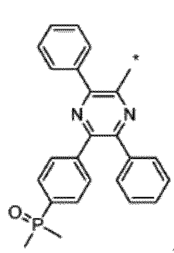,

D1, D2 D3 D4 D5

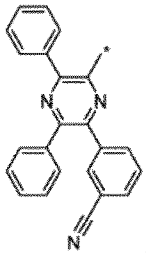
D6,
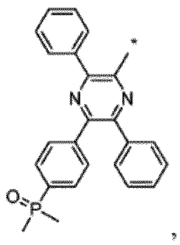
D7,
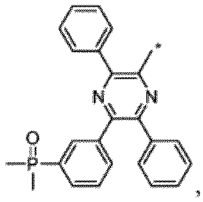
D8,
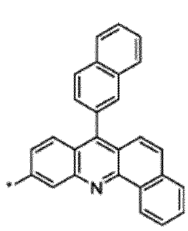
D9,
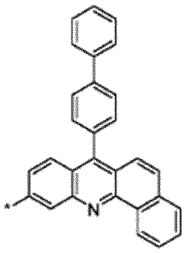
D10

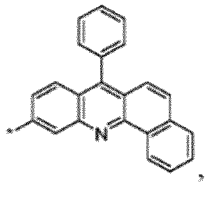
D11,
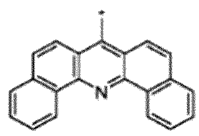
D12;

wobei das Sternchen-Symbol "*" die Anbindungsstelle von $Ar^3$ an L repräsentiert.

**12.** Organische elektronische Vorrichtung nach einem der vorhergehenden Ansprüche 1 bis 11, wobei die Verbindung der Formel (I) aus G1 bis G36 ausgewählt ist:

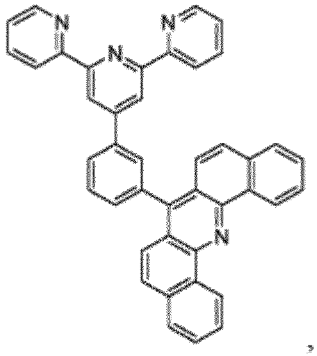
,
(G1)

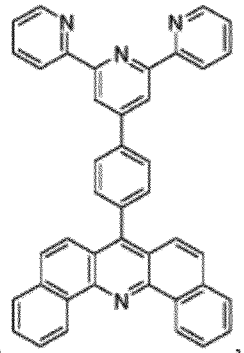
,
(G2)

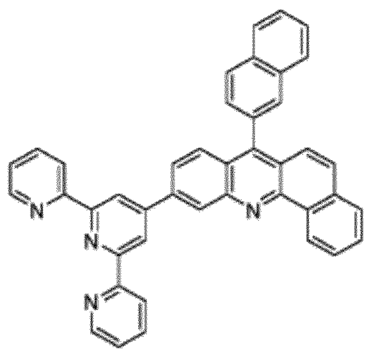
(G3)

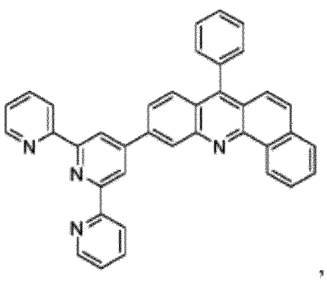
,
(G4)

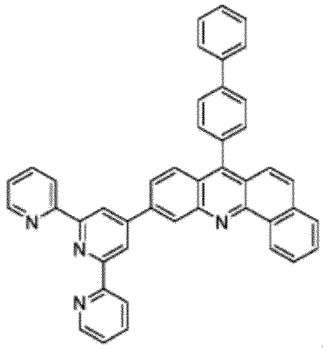
,
(G5)

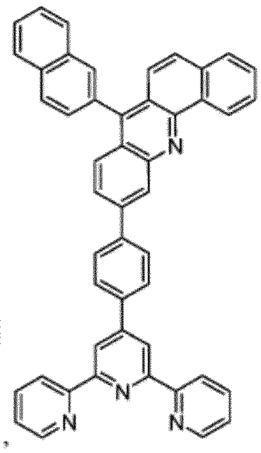
,
(G6)

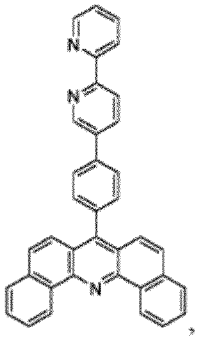,
(G7)

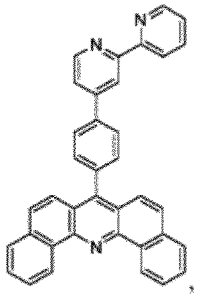,
(G8)

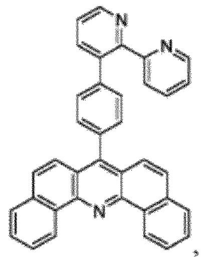,
(G9)

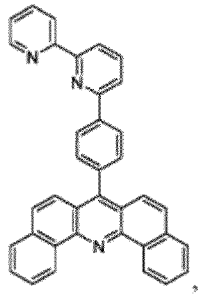,
(G10)

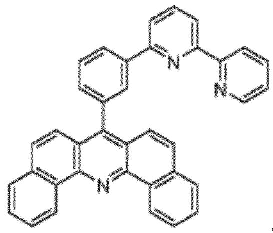,
(G11)

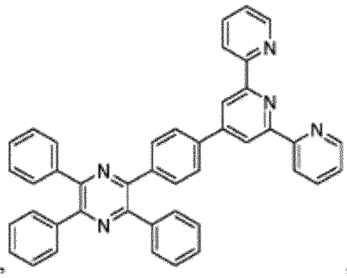,
(G12)

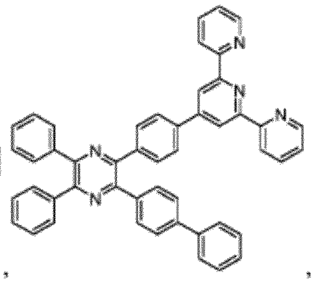,
(G13)

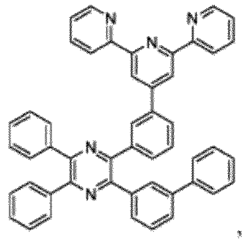,
(G14)

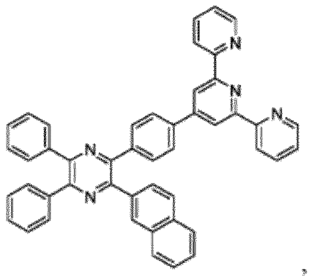,
(G15)

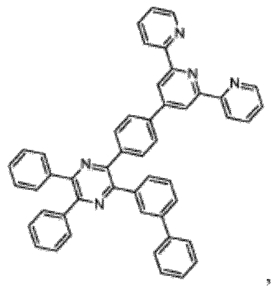,
(G16)

,
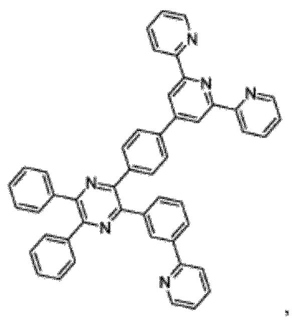,
(G17)

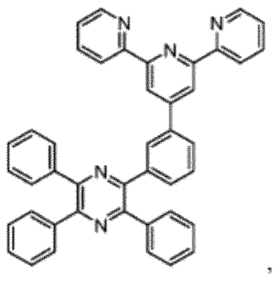,
(G18)

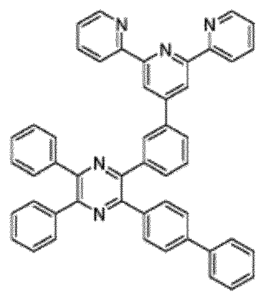,
(G19)

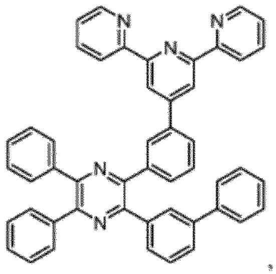
,
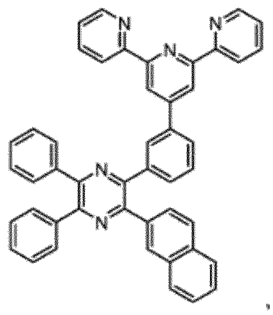
,
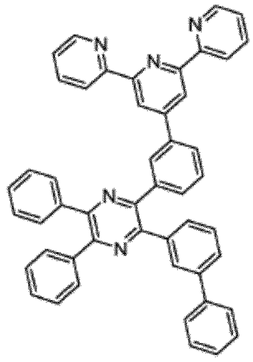
,

(G20) (G21) (G22)

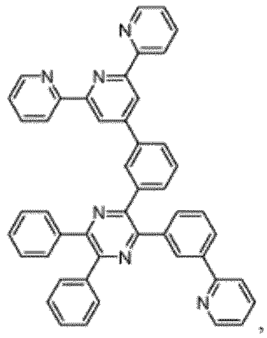
,
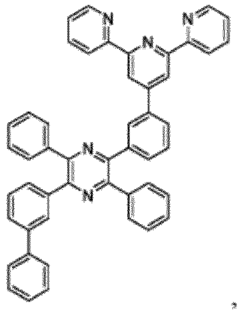
,
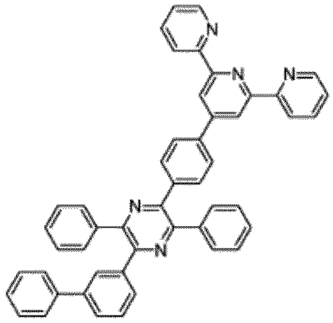

(G23) (G24) (G25)

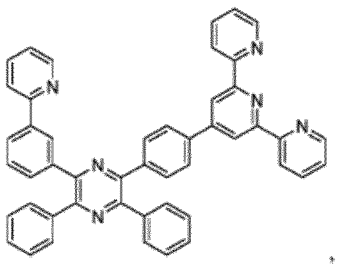
,
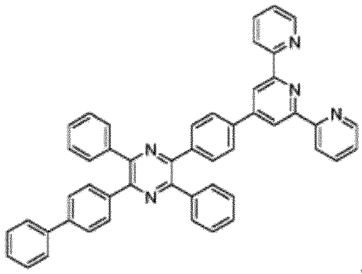
,
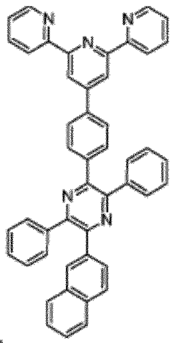
,

(G26) (G27) (G28)

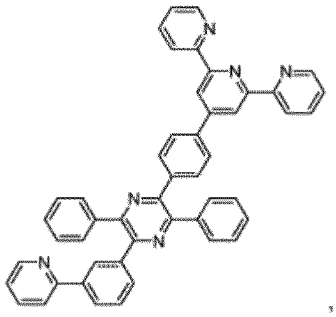
,
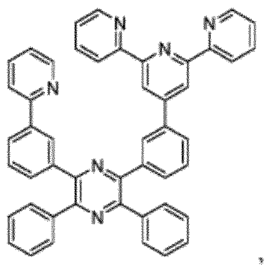
,
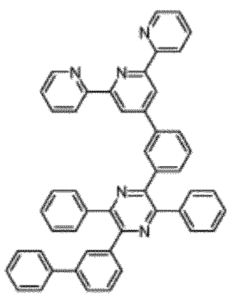

(G29) (G30) (G31)

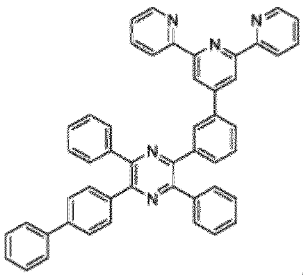
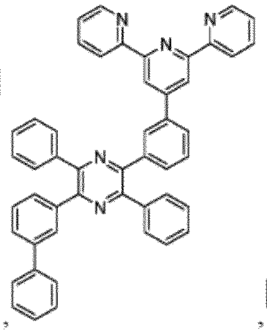
,
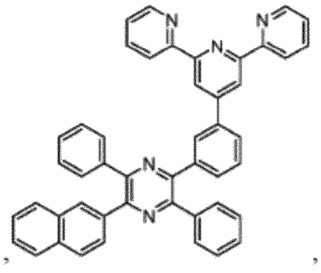
,

(G32) (G33) (G34)

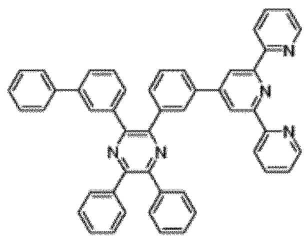
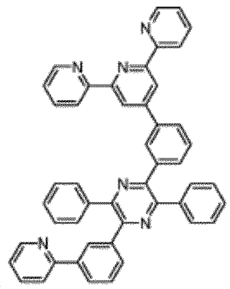
,

(G35) (G36).

**13.** Organische elektronische Vorrichtung nach den Ansprüchen 1 bis 12, wobei es sich bei der elektronischen Vorrichtung um eine lichtemittierende Vorrichtung, einen Dünnschichttransistor, eine Batterie, eine Anzeigevorrichtung oder eine Photovoltaikzelle und vorzugsweise eine lichtemittierende Vorrichtung handelt.

**14.** Verbindung, die durch folgende Formel (I) wiedergegeben wird:

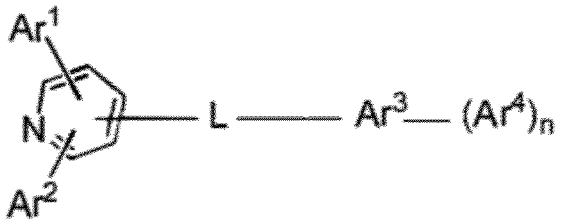

(I)

wobei

$Ar^1$ und $Ar^2$ gleich oder verschieden sind und unabhängig aus Wasserstoff, substituiertem oder unsubstituiertem $C_6$-bis $C_{36}$-Aryl, substituiertem oder unsubstituiertem $C_3$- bis $C_{36}$-Heteroaryl ausgewählt sind, wobei mindestens eine der $Ar^1$- und $Ar^2$-Gruppen ein substituiertes oder unsubstituiertes $C_3$-bis $C_{36}$-Heteroaryl umfasst;

L für eine Einfachbindung, ein substituiertes oder unsubstituiertes $C_6$-bis $C_{18}$-Arylen, ein substituiertes oder unsubstituiertes $C_3$- bis $C_{18}$-Heteroarylen, ein substituiertes oder unsubstituiertes Phenylen, ein substituiertes oder unsubstituiertes Biphenylen, ein substituiertes oder unsubstituiertes Terphenylen, ein substituiertes oder unsubstituiertes Antracenylen, ein substituiertes oder unsubstituiertes Dibenzofuranylen, ein substituiertes oder unsubstituiertes Dibenzothiophenylen, ein substituiertes oder unsubstituiertes Carbazolylen, ein substituiertes oder unsubstituiertes Pyridinylen, ein substituiertes oder unsubstituiertes Phenylpyridinylen, ein substituiertes oder unsubstituiertes Chinolinylen steht;

$Ar^3$ für eine Gruppe steht, die aus einem substituierten Pyrazinylen, einem substituierten oder unsubstituierten Dibenzoacridinylen ausgewählt ist;

$Ar^4$ für eine Gruppe steht, die unabhängig aus H, einem substituierten oder unsubstituierten $C_6$- bis $C_{18}$-Aryl, einem substituierten oder unsubstituierten $C_3$-bis $C_{20}$-Heteroaryl, einem unsubstituierten Phenyl, einem unsubstituierten Biphenyl, einem unsubstituierten Naphthyl, einem unsubstituierten Anthracenyl, einem unsubstituierten Phenanthridinyl, einem unsubstituierten Pyridyl, einem unsubstituierten Chinolinyl, einem unsubstituierten Pyrimidinyl ausgewählt ist, oder die Formel (II) oder (III) aufweist,

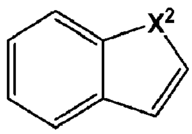

(II),

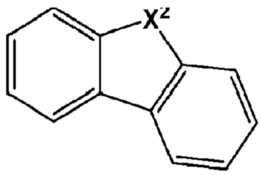

(III),

wobei die Gruppen der Formel (II) und (III) substituiert oder unsubstituiert sind und $X^2$ aus N, S, O oder $C(R^1)_2$ ausgewählt ist;
n für 0, 1, 2, 3 oder 4 steht;

wobei die Substituenten von L, $Ar^1$, $Ar^2$, $Ar^3$, $Ar^4$ und/oder N unabhängig aus:

- $C_6$- bis $C_{18}$-Aryl, $C_3$- bis $C_{20}$-Heteroaryl, $C_1$- bis $C_{16}$-Alkyl, $C_1$- bis $C_{16}$-Alkoxy, verzweigtem $C_3$- bis $C_{16}$-Alkyl, cyclischem $C_3$- bis $C_{16}$-Alkyl, verzweigtem $C_3$- bis $C_{16}$-Alkoxy, cyclischem $C_3$- bis $C_{16}$-Alkoxy, teil- oder perfluoriertem $C_1$- bis $C_{16}$-Alkyl, teil- oder perfluoriertem $C_1$- bis $C_{16}$-Alkoxy, teil- oder perdeuteriertem $C_1$- bis $C_{16}$-Alkyl, teil- oder perdeuteriertem $C_1$- bis $C_{16}$-Alkoxy, $PX^3(R^2)_2$, D, F oder CN ausgewählt sind;
- $R^1$ und $R^2$ unabhängig aus $C_6$- bis $C_{12}$-Aryl, $C_3$- bis $C_{12}$-Heteroaryl, $C_1$- bis $C_{16}$-Alkyl, $C_1$- bis $C_{16}$-Alkoxy, teil- oder perfluoriertem $C_1$- bis $C_{16}$-Alkyl, teil- oder perfluoriertem $C_1$- bis $C_{16}$-Alkoxy, teil- oder perdeuteriertem $C_1$- bis $C_{16}$-Alkyl, teil- oder perdeuteriertem $C_1$- bis $C_{16}$-Alkoxy ausgewählt sind und
- zwei $R^1$ miteinander zu einem Ring verknüpft sein können;
- $X^3$ aus S oder O ausgewählt ist.

**15.** Verbindung nach Anspruch 14, wobei L aus einem unsubstituierten Phenylen und einem unsubstituierten Biphenylen ausgewählt ist.

## Revendications

**1.** Dispositif électronique organique comprenant une anode, une cathode et au moins une couche de semi-conducteur organique, le dispositif électronique organique comprenant en outre au moins une couche d'émission et l'au moins une couche de semi-conducteur organique étant agencée entre l'au moins une couche d'émission et la cathode, l'au moins une couche de semi-conducteur organique comprenant :

- un dopant métallique, et
- un composé représenté par la formule suivante (I) :

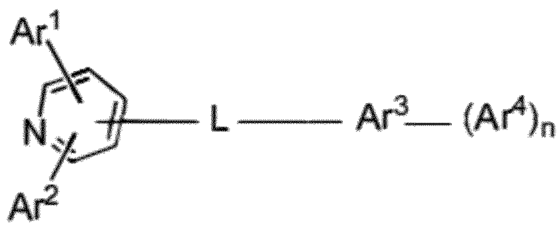

(I)

$Ar^1$ et $Ar^2$ étant identiques ou différents et indépendamment choisis parmi hydrogène, $C_6$ à $C_{36}$ aryle substitué ou non substitué, $C_3$ à $C_{36}$ hétéroaryle substitué ou non substitué, au moins l'un parmi le groupe $Ar^1$ et $Ar^2$ comprenant un $C_3$ à $C_{36}$ hétéroaryle substitué ou non substitué ;
L étant une simple liaison, un $C_6$ à $C_{18}$ arylène substitué ou non substitué, un $C_3$ à $C_{18}$ hétéroarylène substitué ou non substitué, un phénylène substitué ou non substitué, un biphénylène substitué ou non substitué, un terphénylène substitué ou non substitué, un anthracénylène substitué ou non substitué, un dibenzofuranylène substitué ou non substitué, un dibenzothiophénylène substitué ou non substitué, une carbazolylène substituée ou non substituée, un pyridinylène substitué ou non substituée, un phénylpyridinylène substitué ou non substitué, un quinoléinylène substitué ou non substitué ;

$Ar^3$ étant un groupe choisi parmi un N-hétéroarylène en $C_4$ substitué, un $C_{12}$ à $C_{25}$ hétéroarylène substitué ou non substitué comprenant au moins trois cycles condensés et un hétéroatome choisi parmi O, S ou N, un pyrazinylène substitué, un pyrimidinylène substitué, un acridinylène substitué ou non substitué, un benzoacridinylène substitué ou non substitué, un dibenzoacridinylène substitué ou non substitué, un dibenzofuranylène substitué ou non substitué, un carbazolylène substitué ou non substitué, un benzoquinoléinylène substitué ou non substitué, un phénanthridinylène substitué ou non substitué, un dinaphtofuranylène substitué ou non substitué ou un dinaphtothiophénylène substitué ou non substitué ;
$Ar^4$ étant un groupe indépendamment choisi parmi H, un $C_6$ à $C_{18}$ aryle substitué ou non substitué, un $C_3$ à $C_{20}$ hétéroaryle substitué ou non substitué, un phényle non substitué, un biphényle non substitué, un naphtyle non substitué, un anthracényle non substitué, un phénanthridinyle non substitué, un pyridinyle non substitué, un quinoléinyle non substitué, un pyrimidinyle non substitué, ou ayant la formule (II) ou (III),

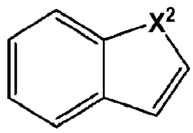 (II), 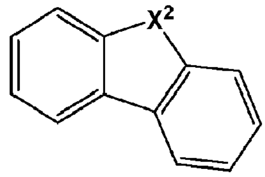 (III),

les groupes de formule (II) et (III) étant non substitués ou substitués et $X^2$ étant choisi parmi N, S,O ou $C(R^1)_2$ ;
n étant 0, 1, 2, 3 ou 4 ;
les substituants de L, $Ar^1$, $Ar^2$, $Ar^3$, $Ar^4$ et/ou N étant indépendamment choisis parmi :

- $C_6$ à $C_{18}$ aryle, $C_3$ à $C_{20}$ hétéroaryle, $C_1$ à $C_{16}$ alkyle, $C_1$ à $C_{16}$ alcoxy, $C_3$ à $C_{16}$ alkyle ramifié, $C_3$ à $C_{16}$ alkyle cyclique, $C_3$ à $C_{16}$ alcoxy ramifié, $C_3$ à $C_{16}$ alcoxy cyclique, $C_1$ à $C_{16}$ alkyle partiellement ou perfluoré, $C_1$ à $C_{16}$ alcoxy partiellement ou perfluoré, $C_1$ à $C_{16}$ alkyle partiellement ou perdeutéré, $C_1$ à $C_{16}$ alcoxy partiellement ou perdeutéré, $-PX^3(R^2)_2$, D, F ou CN ;
- $R^1$ et $R^2$ étant indépendamment choisis parmi $C_6$ à $C_{12}$ aryle, $C_3$ à $C_{12}$ hétéroaryle, $C_1$ à $C_{16}$ alkyle, $C_1$ à $C_{16}$ alcoxy, $C_1$ à $C_{16}$ alkyle partiellement ou perfluoré, $C_1$ à $C_{16}$ alcoxy partiellement ou perfluoré, $C_1$ à $C_{16}$ alkyle partiellement ou perdeutéré, $C_1$ à $C_{16}$ alcoxy partiellement ou perdeutéré et
- deux $R^1$ pouvant être liés ensemble pour former un cycle
- $X^3$ étant choisi parmi S ou O.

**2.** Dispositif électronique organique selon la revendication 1,

- $Ar^1$ et $Ar^2$ étant indépendamment choisis parmi un groupe hydrogène, phényle, pyridinyle, phénylpyridinyle ou quinoléinyle ; ou
- $Ar^1$ étant hydrogène et $Ar^2$ étant un groupe pyridinyle, phénylpyridinyle ou quinoléinyle ;
- $Ar^1$ étant phényle et $Ar^2$ étant un groupe pyridinyle, phénylpyridinyle ou quinoléinyle ;
- $Ar^1$ étant hydrogène et $Ar^2$ étant un groupe pyridinyle ;
- $Ar^1$ étant phényle et $Ar^2$ étant un groupe pyridinyle ; ou
- $Ar^1$ et $Ar^2$ étant des groupes pyridinyle.

**3.** Dispositif électronique organique selon la revendication 1 ou 2,
L étant une simple liaison ou étant choisi parmi un phénylène non substitué, un biphénylène non substitué, un terphénylène non substitué, un anthracénylène non substitué, un dibenzofuranylène non substitué, un dibenzothiophénylène non substitué, un pyridinylène non substitué, un phénylpyridinylène substitué ou non substitué, préférablement L étant une simple liaison ou étant choisi parmi phénylène non substitué ou biphénylène non substitué.

**4.** Dispositif électronique organique selon l'une quelconque des revendications précédentes 1 à 3, $Ar^3$ étant un groupe choisi parmi un pyrazinylène substitué, un pyrimidinylène substitué, un benzoacridinylène non substitué ou un dibenzoacridinylène non substitué.

**5.** Dispositif électronique organique selon l'une quelconque des revendications précédentes 1 à 4,

- $Ar^4$ étant un groupe choisi parmi H, un phényle non substitué, un biphényle non substitué, un pyridinyle non substitué, un naphtyle non substitué, un dibenzofuranyle non substitué, un benzofuranyle non substitué, un

dibenzothiophényle non substitué, un benzothiophényle non substitué ; ou
- H.

6. Dispositif électronique organique selon l'une quelconque des revendications précédentes 1 à 5, le dopant métallique étant :

- un métal choisi dans le groupe constitué par des métaux alcalins, des métaux alcalino-terreux et des métaux des terres rares ;
- un métal choisi parmi Li, Na, Cs, Mg, Ca, Sr, Sm ou Yb ;
- un métal choisi parmi Li, Cs, Mg ou Yb ; ou
- un métal choisi parmi Li ou Yb.

7. Dispositif électronique organique selon l'une quelconque des revendications précédentes 1 à 6, L étant indépendamment choisi parmi B1 à B21 :

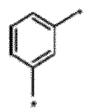, 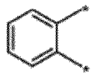, 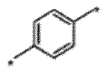, 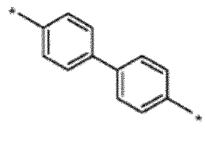, 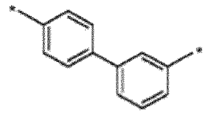, 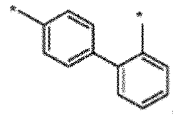,

(B1) (B2) (B3) (B4) (B5), (B6),

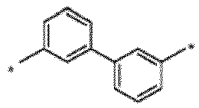, 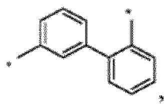, 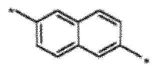, 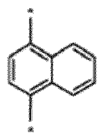, 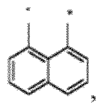,

(B7) (B8) (B9) (B10) (B11),

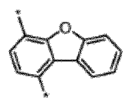, 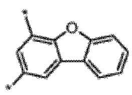, 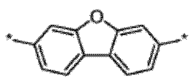, 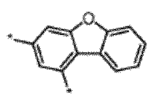, 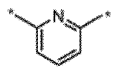, 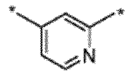,

(B12) (B13) (B14) (B15) (B16), (B17),

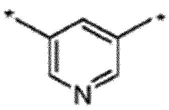, 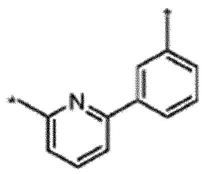, 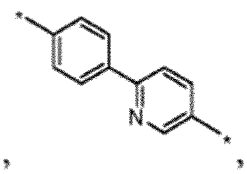, 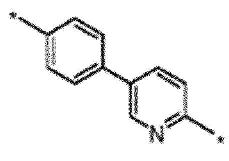,

(B18) (B19) (B20) (B21);

le symbole astérisque « * » représentant la position de liaison de L.

8. Dispositif électronique organique selon l'une quelconque des revendications précédentes 1 à 7, le métal du dopant métallique possédant une électronégativité d'environ ≥ 0,7 à environ ≤ 1,3 selon l'échelle de Pauling, et préférablement le dopant métallique possédant une électronégativité d'environ ≥ 0,9 à environ ≤ 1,2, de manière en outre préférée ≥ 1 à environ ≤ 1,1.

9. Dispositif électronique organique selon l'une quelconque des revendications précédentes 1 à 8, $Ar^3$ étant indépendamment choisi parmi E1 à E9 :

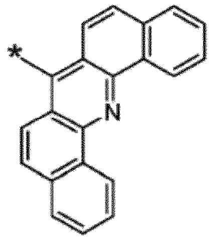 (E1), 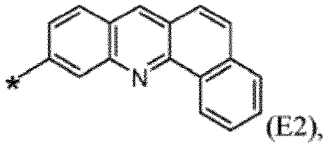 (E2), 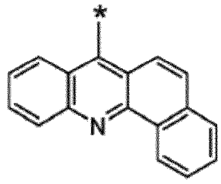 (E3), 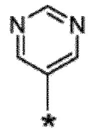 (E4),

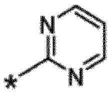 (E5),

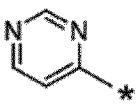 (E6), 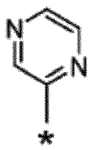 (E7), 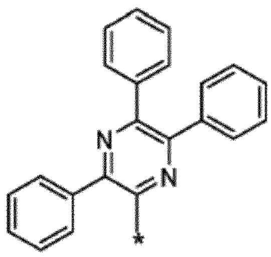 E8, 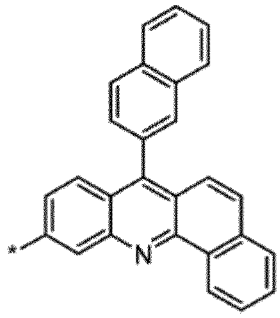 E9,

le symbole astérisque « * » représentant la position de liaison de $Ar^3$ à L et si n = 1, 2, 3 ou 4, $Ar^4$ se lie à un carbone de $Ar^3$ à l'exclusion de la position de liaison de $Ar^3$ à L.

**10.** Dispositif électronique organique selon l'une quelconque des revendications précédentes 1 à 9, $Ar^4$ étant indépendamment choisi parmi hydrogène ou F1 à F13 :

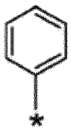 (F1), 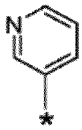 (F2), 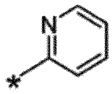 (F3), 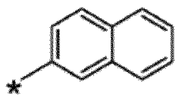 (F4), 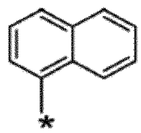 (F5), 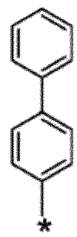 (F5),

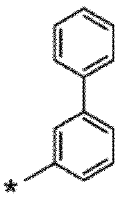 (F6), 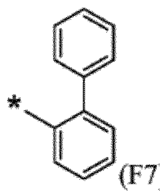 (F7), 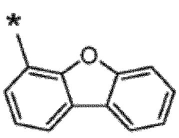 (F8), 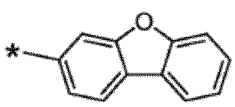 (F8),

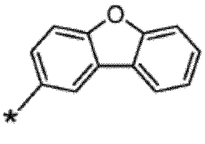 (F8), 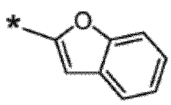 (F9), 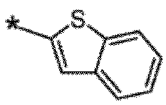 (F10), 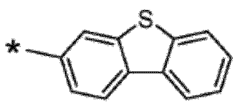 (F11),

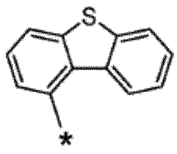

(F12),

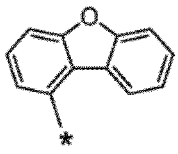

(F13);

le symbole astérisque « * » représentant la position de liaison de $Ar^4$ à $Ar^3$, et $Ar^4$ se liant à un carbone de $Ar^3$ à l'exclusion de la position de liaison de $Ar^3$ à L.

**11.** Dispositif électronique organique selon l'une quelconque des revendications précédentes 1 à 10, selon la formule (I) **$Ar^3$-($Ar^4$)$_n$** étant indépendamment choisi de D1 à D12 :

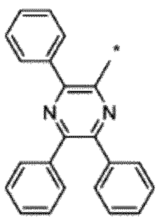, 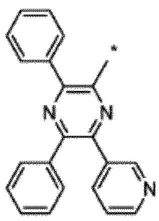, 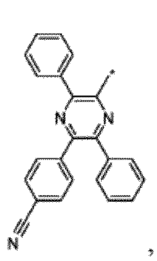, 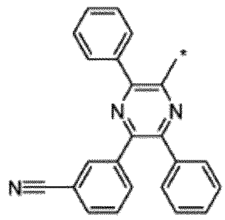, 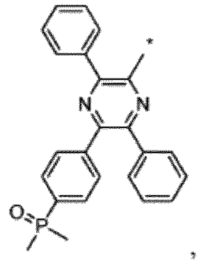,

D1, D2 D3 D4 D5

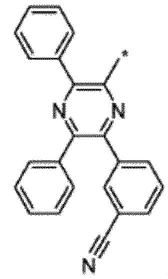, 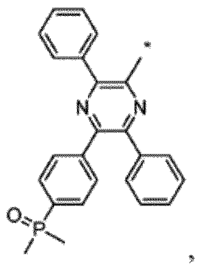, 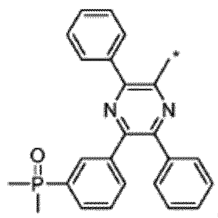, 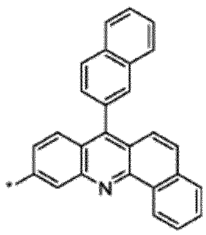, 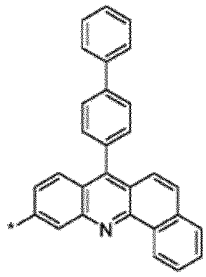

D6, D7, D8 D9 D10

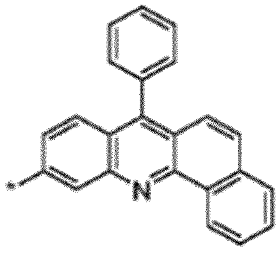, 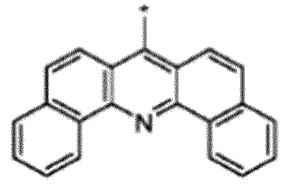

D11 D12;

le symbole astérisque « * » représentant la position de liaison de $Ar^3$ à L.

**12.** Dispositif électronique organique selon l'une quelconque des revendications précédentes 1 à 11, le composé de formule (I) étant choisi parmi G1 à G36 :

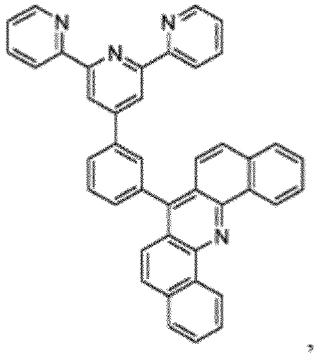,
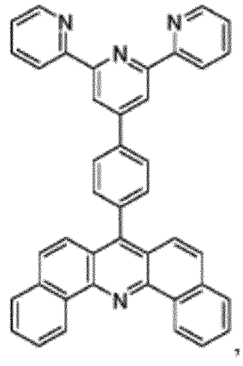,
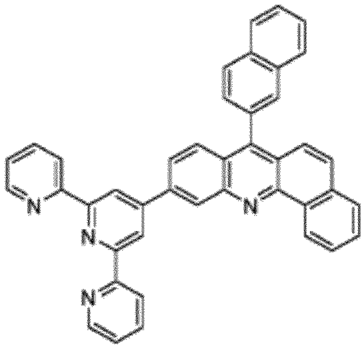

(G1) (G2) (G3)

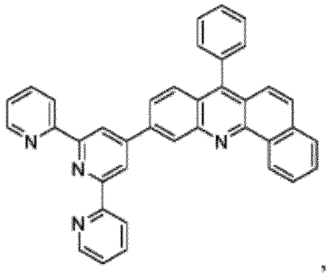,
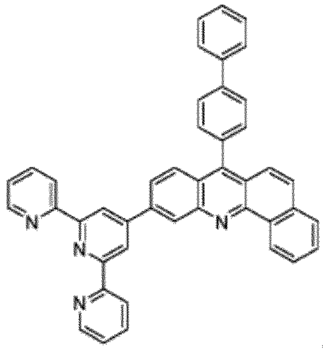,
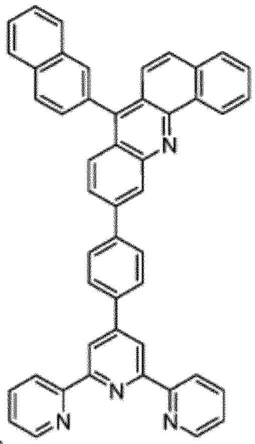,

(G4) (G5) (G6)

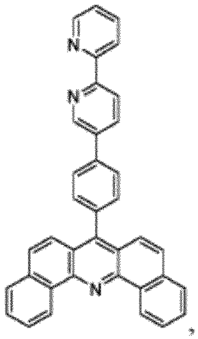,
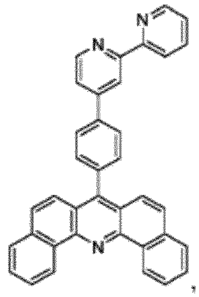,
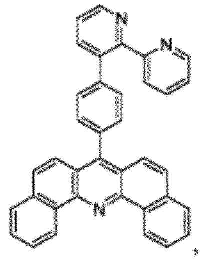,
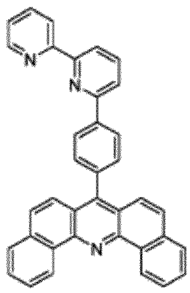,

(G7) (G8) (G9) (G10)

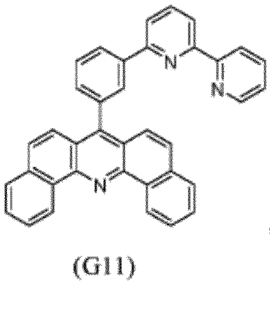,
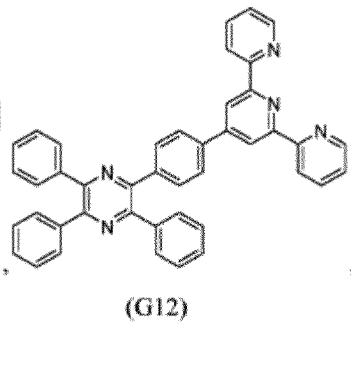,
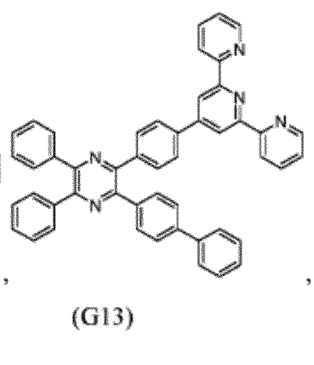,

(G11) (G12) (G13)

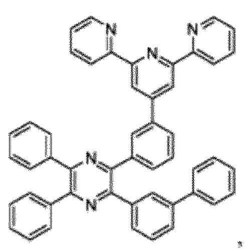,

(G14)

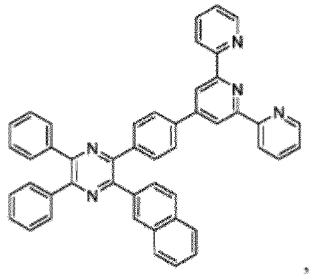,

(G15)

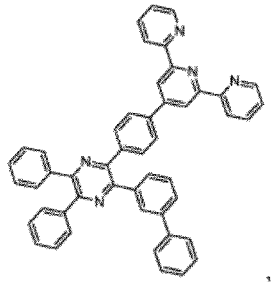,

(G16)

, 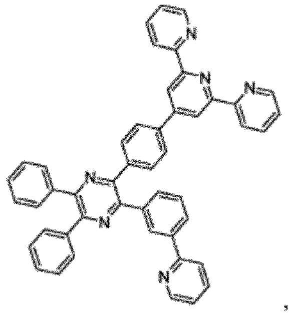,

(G17)

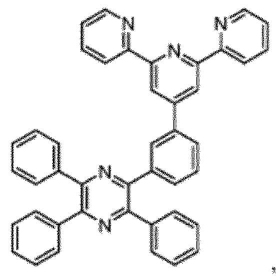,

(G18)

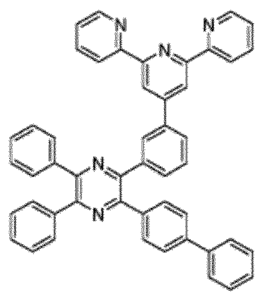,

(G19)

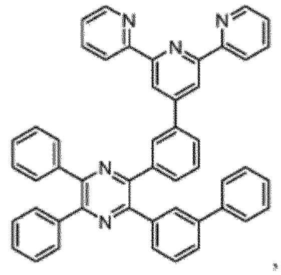,

(G20)

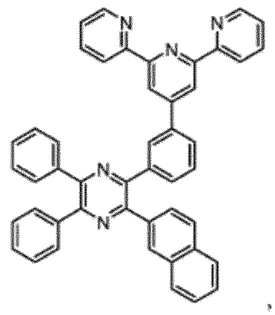,

(G21)

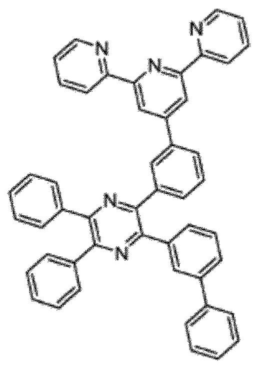,

(G22)

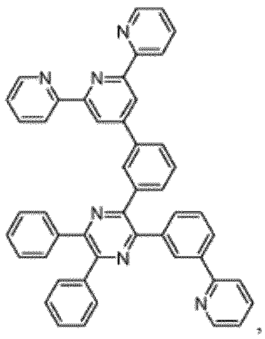,

(G23)

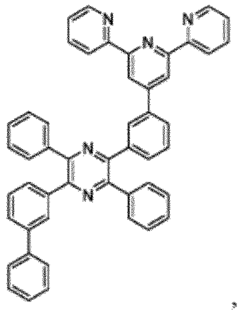,

(G24)

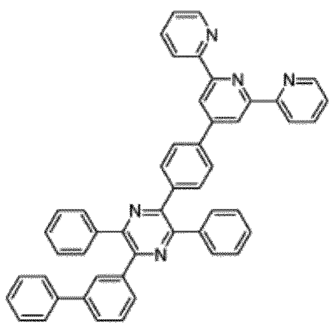

(G25)

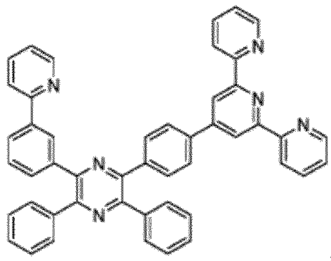
,
(G26)

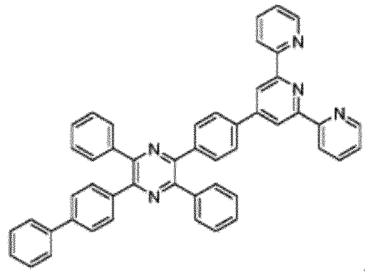
,
(G27)

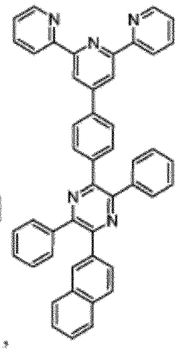
,
(G28)

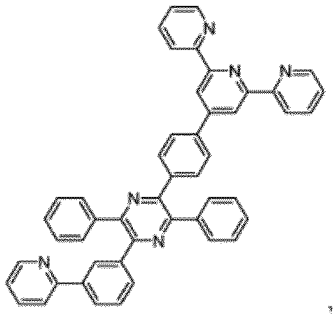
,
(G29)

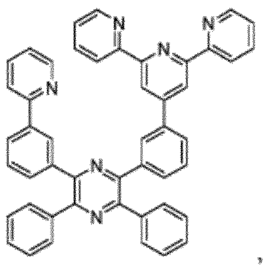
,
(G30)

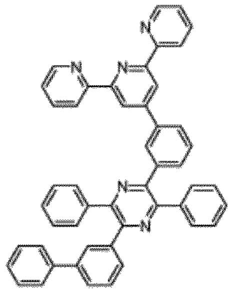

(G31)

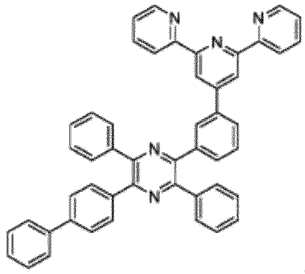

(G32)

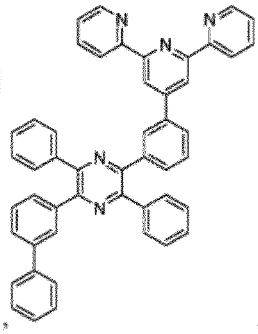
,
(G33)

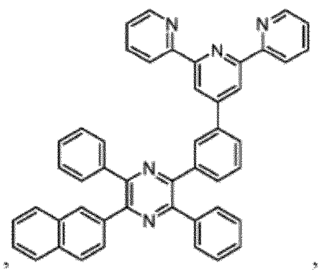
,
(G34)

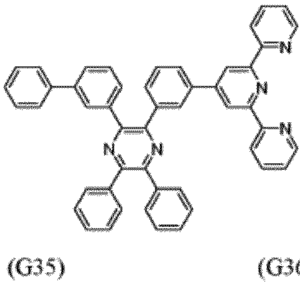
,
(G35)

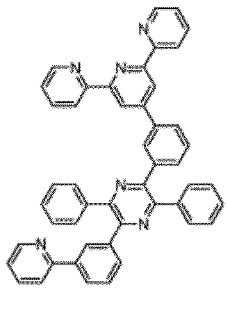

(G36).

**13.** Dispositif électronique organique selon l'une quelconque des revendications 1 à 12, le dispositif électronique étant un dispositif émetteur de lumière, un transistor en film mince, une batterie, un dispositif d'affichage ou une cellule photovoltaïque, et préférablement un dispositif émetteur de lumière.

**14.** Composé représenté par la formule suivante (I) :

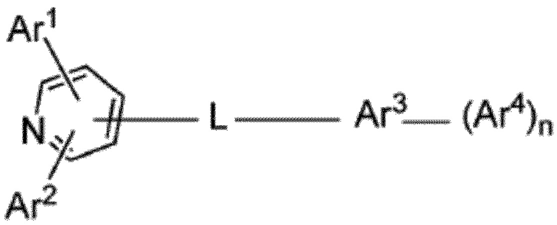

(I) ,

$Ar^1$ et $Ar^2$ étant identiques ou différents et indépendamment choisis parmi hydrogène, $C_6$ à $C_{36}$ aryle substitué ou non substitué, $C_3$ à $C_{36}$ hétéroaryle substitué ou non substitué, au moins l'un parmi le groupe $Ar^1$ et $Ar^2$ comprenant un $C_3$ à $C_{36}$ hétéroaryle substitué ou non substitué ;
L étant une simple liaison, un $C_6$ à $C_{18}$ arylène substitué ou non substitué, un $C_3$ à $C_{18}$ hétéroarylène substitué ou non substitué, un phénylène substitué ou non substitué, un biphénylène substitué ou non substitué, un terphénylène substitué ou non substitué, un anthracénylène substitué ou non substitué, un dibenzofuranylène substitué ou non substitué, un dibenzothiophénylène substitué ou non substitué, un carbazolylène substitué ou non substitué, un pyridinylène substitué ou non substitué, un phénylpyridinylène substitué ou non substitué, un quinoléinylène substitué ou non substitué ;
$Ar^3$ étant un groupe choisi parmi pyrazinylène substitué, dibenzoacridinylène substitué ou non substitué ;
$Ar^4$ étant un groupe indépendamment choisi parmi H, $C_6$ à $C_{18}$ aryle substitué ou non substitué, $C_3$ à $C_{20}$ hétéroaryle substitué ou non substitué, un phényle non substitué, un biphényle non substitué, un naphtyle non substitué, un anthracényle non substitué, un phénanthridinyle non substitué, un pyridinyle non substitué, un quinoléinyle non substitué, un pyrimidinyle non substitué, ou ayant la formule (II) ou (III),

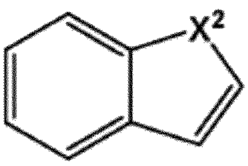

(II),

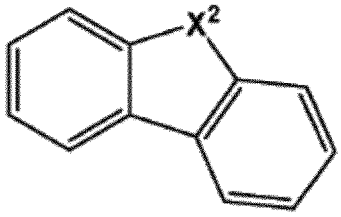

(III),

les groupes de formule (II) et (III) étant substitués ou non substitués et $X^2$ étant choisi parmi N, S,O ou $C(R^1)_2$ ;
n étant 0, 1, 2, 3 ou 4 ;
les substituants de L, $Ar^1$, $Ar^2$, $Ar^3$, $Ar^4$ et/ou N étant indépendamment choisis parmi :

- $C_6$ à $C_{18}$ aryle, $C_3$ à $C_{20}$ hétéroaryle, $C_1$ à $C_{16}$ alkyle, $C_1$ à $C_{16}$ alcoxy, $C_3$ à $C_{16}$ alkyle ramifié, $C_3$ à $C_{16}$ alkyle cyclique, $C_3$ à $C_{16}$ alcoxy ramifié, $C_3$ à $C_{16}$ alcoxy cyclique, $C_1$ à $C_{16}$ alkyle partiellement ou perfluoré, $C_1$ à $C_{16}$ alcoxy partiellement ou perfluoré, $C_1$ à $C_{16}$ alkyle partiellement ou perdeutéré, $C_1$ à $C_{16}$ alcoxy partiellement ou perdeutéré, $-PX^3(R^2)_2$, D, F ou CN ;
- $R^1$ et $R^2$ étant indépendamment choisis parmi $C_6$ à $C_{12}$ aryle, $C_3$ à $C_{12}$ hétéroaryle, $C_1$ à $C_{16}$ alkyle, $C_1$ à $C_{16}$ alcoxy, $C_1$ à $C_{16}$ alkyle partiellement ou perfluoré, $C_1$ à $C_{16}$ alcoxy partiellement ou perfluoré, $C_1$ à $C_{16}$ alkyle partiellement ou perdeutéré, $C_1$ à $C_{16}$ alcoxy partiellement ou perdeutéré et
- deux $R^1$ pouvant être liés ensemble pour former un cycle
- $X^3$ étant choisi parmi S ou O.

**15.** Composé selon la revendication 14, L étant choisi parmi un phénylène non substitué ou un biphénylène non substitué.

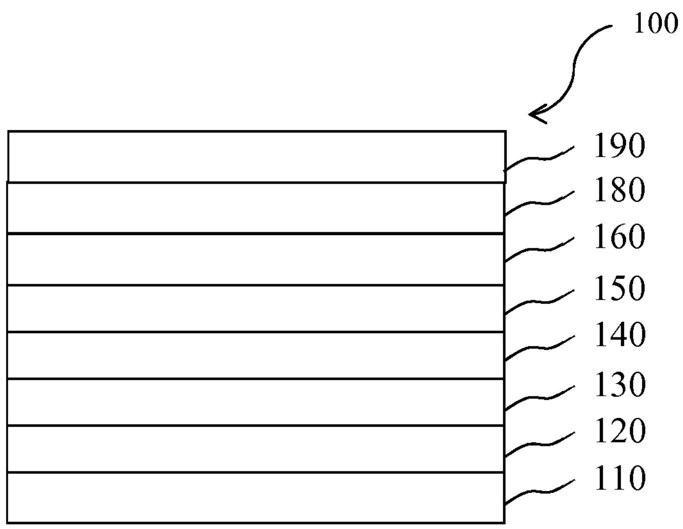
100
190
180
160
150
140
130
120
110

Fig.1

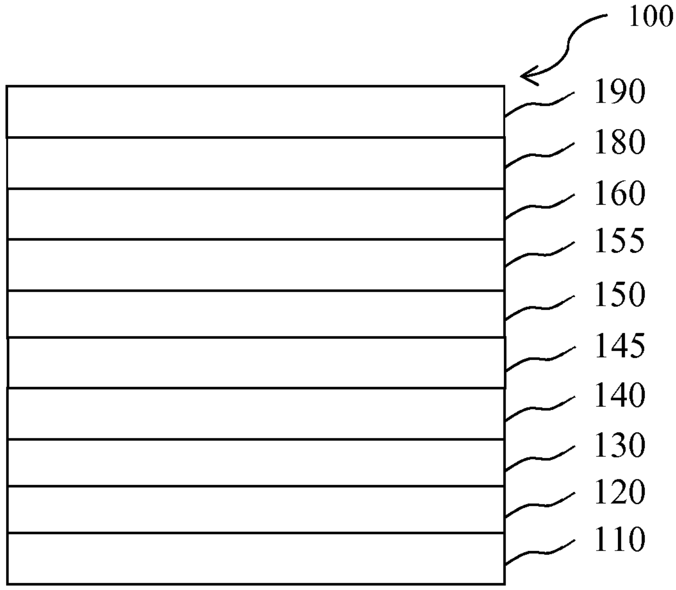
100
190
180
160
155
150
145
140
130
120
110

Fig.2

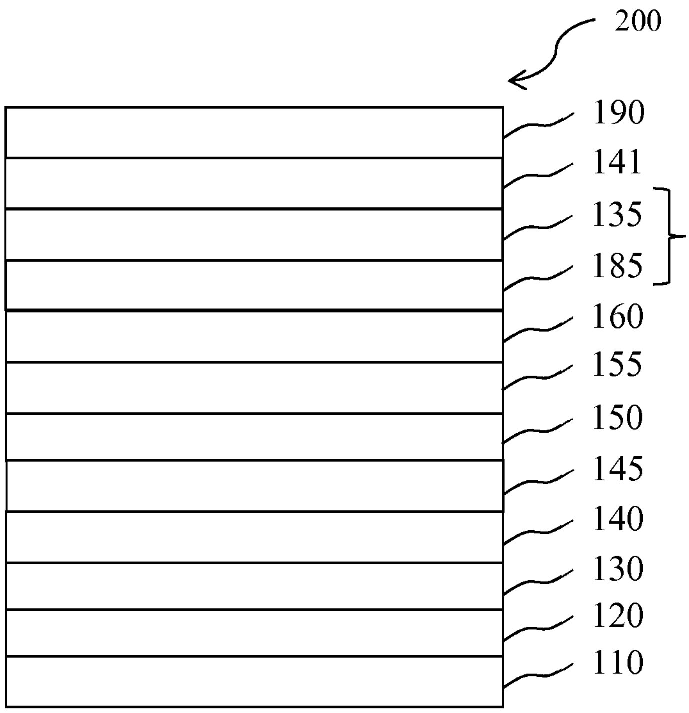


Fig.3

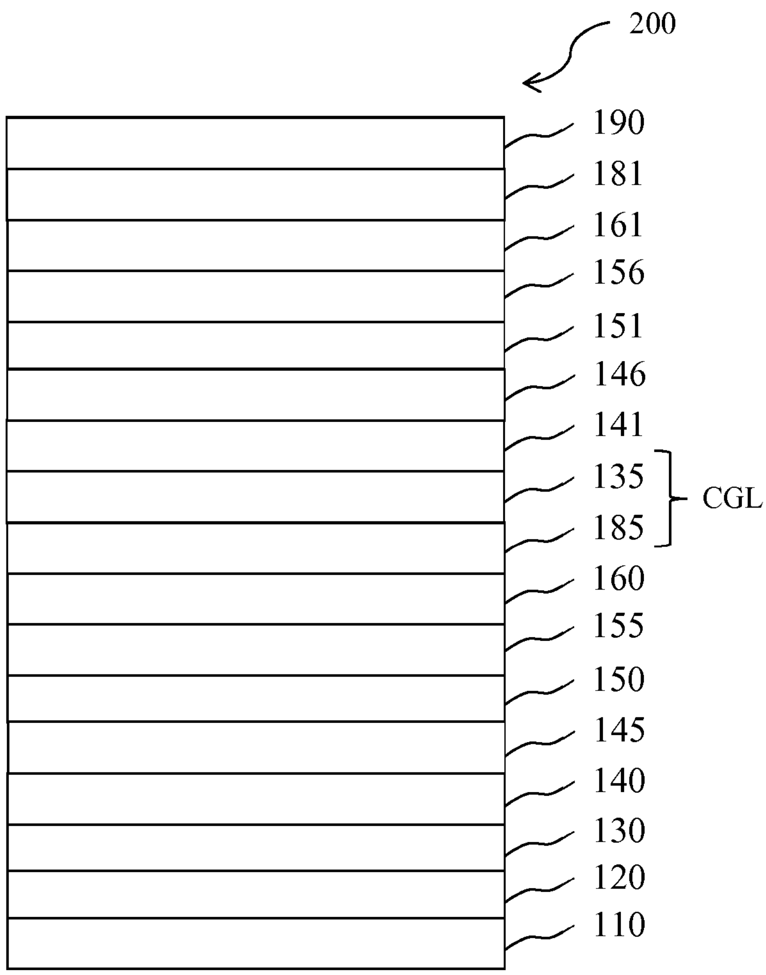
200
190
181
161
156
151
146
141
135
185
CGL
160
155
150
145
140
130
120
110

Fig.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150318510 A1
- WO 201689165 A2
- CN 105810837 A
- KR 20150012974 A
- US 20190263834 A1
- CN 110156755 A
- CN 106916170 A
- CN 107602538 A
- KR 20150135626 A
- KR 20150134923 A
- US 6140763 A
- US 6614176 B
- US 2016248022 A


### Non-patent literature cited in the description


- *CHEMICAL ABSTRACTS,* 1242056-42-3
- *CHEMICAL ABSTRACTS,* 1198399-61-9